(19) Europäisches Patentamt
European Patent Office
Office européen des brevets

(11) EP 3 305 322 A1

(12) EUROPEAN PATENT APPLICATION
published in accordance with Art. 153(4) EPC

(43) Date of publication:
**11.04.2018 Bulletin 2018/15**

(21) Application number: **16803426.2**

(22) Date of filing: **02.06.2016**

(51) Int Cl.:
***A61K 39/395*** *(2006.01)* ***A61P 35/00*** *(2006.01)*

(86) International application number:
**PCT/JP2016/066331**

(87) International publication number:
**WO 2016/194992 (08.12.2016 Gazette 2016/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **05.06.2015 JP 2015114418**

(71) Applicant: **Chugai Seiyaku Kabushiki Kaisha**
**Kita-ku**
**Tokyo 115-8543 (JP)**

(72) Inventors:
- **TANIGUCHI, Kenji**
  **Kamakura-shi**
  **Kanagawa 247-8530 (JP)**
- **MIYAZAKI, Taro**
  **Tokyo 103-8324 (JP)**

(74) Representative: **Vossius & Partner**
**Patentanwälte Rechtsanwälte mbB**
**Siebertstrasse 3**
**81675 München (DE)**

# (54) COMBINED USE OF IMMUNE ACTIVATORS

(57) The present invention is based on the finding that combined use of a multispecific antibody comprising: (1) a cancer-specific antigen-binding domain, (2) a CD3-binding domain, and (3) a domain comprising an Fc region having decreased Fcγ receptor-binding activity with a tumor necrosis factor (TNF) receptor superfamily agonist antibody can reduce side effects such as liver injury observed when the agonist antibody is prescribed alone, and achieve effective therapeutic effects.

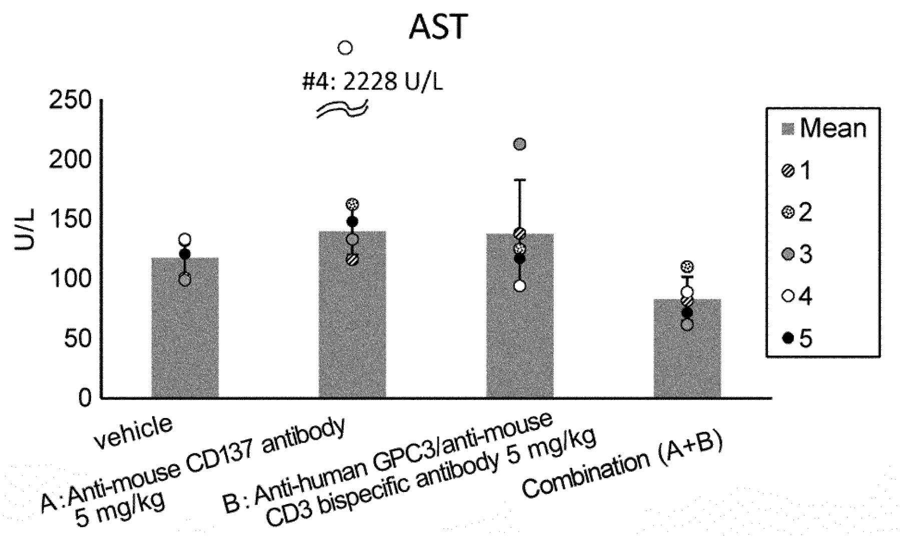


FIG. 2

EP 3 305 322 A1

SEQUENCE LISTING

<110> CHUGAI SEIYAKU KABUSHIKI KAISHA

<120> COMBINATION OF IMMUNE ACTIVATING AGENTS

<130> C1-A1512P

<150> JP 2015-114418
<151> 2015-06-05

<160> 38

<170> PatentIn version 3.5

<210> 1
<211> 330
<212> PRT
<213> Homo sapiens

<400> 1

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
```

```
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330


<210>  2
<211>  326
<212>  PRT
<213>  Homo sapiens

<400>  2

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
```

50 55 60

Leu Ser Ser Val Val Thr Val Pro Ser Ser Asn Phe Gly Thr Gln Thr
65 70 75 80

Tyr Thr Cys Asn Val Asp His Lys Pro Ser Asn Thr Lys Val Asp Lys
85 90 95

Thr Val Glu Arg Lys Cys Cys Val Glu Cys Pro Pro Cys Pro Ala Pro
100 105 110

Pro Val Ala Gly Pro Ser Val Phe Leu Phe Pro Pro Lys Pro Lys Asp
115 120 125

Thr Leu Met Ile Ser Arg Thr Pro Glu Val Thr Cys Val Val Val Asp
130 135 140

Val Ser His Glu Asp Pro Glu Val Gln Phe Asn Trp Tyr Val Asp Gly
145 150 155 160

Val Glu Val His Asn Ala Lys Thr Lys Pro Arg Glu Glu Gln Phe Asn
165 170 175

Ser Thr Phe Arg Val Val Ser Val Leu Thr Val Val His Gln Asp Trp
180 185 190

Leu Asn Gly Lys Glu Tyr Lys Cys Lys Val Ser Asn Lys Gly Leu Pro
195 200 205

Ala Pro Ile Glu Lys Thr Ile Ser Lys Thr Lys Gly Gln Pro Arg Glu
210 215 220

Pro Gln Val Tyr Thr Leu Pro Pro Ser Arg Glu Glu Met Thr Lys Asn
225 230 235 240

Gln Val Ser Leu Thr Cys Leu Val Lys Gly Phe Tyr Pro Ser Asp Ile
245 250 255

Ala Val Glu Trp Glu Ser Asn Gly Gln Pro Glu Asn Asn Tyr Lys Thr
260 265 270

Thr Pro Pro Met Leu Asp Ser Asp Gly Ser Phe Phe Leu Tyr Ser Lys
275 280 285

Leu Thr Val Asp Lys Ser Arg Trp Gln Gln Gly Asn Val Phe Ser Cys
290 295 300

Ser Val Met His Glu Ala Leu His Asn His Tyr Thr Gln Lys Ser Leu
305 310 315 320

Ser Leu Ser Pro Gly Lys
325

<210> 3
<211> 377
<212> PRT
<213> Homo sapiens

<400> 3

Ala Ser Thr Lys Gly Pro Ser Val Phe Pro Leu Ala Pro Cys Ser Arg
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Thr Cys Asn Val Asn His Lys Pro Ser Asn Thr Lys Val Asp Lys
85 90 95

Arg Val Glu Leu Lys Thr Pro Leu Gly Asp Thr Thr His Thr Cys Pro
100 105 110

Arg Cys Pro Glu Pro Lys Ser Cys Asp Thr Pro Pro Pro Cys Pro Arg
115 120 125

Cys Pro Glu Pro Lys Ser Cys Asp Thr Pro Pro Pro Cys Pro Arg Cys
130 135 140

Pro Glu Pro Lys Ser Cys Asp Thr Pro Pro Pro Cys Pro Arg Cys Pro
145 150 155 160

Ala Pro Glu Leu Leu Gly Gly Pro Ser Val Phe Leu Phe Pro Pro Lys
165 170 175

Pro Lys Asp Thr Leu Met Ile Ser Arg Thr Pro Glu Val Thr Cys Val
180 185 190

```
Val Val Asp Val Ser His Glu Asp Pro Glu Val Gln Phe Lys Trp Tyr
        195                 200                 205

Val Asp Gly Val Glu Val His Asn Ala Lys Thr Lys Pro Arg Glu Glu
    210                 215                 220

Gln Tyr Asn Ser Thr Phe Arg Val Val Ser Val Leu Thr Val Leu His
225                 230                 235                 240

Gln Asp Trp Leu Asn Gly Lys Glu Tyr Lys Cys Lys Val Ser Asn Lys
                245                 250                 255

Ala Leu Pro Ala Pro Ile Glu Lys Thr Ile Ser Lys Thr Lys Gly Gln
            260                 265                 270

Pro Arg Glu Pro Gln Val Tyr Thr Leu Pro Pro Ser Arg Glu Glu Met
        275                 280                 285

Thr Lys Asn Gln Val Ser Leu Thr Cys Leu Val Lys Gly Phe Tyr Pro
    290                 295                 300

Ser Asp Ile Ala Val Glu Trp Glu Ser Ser Gly Gln Pro Glu Asn Asn
305                 310                 315                 320

Tyr Asn Thr Thr Pro Pro Met Leu Asp Ser Asp Gly Ser Phe Phe Leu
                325                 330                 335

Tyr Ser Lys Leu Thr Val Asp Lys Ser Arg Trp Gln Gln Gly Asn Ile
            340                 345                 350

Phe Ser Cys Ser Val Met His Glu Ala Leu His Asn Arg Phe Thr Gln
        355                 360                 365

Lys Ser Leu Ser Leu Ser Pro Gly Lys
    370                 375


<210>  4
<211>  327
<212>  PRT
<213>  Homo sapiens

<400>  4

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30
```

```
Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285
```

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  5
<211>  330
<212>  PRT
<213>  Artificial

<220>
<223>  an artificially synthesized sequence

<400>  5

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 6
<211> 326
<212> PRT
<213> Artificial

<220>
<223> an artificially synthesized sequence

<400> 6

Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser

```
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285
```

```
Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325
```

<210> 7
<211> 377
<212> PRT
<213> Artificial

<220>
<223> an artificially synthesized sequence

<400> 7

```
Ala Ser Thr Lys Gly Pro Ser Val Phe Pro Leu Ala Pro Cys Ser Arg
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Thr Cys Asn Val Asn His Lys Pro Ser Asn Thr Lys Val Asp Lys
                85                  90                  95

Arg Val Glu Leu Lys Thr Pro Leu Gly Asp Thr Thr His Thr Cys Pro
            100                 105                 110

Arg Cys Pro Glu Pro Lys Ser Cys Asp Thr Pro Pro Pro Cys Pro Arg
        115                 120                 125

Cys Pro Glu Pro Lys Ser Cys Asp Thr Pro Pro Pro Cys Pro Arg Cys
    130                 135                 140

Pro Glu Pro Lys Ser Cys Asp Thr Pro Pro Pro Cys Pro Arg Cys Pro
145                 150                 155                 160

Ala Pro Glu Leu Leu Gly Gly Pro Ser Val Phe Leu Phe Pro Pro Lys
```

165 170 175

Pro Lys Asp Thr Leu Met Ile Ser Arg Thr Pro Glu Val Thr Cys Val
180 185 190

Val Val Asp Val Ser His Glu Asp Pro Glu Val Gln Phe Lys Trp Tyr
195 200 205

Val Asp Gly Val Glu Val His Asn Ala Lys Thr Lys Pro Arg Glu Glu
210 215 220

Gln Tyr Asn Ser Thr Phe Arg Val Val Ser Val Leu Thr Val Leu His
225 230 235 240

Gln Asp Trp Leu Asn Gly Lys Glu Tyr Lys Cys Lys Val Ser Asn Lys
245 250 255

Ala Leu Pro Ala Pro Ile Glu Lys Thr Ile Ser Lys Thr Lys Gly Gln
260 265 270

Pro Arg Glu Pro Gln Val Tyr Thr Leu Pro Pro Ser Arg Glu Glu Met
275 280 285

Thr Lys Asn Gln Val Ser Leu Thr Cys Leu Val Lys Gly Phe Tyr Pro
290 295 300

Ser Asp Ile Ala Val Glu Trp Glu Ser Ser Gly Gln Pro Glu Asn Asn
305 310 315 320

Tyr Asn Thr Thr Pro Pro Met Leu Asp Ser Asp Gly Ser Phe Phe Leu
325 330 335

Tyr Ser Lys Leu Thr Val Asp Lys Ser Arg Trp Gln Gln Gly Asn Ile
340 345 350

Phe Ser Cys Ser Val Met His Glu Ala Leu His Asn Arg Phe Thr Gln
355 360 365

Lys Ser Leu Ser Leu Ser Pro Gly Lys
370 375

<210> 8
<211> 327
<212> PRT
<213> Artificial

<220>
<223> an artificially synthesized sequence

<400> 8

```
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
```

245 250 255

Ile Ala Val Glu Trp Glu Ser Asn Gly Gln Pro Glu Asn Asn Tyr Lys
260 265 270

Thr Thr Pro Pro Val Leu Asp Ser Asp Gly Ser Phe Phe Leu Tyr Ser
275 280 285

Arg Leu Thr Val Asp Lys Ser Arg Trp Gln Glu Gly Asn Val Phe Ser
290 295 300

Cys Ser Val Met His Glu Ala Leu His Asn His Tyr Thr Gln Lys Ser
305 310 315 320

Leu Ser Leu Ser Leu Gly Lys
325

<210> 9
<211> 141
<212> PRT
<213> Homo sapiens

<400> 9

Asp Ile Gln Asn Pro Asp Pro Ala Val Tyr Gln Leu Arg Asp Ser Lys
1 5 10 15

Ser Ser Asp Lys Ser Val Cys Leu Phe Thr Asp Phe Asp Ser Gln Thr
20 25 30

Asn Val Ser Gln Ser Lys Asp Ser Asp Val Tyr Ile Thr Asp Lys Thr
35 40 45

Val Leu Asp Met Arg Ser Met Asp Phe Lys Ser Asn Ser Ala Val Ala
50 55 60

Trp Ser Asn Lys Ser Asp Phe Ala Cys Ala Asn Ala Phe Asn Asn Ser
65 70 75 80

Ile Ile Pro Glu Asp Thr Phe Phe Pro Ser Pro Glu Ser Ser Cys Asp
85 90 95

Val Lys Leu Val Glu Lys Ser Phe Glu Thr Asp Thr Asn Leu Asn Phe
100 105 110

Gln Asn Leu Ser Val Ile Gly Phe Arg Ile Leu Leu Leu Lys Val Ala
115 120 125

Gly Phe Asn Leu Leu Met Thr Leu Arg Leu Trp Ser Ser

130 135 140

<210> 10
<211> 179
<212> PRT
<213> Homo sapiens

<400> 10

Glu Asp Leu Lys Asn Val Phe Pro Pro Glu Val Ala Val Phe Glu Pro
1 5 10 15

Ser Glu Ala Glu Ile Ser His Thr Gln Lys Ala Thr Leu Val Cys Leu
20 25 30

Ala Thr Gly Phe Tyr Pro Asp His Val Glu Leu Ser Trp Trp Val Asn
35 40 45

Gly Lys Glu Val His Ser Gly Val Ser Thr Asp Pro Gln Pro Leu Lys
50 55 60

Glu Gln Pro Ala Leu Asn Asp Ser Arg Tyr Cys Leu Ser Ser Arg Leu
65 70 75 80

Arg Val Ser Ala Thr Phe Trp Gln Asn Pro Arg Asn His Phe Arg Cys
85 90 95

Gln Val Gln Phe Tyr Gly Leu Ser Glu Asn Asp Glu Trp Thr Gln Asp
100 105 110

Arg Ala Lys Pro Val Thr Gln Ile Val Ser Ala Glu Ala Trp Gly Arg
115 120 125

Ala Asp Cys Gly Phe Thr Ser Glu Ser Tyr Gln Gln Gly Val Leu Ser
130 135 140

Ala Thr Ile Leu Tyr Glu Ile Leu Leu Gly Lys Ala Thr Leu Tyr Ala
145 150 155 160

Val Leu Val Ser Ala Leu Val Leu Met Ala Met Val Lys Arg Lys Asp
165 170 175

Ser Arg Gly

<210> 11
<211> 173
<212> PRT
<213> Homo sapiens

<400> 11

Asp Lys Gln Leu Asp Ala Asp Val Ser Pro Lys Pro Thr Ile Phe Leu
1 5 10 15

Pro Ser Ile Ala Glu Thr Lys Leu Gln Lys Ala Gly Thr Tyr Leu Cys
20 25 30

Leu Leu Glu Lys Phe Phe Pro Asp Val Ile Lys Ile His Trp Gln Glu
35 40 45

Lys Lys Ser Asn Thr Ile Leu Gly Ser Gln Glu Gly Asn Thr Met Lys
50 55 60

Thr Asn Asp Thr Tyr Met Lys Phe Ser Trp Leu Thr Val Pro Glu Lys
65 70 75 80

Ser Leu Asp Lys Glu His Arg Cys Ile Val Arg His Glu Asn Asn Lys
85 90 95

Asn Gly Val Asp Gln Glu Ile Ile Phe Pro Pro Ile Lys Thr Asp Val
100 105 110

Ile Thr Met Asp Pro Lys Asp Asn Cys Ser Lys Asp Ala Asn Asp Thr
115 120 125

Leu Leu Leu Gln Leu Thr Asn Thr Ser Ala Tyr Tyr Met Tyr Leu Leu
130 135 140

Leu Leu Leu Lys Ser Val Val Tyr Phe Ala Ile Ile Thr Cys Cys Leu
145 150 155 160

Leu Arg Arg Thr Ala Phe Cys Cys Asn Gly Glu Lys Ser
165 170

<210> 12
<211> 204
<212> PRT
<213> Homo sapiens

<400> 12

Lys Gln Leu Asp Ala Asp Val Ser Pro Lys Pro Thr Ile Phe Leu Pro
1 5 10 15

Ser Ile Ala Glu Thr Lys Leu Gln Lys Ala Gly Thr Tyr Leu Cys Leu
20 25 30

Leu Glu Lys Phe Phe Pro Asp Ile Ile Lys Ile His Trp Gln Glu Lys
35 40 45

```
Lys Ser Asn Thr Ile Leu Gly Ser Gln Glu Gly Asn Thr Met Lys Thr
    50                  55                  60

Asn Asp Thr Tyr Met Lys Phe Ser Trp Leu Thr Val Pro Glu Glu Ser
65                  70                  75                  80

Leu Asp Lys Glu His Arg Cys Ile Val Arg His Glu Asn Asn Lys Asn
                85                  90                  95

Gly Ile Asp Gln Glu Ile Ile Phe Pro Pro Ile Lys Thr Asp Val Thr
            100                 105                 110

Thr Val Asp Pro Lys Asp Ser Tyr Ser Lys Asp Ala Asn Asp Val Thr
        115                 120                 125

Thr Val Asp Pro Lys Tyr Asn Tyr Ser Lys Asp Ala Asn Asp Val Ile
    130                 135                 140

Thr Met Asp Pro Lys Asp Asn Trp Ser Lys Asp Ala Asn Asp Thr Leu
145                 150                 155                 160

Leu Leu Gln Leu Thr Asn Thr Ser Ala Tyr Tyr Met Tyr Leu Leu Leu
                165                 170                 175

Leu Leu Lys Ser Val Val Tyr Phe Ala Ile Ile Thr Cys Cys Leu Leu
            180                 185                 190

Gly Arg Thr Ala Phe Cys Cys Asn Gly Glu Lys Ser
        195                 200


<210>  13
<211>  177
<212>  PRT
<213>  Homo sapiens

<400>  13

Pro Ser Tyr Thr Gly Gly Tyr Ala Asp Lys Leu Ile Phe Gly Lys Gly
1               5                   10                  15

Thr Arg Val Thr Val Glu Pro Arg Ser Gln Pro His Thr Lys Pro Ser
            20                  25                  30

Val Phe Val Met Lys Asn Gly Thr Asn Val Ala Cys Leu Val Lys Glu
        35                  40                  45

Phe Tyr Pro Lys Asp Ile Arg Ile Asn Leu Val Ser Ser Lys Lys Ile
    50                  55                  60
```

Thr Glu Phe Asp Pro Ala Ile Val Ile Ser Pro Ser Gly Lys Tyr Asn
65 70 75 80

Ala Val Lys Leu Gly Lys Tyr Glu Asp Ser Asn Ser Val Thr Cys Ser
85 90 95

Val Gln His Asp Asn Lys Thr Val His Ser Thr Asp Phe Glu Val Lys
100 105 110

Thr Asp Ser Thr Asp His Val Lys Pro Lys Glu Thr Glu Asn Thr Lys
115 120 125

Gln Pro Ser Lys Ser Cys His Lys Pro Lys Ala Ile Val His Thr Glu
130 135 140

Lys Val Asn Met Met Ser Leu Thr Val Leu Gly Leu Arg Met Leu Phe
145 150 155 160

Ala Lys Thr Val Ala Val Asn Phe Leu Leu Thr Ala Lys Leu Phe Phe
165 170 175

Leu

<210> 14
<211> 549
<212> DNA
<213> Homo sapiens

<400> 14
atggaacagg ggaagggcct ggctgtcctc atcctggcta tcattcttct tcaaggtact 60

ttggcccagt caatcaaagg aaaccacttg gttaaggtgt atgactatca agaagatggt 120

tcggtacttc tgacttgtga tgcagaagcc aaaaatatca catggtttaa agatgggaag 180

atgatcggct tcctaactga agataaaaaa aaatggaatc tgggaagtaa tgccaaggac 240

cctcgaggga tgtatcagtg taaaggatca cagaacaagt caaaaccact ccaagtgtat 300

tacagaatgt gtcagaactg cattgaacta aatgcagcca ccatatctgg ctttctcttt 360

gctgaaatcg tcagcatttt cgtccttgct gttggggtct acttcattgc tggacaggat 420

ggagttcgcc agtcgagagc ttcagacaag cagactctgt tgcccaatga ccagctctac 480

cagcccctca aggatcgaga agatgaccag tacagccacc ttcaaggaaa ccagttgagg 540

aggaattga 549

<210> 15
<211> 182

<212> PRT
<213> Homo sapiens

<400> 15

Met Glu Gln Gly Lys Gly Leu Ala Val Leu Ile Leu Ala Ile Ile Leu
1 5 10 15

Leu Gln Gly Thr Leu Ala Gln Ser Ile Lys Gly Asn His Leu Val Lys
20 25 30

Val Tyr Asp Tyr Gln Glu Asp Gly Ser Val Leu Leu Thr Cys Asp Ala
35 40 45

Glu Ala Lys Asn Ile Thr Trp Phe Lys Asp Gly Lys Met Ile Gly Phe
50 55 60

Leu Thr Glu Asp Lys Lys Lys Trp Asn Leu Gly Ser Asn Ala Lys Asp
65 70 75 80

Pro Arg Gly Met Tyr Gln Cys Lys Gly Ser Gln Asn Lys Ser Lys Pro
85 90 95

Leu Gln Val Tyr Tyr Arg Met Cys Gln Asn Cys Ile Glu Leu Asn Ala
100 105 110

Ala Thr Ile Ser Gly Phe Leu Phe Ala Glu Ile Val Ser Ile Phe Val
115 120 125

Leu Ala Val Gly Val Tyr Phe Ile Ala Gly Gln Asp Gly Val Arg Gln
130 135 140

Ser Arg Ala Ser Asp Lys Gln Thr Leu Leu Pro Asn Asp Gln Leu Tyr
145 150 155 160

Gln Pro Leu Lys Asp Arg Glu Asp Asp Gln Tyr Ser His Leu Gln Gly
165 170 175

Asn Gln Leu Arg Arg Asn
180

<210> 16
<211> 516
<212> DNA
<213> Homo sapiens

<400> 16
atggaacata gcacgtttct ctctggcctg gtactggcta cccttctctc gcaagtgagc 60

cccttcaaga tacctataga ggaacttgag gacagagtgt ttgtgaattg caataccagc 120

atcacatggg tagagggaac ggtgggaaca ctgctctcag acattacaag actggacctg 180

ggaaaacgca tcctggaccc acgaggaata tataggtgta atgggacaga tatatacaag 240

gacaaagaat ctaccgtgca agttcattat cgaatgtgcc agagctgtgt ggagctggat 300

ccagccaccg tggctggcat cattgtcact gatgtcattg ccactctgct ccttgctttg 360

ggagtcttct gctttgctgg acatgagact ggaaggctgt ctggggctgc cgacacacaa 420

gctctgttga ggaatgacca ggtctatcag cccctccgag atcgagatga tgctcagtac 480

agccaccttg gaggaaactg ggctcggaac aagtga 516

<210> 17
<211> 171
<212> PRT
<213> Homo sapiens

<400> 17

Met Glu His Ser Thr Phe Leu Ser Gly Leu Val Leu Ala Thr Leu Leu
1 5 10 15

Ser Gln Val Ser Pro Phe Lys Ile Pro Ile Glu Glu Leu Glu Asp Arg
20 25 30

Val Phe Val Asn Cys Asn Thr Ser Ile Thr Trp Val Glu Gly Thr Val
35 40 45

Gly Thr Leu Leu Ser Asp Ile Thr Arg Leu Asp Leu Gly Lys Arg Ile
50 55 60

Leu Asp Pro Arg Gly Ile Tyr Arg Cys Asn Gly Thr Asp Ile Tyr Lys
65 70 75 80

Asp Lys Glu Ser Thr Val Gln Val His Tyr Arg Met Cys Gln Ser Cys
85 90 95

Val Glu Leu Asp Pro Ala Thr Val Ala Gly Ile Ile Val Thr Asp Val
100 105 110

Ile Ala Thr Leu Leu Leu Ala Leu Gly Val Phe Cys Phe Ala Gly His
115 120 125

Glu Thr Gly Arg Leu Ser Gly Ala Ala Asp Thr Gln Ala Leu Leu Arg
130 135 140

Asn Asp Gln Val Tyr Gln Pro Leu Arg Asp Arg Asp Asp Ala Gln Tyr
145 150 155 160

Ser His Leu Gly Gly Asn Trp Ala Arg Asn Lys

165 170

<210> 18
<211> 624
<212> DNA
<213> Homo sapiens

<400> 18
atgcagtcgg gcactcactg gagagttctg ggcctctgcc tcttatcagt tggcgtttgg 60

gggcaagatg gtaatgaaga aatgggtggt attacacaga caccatataa agtctccatc 120

tctggaacca cagtaatatt gacatgccct cagtatcctg gatctgaaat actatggcaa 180

cacaatgata aaaacatagg cggtgatgag gatgataaaa acataggcag tgatgaggat 240

cacctgtcac tgaaggaatt ttcagaattg gagcaaagtg gttattatgt ctgctacccc 300

agaggaagca aaccagaaga tgcgaacttt tatctctacc tgagggcaag agtgtgtgag 360

aactgcatgg agatggatgt gatgtcggtg gccacaattg tcatagtgga catctgcatc 420

actgggggct tgctgctgct ggtttactac tggagcaaga atagaaaggc caaggccaag 480

cctgtgacac gaggagcggg tgctggcggc aggcaaaggg gacaaaacaa ggagaggcca 540

ccacctgttc ccaacccaga ctatgagccc atccggaaag gccagcggga cctgtattct 600

ggcctgaatc agagacgcat ctga 624

<210> 19
<211> 207
<212> PRT
<213> Homo sapiens

<400> 19

Met Gln Ser Gly Thr His Trp Arg Val Leu Gly Leu Cys Leu Leu Ser
1 5 10 15

Val Gly Val Trp Gly Gln Asp Gly Asn Glu Glu Met Gly Gly Ile Thr
20 25 30

Gln Thr Pro Tyr Lys Val Ser Ile Ser Gly Thr Thr Val Ile Leu Thr
35 40 45

Cys Pro Gln Tyr Pro Gly Ser Glu Ile Leu Trp Gln His Asn Asp Lys
50 55 60

Asn Ile Gly Gly Asp Glu Asp Asp Lys Asn Ile Gly Ser Asp Glu Asp
65 70 75 80

His Leu Ser Leu Lys Glu Phe Ser Glu Leu Glu Gln Ser Gly Tyr Tyr
85 90 95

Val Cys Tyr Pro Arg Gly Ser Lys Pro Glu Asp Ala Asn Phe Tyr Leu
100 105 110

Tyr Leu Arg Ala Arg Val Cys Glu Asn Cys Met Glu Met Asp Val Met
115 120 125

Ser Val Ala Thr Ile Val Ile Val Asp Ile Cys Ile Thr Gly Gly Leu
130 135 140

Leu Leu Leu Val Tyr Tyr Trp Ser Lys Asn Arg Lys Ala Lys Ala Lys
145 150 155 160

Pro Val Thr Arg Gly Ala Gly Ala Gly Gly Arg Gln Arg Gly Gln Asn
165 170 175

Lys Glu Arg Pro Pro Pro Val Pro Asn Pro Asp Tyr Glu Pro Ile Arg
180 185 190

Lys Gly Gln Arg Asp Leu Tyr Ser Gly Leu Asn Gln Arg Arg Ile
195 200 205

<210> 20
<211> 4
<212> PRT
<213> Artificial

<220>
<223> an artificially synthesized sequence

<400> 20

Gly Gly Gly Ser
1

<210> 21
<211> 4
<212> PRT
<213> Artificial

<220>
<223> an artificially synthesized sequence

<400> 21

Ser Gly Gly Gly
1

<210> 22
<211> 5
<212> PRT
<213> Artificial

<220>
<223> an artificially synthesized sequence

<400> 22

Gly Gly Gly Gly Ser
1               5

<210> 23
<211> 5
<212> PRT
<213> Artificial

<220>
<223> an artificially synthesized sequence

<400> 23

Ser Gly Gly Gly Gly
1               5

<210> 24
<211> 6
<212> PRT
<213> Artificial

<220>
<223> an artificially synthesized sequence

<400> 24

Gly Gly Gly Gly Gly Ser
1               5

<210> 25
<211> 6
<212> PRT
<213> Artificial

<220>
<223> an artificially synthesized sequence

<400> 25

Ser Gly Gly Gly Gly Gly
1               5

<210> 26
<211> 7
<212> PRT
<213> Artificial

<220>
<223> an artificially synthesized sequence

<400> 26

Gly Gly Gly Gly Gly Gly Ser
1               5

<210>  27
<211>  7
<212>  PRT
<213>  Artificial

<220>
<223>  an artificially synthesized sequence

<400>  27

Ser Gly Gly Gly Gly Gly Gly
1               5

<210>  28
<211>  113
<212>  PRT
<213>  Artificial

<220>
<223>  an artificially synthesized sequence

<400>  28

Gln Val Gln Leu Lys Glu Ala Gly Pro Gly Leu Val Gln Pro Thr Gln
1               5                   10                  15

Thr Leu Ser Leu Thr Cys Thr Val Ser Gly Phe Ser Leu Thr Ser Asp
            20                  25                  30

Gly Val His Trp Ile Arg Gln Pro Pro Gly Lys Gly Leu Glu Trp Met
        35                  40                  45

Gly Ile Ile Tyr Tyr Asp Gly Gly Thr Asp Tyr Asn Ser Ala Ile Lys
    50                  55                  60

Ser Arg Leu Ser Ile Ser Arg Asp Thr Ser Lys Ser Gln Val Phe Leu
65                  70                  75                  80

Lys Ile Asn Ser Leu Gln Thr Asp Asp Thr Ala Met Tyr Tyr Cys Ala
                85                  90                  95

Arg Ile His Phe Asp Tyr Trp Gly Gln Gly Val Met Val Thr Val Ser
            100                 105                 110

Ser

<210>  29
<211>  437
<212>  PRT
<213>  Artificial

<220>
<223>  an artificially synthesized sequence

<400> 29

Gln Val Gln Leu Lys Glu Ala Gly Pro Gly Leu Val Gln Pro Thr Gln
1               5                   10                  15

Thr Leu Ser Leu Thr Cys Thr Val Ser Gly Phe Ser Leu Thr Ser Asp
            20                  25                  30

Gly Val His Trp Ile Arg Gln Pro Pro Gly Lys Gly Leu Glu Trp Met
        35                  40                  45

Gly Ile Ile Tyr Tyr Asp Gly Gly Thr Asp Tyr Asn Ser Ala Ile Lys
    50                  55                  60

Ser Arg Leu Ser Ile Ser Arg Asp Thr Ser Lys Ser Gln Val Phe Leu
65                  70                  75                  80

Lys Ile Asn Ser Leu Gln Thr Asp Asp Thr Ala Met Tyr Tyr Cys Ala
                85                  90                  95

Arg Ile His Phe Asp Tyr Trp Gly Gln Gly Val Met Val Thr Val Ser
            100                 105                 110

Ser Ala Lys Thr Thr Pro Pro Ser Val Tyr Pro Leu Ala Pro Gly Ser
        115                 120                 125

Ala Ala Gln Thr Asn Ser Met Val Thr Leu Gly Cys Leu Val Lys Gly
    130                 135                 140

Tyr Phe Pro Glu Pro Val Thr Val Thr Trp Asn Ser Gly Ser Leu Ser
145                 150                 155                 160

Ser Gly Val His Thr Phe Pro Ala Val Leu Gln Ser Asp Leu Tyr Thr
                165                 170                 175

Leu Ser Ser Ser Val Thr Val Pro Ser Ser Thr Trp Pro Ser Glu Thr
            180                 185                 190

Val Thr Cys Asn Val Ala His Pro Ala Ser Ser Thr Lys Val Asp Lys
        195                 200                 205

Lys Ile Val Pro Arg Asp Cys Gly Cys Lys Pro Cys Ile Cys Glu Pro
    210                 215                 220

Asn Glu Val Glu Asp Val Phe Ile Phe Pro Pro Lys Pro Lys Asp Val
225                 230                 235                 240

Leu Thr Ile Thr Leu Thr Pro Lys Val Thr Cys Val Val Val Asp Ile
245 250 255

Ser Lys Asp Asp Pro Glu Val Gln Phe Ser Trp Phe Val Asp Asp Val
260 265 270

Glu Val His Thr Ala Gln Thr Gln Pro Arg Glu Glu Gln Phe Asn Ser
275 280 285

Thr Phe Arg Ser Val Ser Glu Leu Pro Ile Met His Gln Asp Trp Leu
290 295 300

Asn Gly Lys Glu Phe Lys Cys Arg Val Asp Ser Ala Ala Phe Pro Ala
305 310 315 320

Pro Ile Glu Lys Thr Ile Ser Lys Thr Lys Gly Arg Pro Lys Ala Pro
325 330 335

Gln Val Tyr Thr Ile Pro Pro Pro Lys Glu Gln Met Ala Lys Asp Lys
340 345 350

Val Ser Leu Thr Cys Met Ile Thr Asp Phe Phe Pro Glu Asp Ile Thr
355 360 365

Val Glu Trp Gln Trp Asn Gly Gln Pro Ala Glu Asn Tyr Lys Asn Thr
370 375 380

Gln Pro Ile Met Asp Thr Asp Gly Ser Tyr Phe Val Tyr Ser Lys Leu
385 390 395 400

Asn Val Gln Lys Ser Asn Trp Glu Ala Gly Asn Thr Phe Thr Cys Ser
405 410 415

Val Leu His Glu Gly Leu His Asn His His Thr Glu Lys Ser Leu Ser
420 425 430

His Ser Pro Gly Lys
435

<210> 30
<211> 213
<212> PRT
<213> Artificial

<220>
<223> an artificially synthesized sequence

<400> 30

Asp Ile Val Leu Thr Gln Ser Pro Thr Thr Ile Ala Ala Ser Pro Gly

```
1               5                   10                  15

Glu Lys Val Thr Ile Thr Cys Arg Ala Ser Ser Ser Val Ser Tyr Met
            20                  25                  30

Tyr Trp Tyr Gln Gln Lys Ser Gly Ala Ser Pro Lys Leu Trp Ile Tyr
        35                  40                  45

Asp Thr Ser Lys Leu Ala Ser Gly Val Pro Asn Arg Phe Ser Gly Ser
    50                  55                  60

Gly Ser Gly Thr Ser Tyr Ser Leu Ala Leu Asn Thr Met Glu Thr Glu
65                  70                  75                  80

Asp Ala Ala Thr Tyr Tyr Cys Gln Gln Trp Ser Ser Thr Pro Leu Thr
                85                  90                  95

Phe Gly Ser Gly Thr Lys Leu Glu Ile Lys Arg Ala Asp Ala Ala Pro
            100                 105                 110

Thr Val Ser Ile Phe Pro Pro Ser Ser Glu Gln Leu Thr Ser Gly Gly
        115                 120                 125

Ala Ser Val Val Cys Phe Leu Asn Asn Phe Tyr Pro Lys Asp Ile Asn
    130                 135                 140

Val Lys Trp Lys Ile Asp Gly Ser Glu Arg Gln Asn Gly Val Leu Asn
145                 150                 155                 160

Ser Trp Thr Asp Gln Asp Ser Lys Asp Ser Thr Tyr Ser Met Ser Ser
                165                 170                 175

Thr Leu Thr Leu Thr Lys Asp Glu Tyr Glu Arg His Asn Ser Tyr Thr
            180                 185                 190

Cys Glu Ala Thr His Lys Thr Ser Thr Ser Pro Ile Val Lys Ser Phe
        195                 200                 205

Asn Arg Asn Glu Cys
    210
```

<210> 31
<211> 450
<212> PRT
<213> Artificial

<220>
<223> an artificially synthesized sequence

<400> 31

```
Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Arg Asn Thr Tyr Leu His Trp Tyr Gln Gln Lys Pro Gly Gln Ala
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Pro Thr Phe Gly Gln Gly Thr Lys Leu Glu Ile Lys
            100                 105                 110

Ser Ser Ala Ser Thr Lys Gly Pro Ser Val Phe Pro Leu Ala Pro Ser
        115                 120                 125

Ser Lys Ser Thr Ser Gly Gly Thr Ala Ala Leu Gly Cys Leu Val Lys
    130                 135                 140

Asp Tyr Phe Pro Glu Pro Val Thr Val Ser Trp Asn Ser Gly Ala Leu
145                 150                 155                 160

Thr Ser Gly Val His Thr Phe Pro Ala Val Leu Gln Ser Ser Gly Leu
                165                 170                 175

Tyr Ser Leu Ser Ser Val Val Thr Val Pro Ser Ser Ser Leu Gly Thr
            180                 185                 190

Gln Thr Tyr Ile Cys Asn Val Asn His Lys Pro Ser Asn Thr Lys Val
        195                 200                 205

Asp Lys Lys Val Glu Pro Lys Ser Cys Asp Lys Thr His Thr Cys Pro
    210                 215                 220

Pro Cys Pro Ala Pro Glu Ala Ala Gly Gly Pro Ser Val Phe Leu Phe
225                 230                 235                 240

Pro Pro Lys Pro Lys Asp Thr Leu Met Ile Ser Arg Thr Pro Glu Val
```

245 250 255

Thr Cys Val Val Val Asp Val Ser His Glu Asp Pro Glu Val Lys Phe
260 265 270

Asn Trp Tyr Val Asp Gly Val Glu Val His Asn Ala Lys Thr Lys Pro
275 280 285

Arg Glu Glu Gln Tyr Ala Ser Thr Tyr Arg Val Val Ser Val Leu Thr
290 295 300

Val Leu His Gln Asp Trp Leu Asn Gly Lys Glu Tyr Lys Cys Lys Val
305 310 315 320

Ser Asn Lys Ala Leu Pro Ala Pro Ile Glu Lys Thr Ile Ser Lys Ala
325 330 335

Lys Gly Gln Pro Arg Glu Pro Gln Val Cys Thr Leu Pro Pro Ser Arg
340 345 350

Asp Glu Leu Thr Lys Asn Gln Val Ser Leu Trp Cys Leu Val Lys Gly
355 360 365

Phe Tyr Pro Ser Asp Ile Ala Val Glu Trp Glu Ser Asn Gly Gln Pro
370 375 380

Glu Asn Asn Tyr Lys Thr Thr Pro Pro Val Leu Asp Ser Asp Gly Ser
385 390 395 400

Phe Phe Leu Tyr Ser Lys Leu Thr Val Asp Lys Ser Arg Trp Gln Gln
405 410 415

Gly Asn Val Phe Ser Cys Ser Val Met His Glu Ala Leu His Asn His
420 425 430

Tyr Thr Gln Lys Ser Leu Ser Leu Ser Pro His His His His His His
435 440 445

His His
450

<210> 32
<211> 116
<212> PRT
<213> Artificial

<220>
<223> an artificially synthesized sequence

<400> 32

Glu Val Gln Leu Val Glu Ser Gly Gly Gly Leu Val Gln Pro Gly Lys
1 5 10 15

Ser Leu Lys Leu Ser Cys Glu Ala Ser Gly Phe Thr Phe Ser Gly Tyr
20 25 30

Gly Met His Trp Val Arg Gln Ala Pro Gly Arg Gly Leu Glu Ser Val
35 40 45

Ala Tyr Ile Thr Ser Ser Ser Ile Asn Ile Lys Tyr Ala Asp Ala Val
50 55 60

Lys Gly Arg Phe Thr Val Ser Arg Asp Asn Ala Lys Asn Leu Leu Phe
65 70 75 80

Leu Gln Met Asn Ile Leu Lys Ser Glu Asp Thr Ala Met Tyr Tyr Cys
85 90 95

Ala Arg Phe Asp Trp Asp Lys Asn Tyr Trp Gly Gln Gly Thr Met Val
100 105 110

Thr Val Ser Ser
115

<210> 33
<211> 444
<212> PRT
<213> Artificial

<220>
<223> an artificially synthesized sequence

<400> 33

Glu Val Gln Leu Val Glu Ser Gly Gly Gly Leu Val Gln Pro Gly Lys
1 5 10 15

Ser Leu Lys Leu Ser Cys Glu Ala Ser Gly Phe Thr Phe Ser Gly Tyr
20 25 30

Gly Met His Trp Val Arg Gln Ala Pro Gly Arg Gly Leu Glu Ser Val
35 40 45

Ala Tyr Ile Thr Ser Ser Ser Ile Asn Ile Lys Tyr Ala Asp Ala Val
50 55 60

Lys Gly Arg Phe Thr Val Ser Arg Asp Asn Ala Lys Asn Leu Leu Phe
65 70 75 80

```
Leu Gln Met Asn Ile Leu Lys Ser Glu Asp Thr Ala Met Tyr Tyr Cys
                85                  90                  95

Ala Arg Phe Asp Trp Asp Lys Asn Tyr Trp Gly Gln Gly Thr Met Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Ala Ala Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Ala Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335
```

```
Lys Ala Lys Gly Gln Pro Arg Glu Pro Gln Val Tyr Thr Leu Pro Pro
            340                 345                 350

Ser Arg Cys Glu Leu Thr Lys Asn Gln Val Ser Leu Ser Cys Ala Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Val Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro
        435                 440


<210>  34
<211>  222
<212>  PRT
<213>  Artificial

<220>
<223>  an artificially synthesized sequence

<400>  34

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Glu Met His Trp Ile Arg Gln Pro Pro Gly Gln Gly Leu Glu Trp Ile
        35                  40                  45

Gly Ala Ile Asp Pro Lys Thr Gly Asp Thr Ala Tyr Ser Gln Lys Phe
    50                  55                  60

Lys Gly Arg Val Thr Leu Thr Ala Asp Lys Ser Thr Ser Thr Ala Tyr
65                  70                  75                  80

Met Glu Leu Ser Ser Leu Thr Ser Glu Asp Thr Ala Val Tyr Tyr Cys
                85                  90                  95
```

```
Thr Arg Phe Tyr Ser Tyr Thr Tyr Trp Gly Gln Gly Thr Leu Val Thr
            100                 105                 110

Val Ser Ser Arg Thr Val Ala Ala Pro Ser Val Phe Ile Phe Pro Pro
        115                 120                 125

Ser Asp Glu Gln Leu Lys Ser Gly Thr Ala Ser Val Val Cys Leu Leu
    130                 135                 140

Asn Asn Phe Tyr Pro Arg Glu Ala Lys Val Gln Trp Lys Val Asp Asn
145                 150                 155                 160

Ala Leu Gln Ser Gly Asn Ser Gln Glu Ser Val Thr Glu Gln Asp Ser
                165                 170                 175

Lys Asp Ser Thr Tyr Ser Leu Ser Ser Thr Leu Thr Leu Ser Lys Ala
            180                 185                 190

Asp Tyr Glu Lys His Lys Val Tyr Ala Cys Glu Val Thr His Gln Gly
        195                 200                 205

Leu Ser Ser Pro Val Thr Lys Ser Phe Asn Arg Gly Glu Cys
    210                 215                 220


<210>  35
<211>  214
<212>  PRT
<213>  Artificial

<220>
<223>  an artificially synthesized sequence

<400>  35

Asp Ile Gln Met Thr Gln Ser Pro Ser Ser Leu Pro Ala Ser Leu Gly
1               5                   10                  15

Asp Arg Val Thr Ile Asn Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Asn Lys Leu Ala Asp Gly Val Pro Ser Arg Phe Ser Gly
    50                  55                  60

Ser Gly Ser Gly Arg Asp Ser Ser Phe Thr Ile Ser Ser Leu Glu Ser
65                  70                  75                  80
```

Glu Asp Ile Gly Ser Tyr Tyr Cys Gln Gln Tyr Tyr Asn Tyr Pro Trp
85 90 95

Thr Phe Gly Pro Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 36
<211> 443
<212> PRT
<213> Artificial

<220>
<223> an artificially synthesized sequence

<400> 36

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Glu Met His Trp Ile Arg Gln Pro Pro Gly Gln Gly Leu Glu Trp Ile
35 40 45

Gly Ala Ile Asp Pro Lys Thr Gly Asp Thr Ala Tyr Ser Gln Lys Phe
50 55 60

Lys Gly Arg Val Thr Leu Thr Ala Asp Lys Ser Thr Ser Thr Ala Tyr

```
65                  70                  75                  80

Met Glu Leu Ser Ser Leu Thr Ser Glu Asp Thr Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Leu Arg Gly Gly Pro Lys Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Ala Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320
```

Val Ser Asn Lys Ala Leu Pro Ala Pro Ile Glu Lys Thr Ile Ser Lys
325 330 335

Ala Lys Gly Gln Pro Arg Glu Pro Gln Val Tyr Thr Leu Pro Pro Ser
340 345 350

Arg Asp Glu Leu Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Tyr Leu Asp Ser Asp Gly
385 390 395 400

Ser Phe Phe Leu Tyr Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Glu Ser Leu Ser Leu Ser Pro
435 440

<210> 37
<211> 219
<212> PRT
<213> Artificial

<220>
<223> an artificially synthesized sequence

<400> 37

Asp Ile Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Arg Asn Thr Tyr Leu His Trp Tyr Gln Gln Lys Pro Gly Gln Ala
35 40 45

Pro Arg Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn

85 90 95

Thr His Val Pro Pro Thr Phe Gly Gln Gly Thr Lys Leu Glu Ile Lys
100 105 110

Arg Thr Val Ala Ala Pro Ser Val Phe Ile Phe Pro Pro Ser Asp Glu
115 120 125

Gln Leu Lys Ser Gly Thr Ala Ser Val Val Cys Leu Leu Asn Asn Phe
130 135 140

Tyr Pro Arg Glu Ala Lys Val Gln Trp Lys Val Asp Asn Ala Leu Gln
145 150 155 160

Ser Gly Asn Ser Gln Glu Ser Val Thr Glu Gln Asp Ser Lys Asp Ser
165 170 175

Thr Tyr Ser Leu Ser Ser Thr Leu Thr Leu Ser Lys Ala Asp Tyr Glu
180 185 190

Lys His Lys Val Tyr Ala Cys Glu Val Thr His Gln Gly Leu Ser Ser
195 200 205

Pro Val Thr Lys Ser Phe Asn Arg Gly Glu Cys
210 215

<210> 38
<211> 444
<212> PRT
<213> Artificial

<220>
<223> an artificially synthesized sequence

<400> 38

Glu Val Gln Leu Val Glu Ser Gly Gly Gly Leu Val Gln Pro Gly Lys
1 5 10 15

Ser Leu Lys Leu Ser Cys Glu Ala Ser Gly Phe Thr Phe Ser Gly Tyr
20 25 30

Gly Met His Trp Val Arg Gln Ala Pro Gly Arg Gly Leu Glu Ser Val
35 40 45

Ala Tyr Ile Thr Ser Ser Ser Ile Asn Ile Lys Tyr Ala Asp Ala Val
50 55 60

Lys Gly Arg Phe Thr Val Ser Arg Asp Asn Ala Lys Asn Leu Leu Phe
65 70 75 80

Leu Gln Met Asn Ile Leu Lys Ser Glu Asp Thr Ala Met Tyr Tyr Cys
85 90 95

Ala Arg Phe Asp Trp Asp Lys Asn Tyr Trp Gly Gln Gly Thr Met Val
100 105 110

Thr Val Ser Ser Ala Ser Thr Lys Gly Pro Ser Val Phe Pro Leu Ala
115 120 125

Pro Ser Ser Lys Ser Thr Ser Gly Gly Thr Ala Ala Leu Gly Cys Leu
130 135 140

Val Lys Asp Tyr Phe Pro Glu Pro Val Thr Val Ser Trp Asn Ser Gly
145 150 155 160

Ala Leu Thr Ser Gly Val His Thr Phe Pro Ala Val Leu Gln Ser Ser
165 170 175

Gly Leu Tyr Ser Leu Ser Ser Val Val Thr Val Pro Ser Ser Ser Leu
180 185 190

Gly Thr Gln Thr Tyr Ile Cys Asn Val Asn His Lys Pro Ser Asn Thr
195 200 205

Lys Val Asp Lys Lys Val Glu Pro Lys Ser Cys Asp Lys Thr His Thr
210 215 220

Cys Pro Pro Cys Pro Ala Pro Glu Leu Arg Gly Gly Pro Lys Val Phe
225 230 235 240

Leu Phe Pro Pro Lys Pro Lys Asp Thr Leu Met Ile Ser Arg Thr Pro
245 250 255

Glu Val Thr Cys Val Val Val Asp Val Ser His Glu Asp Pro Glu Val
260 265 270

Lys Phe Asn Trp Tyr Val Asp Gly Val Glu Val His Asn Ala Lys Thr
275 280 285

Lys Pro Arg Glu Glu Gln Tyr Ala Ser Thr Tyr Arg Val Val Ser Val
290 295 300

Leu Thr Val Leu His Gln Asp Trp Leu Asn Gly Lys Glu Tyr Lys Cys
305 310 315 320

Lys Val Ser Asn Lys Ala Leu Pro Ala Pro Ile Glu Lys Thr Ile Ser

```
                    325                 330                 335

Lys Ala Lys Gly Gln Pro Arg Glu Pro Gln Val Tyr Thr Leu Pro Pro
            340                 345                 350

Ser Arg Lys Glu Leu Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Tyr Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro
        435                 440
```

## Description

### Technical Field

The present invention relates to pharmaceutical compositions for combined use of multiple T cell-activating agonist antibodies.

### Background Art

With the exception of some cases, cancer is one of the fatal diseases that are difficult to be cured completely. The outcome of treatment of using chemotherapeutic agents, which is the major therapeutic method, is not necessarily good. Heterogeneity among cancer cells per se is not the only factor that makes cancer treatment difficult, and the tumor microenvironment has been suggested to play a major role (Non-patent Document 1). Recently, the possibility of treating unresectable malignant melanoma and such with an anti-CTLA-4 antibody that attenuates suppressor T cells has been suggested (Non-patent Document 2). Furthermore, therapeutic effects achieved by inhibitory antibodies against PD-1 and PD-L1, which are immune checkpoint molecules besides CTLA-4, have also been reported (Non-patent Document 3). These findings have suggested that tumor immunostimulation may form the basis of a new strategy for cancer treatment.

It is understood that activation of T cells which have important roles in tumor immunity is done through two signals: 1) binding of a T cell receptor (TCR) to an antigenic peptide presented by major histocompatibility complex (MHC) class I molecules and activation of the TCR; and 2) binding of a costimulatory molecule on the surface of T cells to the ligands on the antigen-presenting cells and activation of the costimulatory molecule. Furthermore, activation of costimulatory molecules belonging to the tumor necrosis factor receptor superfamily (TNFRSF), including CD137 (4-1BB), on the surface of T cells has been described to be important for T cell activation (Non-patent Document 4).

TNFRSF includes molecules such as CD137, CD40, OX40, RANK, and GITR. CD137 has been reported to be expressed not only on the surface of T cells but also on the surface of other immune cells such as dendritic cells (DC), B cells, NK cells, and neutrophils (Non-patent Document 5).

CD137 agonist antibodies have already been demonstrated to show anti-tumor effects, and this has been shown experimentally in mouse models to be mainly due to activation of CD8-positive T cells and NK cells (Non-patent Document 6). However, side effects due to nonspecific hepatotoxicity of CD137 agonist antibodies have become a problem clinically and non-clinically, and development of pharmaceutical agents has not advanced as expected (Non-patent Document 7). The main cause of the side effects has been suggested to involve binding to the Fcγ receptor *via* the antibody constant region (Non-patent Document 8).

In the development of CD137 agonist antibodies, toxicity of the antibodies when used as a single agent at high doses becomes a matter of extreme concern. Therefore, clinical development of low dosage and use in combination with other pharmaceutical agents is mainly progressing at the present (Non-patent Document 9). Pharmaceutical agents for use in combination include anti-CD20 antibodies, anti-EGFR antibodies, anti-PD-1 antibodies, and such. While use in combination with a CD3 agonist antibody can be expected to increase T cell activation (Non-patent Document 10), there are no actual examples of clinical trials. Modified T cells having a chimeric antigen receptor that has a cancer antigen-recognizing site in the extracellular domain, and CD3 and CD137 signaling regions incorporated into the intracellular domain (CAR-Ts) are known to enhance the prolonged effect of drug efficacy, but toxicity such as grade 4 lymphopenia has been reported in clinical trials (Non-patent Document 11). More specifically, while enhancement of drug efficacy can be expected in simple combined use of multiple T cell-activating agonists, an increased risk of toxicity is also readily envisaged.

Bispecific antibodies are characterized in that they have at least two binding domains, and their molecular morphology is already well known to those skilled in the art. Among them, molecules in which one of the two binding domains binds specifically to a cancer surface antigen and the second binding domain binds to a T cell surface antigen CD3 have also been constructed (Non-patent Document 12). Such bispecific single-chain antibodies have been shown to exert an antitumor effect by activating T cells in a cancer antigen-dependent manner.

Glypican 3 (GPC3) is a protein that belongs to the glypican family, i.e., a group of heparan sulfate proteoglycans bound to cell surface *via* glycosylphosphatidylinositol (Non-patent Document 13). Glypicans play an important role in cell proliferation, differentiation, and migration. GPC3 is expressed in 70% or more of hepatoma tissues obtained by surgical excision or biopsy, and is hardly or not at all expressed in neighboring nonneoplastic hepatic lesions and most adult tissues (Non-patent Document 14). Furthermore, patients with high expression of hepatoma tissue GPC3 have been reported to have a poor prognosis (Non-patent Document 15), and GPC3 is considered to be a promising target molecule for hepatoma.

Prior Art Documents

[Non-patent Documents]

[Non-patent Document 1] Hanahan, Cell, 2011, 144, 646-74
[Non-patent Document 2] Prieto, Clin Cancer Res. 2012, 18, 2039-47
[Non-patent Document 3] Hamid, 2013, Expert Opin. Biol. Ther., 6, 847-61
[Non-patent Document 4] Summers, 2012, Nat Rev Immunol, 12, 339-51
[Non-patent Document 5] Vinay, 2011, Cellular & Molecular Immunology, 8, 281-284
[Non-patent Document 6] Houot, 2009, Blood, 114, 3431-8
[Non-patent Document 7] Ascierto, 2010, Semin Oncol, 37, 508-16; Dubrot, 2010, Cancer Immunol Immunother, 59, 1223-33
[Non-patent Document 8] Schabowsky, 2009, Vaccine, 28, 512-22
[Non-patent Document 9] Yonezawa, 2015, Clin. Cancer Res. Apr.23
[Non-patent Document 10] Son, 2004, J Immunol Methods, 286, 187-201
[Non-patent Document 11] Porter, N ENGL J MED, 2011, 365, 725-733
[Non-patent Document 12] Brandl, 2007, Cancer Immunol Immunother, 56, 1551-63
[Non-patent Document 13] Filmus, J. Clin. Invest., 2001, 108, 497-501
[Non-patent Document 14] Zhu-Zu-W, Gut, 2001, 48, 558-564; Yamauchi, Mod. Pathol., 2005, 18, 1591-1598
[Non-patent Document 15] Yorita, Liver Int., 2010, 1, 120-131

Summary of the Invention

[Problems to be Solved by the Invention]

The present invention was achieved in view of the above circumstances. An objective of the present invention is to provide pharmaceutical compositions comprising as an active ingredient a multispecific antibody having the effect of accumulating T cells at a local tumor site, for use in combination with a tumor necrosis factor (TNF) receptor superfamily agonist antibody; pharmaceutical compositions comprising the agonist antibody as an active ingredient for use in combination with the multispecific antibody; and methods for inducing the effect of the agonist antibody specifically towards a tumor tissue by using the multispecific antibody in combination with the agonist antibody.

[Means for Solving the Problems]

The present inventors discovered that by combining a multispecific antibody having the effect of accumulating T cells at a local tumor site with a tumor necrosis factor (TNF) receptor superfamily agonist antibody, their combined use can unexpectedly reduce side effects such as liver injury that are observed when the agonist antibody is prescribed alone and achieve effective therapeutic effects, and thereby completed the present invention.
More specifically, the present invention provides the following:

[1] a pharmaceutical composition that comprises as an active ingredient a multispecific antibody comprising:

(1) a cancer-specific antigen-binding domain,
(2) a CD3-binding domain, and
(3) a domain comprising an Fc region having decreased Fcγ receptor-binding activity,

for use in combination with a tumor necrosis factor (TNF) receptor superfamily agonist antibody;
[2] the pharmaceutical composition of [1], for reducing or eliminating a side effect associated with treatment with the tumor necrosis factor (TNF) receptor superfamily agonist antibody;
[3] the pharmaceutical composition of [1] or [2], which is a composition for use in the treatment of cancer;
[4] the pharmaceutical composition of any one of [1] to [3], wherein the tumor necrosis factor (TNF) receptor superfamily agonist antibody is an agonist antibody against CD137;
[5] the pharmaceutical composition of [4], wherein the agonist antibody against CD137 comprises an Fc region, wherein the Fc region is an antibody Fc region having an increased binding activity to an inhibitory Fcγ receptor;
[6] the pharmaceutical composition of any one of [2] to [5], wherein the side effect is mainly liver injury;
[7] the pharmaceutical composition of any one of [1] to [6], wherein the multispecific antibody is a bispecific antibody;

[8] the pharmaceutical composition of any one of [1] to [7], wherein an effect of the tumor necrosis factor (TNF) receptor superfamily agonist antibody is a cytotoxicity-inducing effect;
[9] the pharmaceutical composition of any one of [1] to [8], which is administered simultaneously with the tumor necrosis factor (TNF) receptor superfamily agonist antibody;
[10] the pharmaceutical composition of any one of [1] to [8], which is administered separately from the tumor necrosis factor (TNF) receptor superfamily agonist antibody;
[11] a pharmaceutical composition that comprises a tumor necrosis factor (TNF) receptor superfamily agonist antibody as an active ingredient, for use in combination with a multispecific antibody comprising:

(1) a cancer-specific antigen-binding domain,
(2) a CD3-binding domain, and
(3) a domain comprising an Fc region having decreased Fcγ receptor-binding activity;

[12] the pharmaceutical composition of [11], for inducing an effect of the tumor necrosis factor (TNF) receptor superfamily agonist antibody specifically towards a tumor tissue;
[13] the pharmaceutical composition of [11] or [12], which is a composition for use in the treatment of cancer;
[14] the pharmaceutical composition of any one of [11] to [13], wherein the tumor necrosis factor (TNF) receptor superfamily agonist antibody is an agonist antibody against CD137;
[15] the pharmaceutical composition of [14], wherein the agonist antibody against CD137 comprises an Fc region, wherein the Fc region is an antibody Fc region having an increased binding activity to an inhibitory Fcγ receptor;
[16] the pharmaceutical composition of any one of [11] to [15], wherein the multispecific antibody is a bispecific antibody;
[17] the pharmaceutical composition of any one of [11] to [16], wherein an effect of the multispecific antibody is an effect of reducing or eliminating a side effect associated with treatment with the tumor necrosis factor (TNF) receptor superfamily agonist antibody;
[18] the pharmaceutical composition of [17], wherein the side effect is mainly liver injury;
[19] the pharmaceutical composition of any one of [11] to [18], which is administered simultaneously with the multispecific antibody;
[20] the pharmaceutical composition of any one of [11] to [18], which is administered separately from the multispecific antibody;
[21] a pharmaceutical composition that comprises a combination of a multispecific antibody comprising:

(1) a cancer-specific antigen-binding domain,
(2) a CD3-binding domain, and
(3) a domain comprising an Fc region having decreased Fcγ receptor-binding activity,

and a tumor necrosis factor (TNF) receptor superfamily agonist antibody;
[22] the pharmaceutical composition of [21], wherein the pharmaceutical composition is a combination preparation;
[23] the pharmaceutical composition of [21], wherein the multispecific antibody and the agonist antibody are used in combination;
[24] the pharmaceutical composition of [23], wherein the multispecific antibody and the agonist antibody are administered simultaneously or sequentially;
[25] the pharmaceutical composition of [23], wherein the multispecific antibody and the agonist antibody are administered separately;
[26] the pharmaceutical composition of any one of [21] to [25], for reducing or eliminating a side effect associated with treatment with the tumor necrosis factor (TNF) receptor superfamily agonist antibody;
[27] the pharmaceutical composition of any one of [21] to [26], which is a composition for use in the treatment of cancer;
[28] the pharmaceutical composition of any one of [21] to [27], wherein the tumor necrosis factor (TNF) receptor superfamily agonist antibody is an agonist antibody against CD137;
[29] the pharmaceutical composition of [28], wherein the CD137 agonist antibody comprises an Fc region, wherein the Fc region is an antibody Fc region having an increased binding activity to an inhibitory Fcγ receptor;
[30] the pharmaceutical composition of any one of [26] to [29], wherein the side effect is mainly liver injury;
[31] the pharmaceutical composition of any one of [21] to [30], wherein the multispecific antibody is a bispecific antibody;
[32] a method for inducing an effect of a tumor necrosis factor (TNF) receptor superfamily agonist antibody specifically towards a tumor tissue, which comprises using a multispecific antibody comprising:

(1) a cancer-specific antigen-binding domain, and

(2) a CD3-binding domain; and

accumulating T cells in the tumor tissue expressing the cancer-specific antigen, wherein the tumor necrosis factor (TNF) receptor superfamily agonist antibody is used in combination with the multispecific antibody;
[33] the method of [32], for reducing or eliminating a side effect associated with the agonist antibody treatment; and
[34] the method of [32] or [33], wherein the tumor necrosis factor (TNF) receptor superfamily agonist antibody is an agonist antibody against CD137.

The present invention also provides the following:

[35] a method for reducing or eliminating a side effect associated with treatment with a tumor necrosis factor (TNF) receptor superfamily agonist antibody, a method for enhancing the effect of the treatment, or a method for treating or preventing cancer, which comprises the step of administering an effective amount of the pharmaceutical composition of any one of [1] to [8];
[36] the pharmaceutical composition of any one of [1] to [8], for use in reducing or eliminating a side effect associated with treatment with the tumor necrosis factor (TNF) receptor superfamily agonist antibody, in enhancing the effect of the treatment, or in treating or preventing cancer;
[37] use of the pharmaceutical composition of any one of [1] to [8] in the production of an agent for reducing or eliminating a side effect associated with treatment with the tumor necrosis factor (TNF) receptor superfamily agonist antibody, an agent for enhancing the effect of the treatment, or an agent for treating or preventing cancer; and
[38] an agent for reducing or eliminating a side effect associated with treatment with a tumor necrosis factor (TNF) receptor superfamily agonist antibody, an agent for enhancing the effect of the treatment, or an agent for treating or preventing cancer, which comprises the pharmaceutical composition of any one of [1] to [8].

The present invention also provides the following:

[39] a method for inducing or enhancing an immune response in a tumor tissue-specific manner, or a method for treating or preventing cancer, which comprises the step of administering an effective amount of the pharmaceutical composition of any one of [11] to [31];
[40] the pharmaceutical composition of any one of [11] to [31], for use in inducing or enhancing an immune response in a tumor tissue-specific manner, or in treating or preventing cancer;
[41] use of the pharmaceutical composition of any one of [11] to [31] in the production of an agent for inducing or enhancing an immune response in a tumor tissue-specific manner, or an agent for treating or preventing cancer; and
[42] an agent for inducing or enhancing an immune response in a tumor tissue-specific manner, or an agent for treating or preventing cancer, which comprises the pharmaceutical composition of any one of [11] to [31].

## Brief Description of the Drawings

Fig. 1 presents a graph showing the results of measuring tumor volume over time in mice to which an anti-mouse CD137 antibody or an anti-human GPC3/anti-mouse CD3 bispecific antibody was administered or in mice to which these antibodies were administered in combination in Example 3.
Fig. 2 presents a graph showing the results of measuring blood AST in mice to which an anti-mouse CD137 antibody or an anti-human GPC3/anti-mouse CD3 bispecific antibody was administered or in mice to which these antibodies were administered in combination in Example 4.
Fig. 3 presents a graph showing the results of measuring blood ALT in mice to which an anti-mouse CD137 antibody or an anti-human GPC3/anti-mouse CD3 bispecific antibody was administered or in mice to which these antibodies were administered in combination in Example 4.
Fig. 4 presents a graph showing the results of measuring blood TBIL in mice to which an anti-mouse CD137 antibody or an anti-human GPC3/anti-mouse CD3 bispecific antibody was administered or in mice to which these antibodies were administered in combination in Example 4.
Fig. 5 presents photographs showing the results of hematoxylin-eosin (HE) staining or anti-mouse CD3 immunostaining using liver tissue specimens collected from mice to which an anti-mouse CD137 antibody or an anti-human GPC3/anti-mouse CD3 bispecific antibody was administered or from mice to which these antibodies were administered in combination in Example 4.
Fig. 6 presents a graph showing the results of measuring the respective target genes by real-time PCR using RNAs isolated from liver tissues collected from mice to which an anti-mouse CD137 antibody or an anti-human GPC3/anti-mouse CD3 bispecific antibody was administered or from mice to which these antibodies were administered in combination in Example 4.

Fig. 7 shows the relationship between the amino acid residues constituting the Fc regions of IgG1, IgG2, IgG3, and IgG4, and Kabat's EU numbering (herein, also referred to as EU INDEX).

Fig. 8 presents a graph showing the results of measuring tumor volume over time in mice to which an anti-mouse CD137 antibody or an anti-human GPC3/anti-mouse CD3 bispecific antibody was administered or in mice to which these antibodies were administered in combination in Example 6.

Fig. 9 presents a graph showing the results of measuring blood ALT in mice to which an anti-mouse CD137 antibody or an anti-human GPC3/anti-mouse CD3 bispecific antibody was administered or in mice to which these antibodies were administered in combination in Example 7.

## Mode for Carrying Out the Invention

As an embodiment of the present invention, a pharmaceutical composition that comprises a combination of a multispecific antibody comprising: (1) a cancer-specific antigen-binding domain, (2) a CD3-binding domain, and (3) a domain comprising an Fc region having decreased Fcγ receptor-binding activity, and a tumor necrosis factor (TNF) receptor superfamily agonist antibody is provided. The pharmaceutical composition can be used, for example, for inducing or enhancing immune responses in a tumor tissue-specific manner or for treating or preventing cancer in a subject. Use of the pharmaceutical composition enables induction of an immune response in a tumor tissue-specific manner and treatment or prevention of cancer with reduction or elimination of side effects.

In the present invention, a "pharmaceutical composition that comprises a combination of a multispecific antibody comprising: (1) a cancer-specific antigen-binding domain, (2) a CD3-binding domain, and (3) a domain comprising an Fc region having decreased Fcγ receptor-binding activity, and a tumor necrosis factor (TNF) receptor superfamily agonist antibody" refers to a pharmaceutical composition in which the multispecific antibody and the agonist antibody have been combined for simultaneous, separate, or sequential administration. The pharmaceutical composition of the present invention can be provided in the form of a combination preparation containing both the multispecific antibody and the agonist antibody. Alternatively, an agent comprising the multispecific antibody and an agent comprising the agonist antibody can be provided separately, and these agents can be used simultaneously, separately, or sequentially. Furthermore, the invention can be provided as a kit composed of an agent comprising the multispecific antibody and an agent comprising the agonist antibody.

In the above-mentioned pharmaceutical compositions, when the multispecific antibody and the agonist antibody are included and provided in separate agents, the dosage forms of these agents may be the same or different. For example, the two may have different dosage forms from each other, and either one of them may be any one of a parenteral preparation, an injection, a drip, and an intravenous drip; or the two may have the same dosage form of any one of a parenteral preparation, an injection, a drip, and an intravenous drip. Furthermore, the above-mentioned pharmaceutical composition can be further combined with one or more different types of preparations.

As an embodiment of the present invention, the present invention provides a pharmaceutical composition that comprises as an active ingredient a multispecific antibody comprising: (1) a cancer-specific antigen-binding domain, (2) a CD3-binding domain, and (3) a domain comprising an Fc region having decreased Fcγ receptor-binding activity, for use in combination with a tumor necrosis factor (TNF) receptor superfamily agonist antibody. The pharmaceutical composition can be used, for example, for reducing or eliminating side effects associated with the agonist antibody treatment, for enhancing effects of the treatment, or for treating or preventing cancer in a subject. When a pharmaceutical composition of the present invention, which comprises the multispecific antibody as an active ingredient, is used in combination with the agonist agent, it may be administered simultaneously with the agonist antibody, or it may be administered before or after administration of the agonist antibody. When the multispecific antibody is administered before or after administration of the agonist antibody, the timing of its administration may be optimized by measuring the residual concentration of the agonist antibody in a subject. The concentration can be determined using samples collected from the subject by an immunological assay known to those skilled in the art, such as ELISA described below.

As an embodiment of the present invention, the present invention provides a pharmaceutical composition that comprises a tumor necrosis factor (TNF) receptor superfamily agonist antibody as an active ingredient, for use in combination with a multispecific antibody comprising: (1) a cancer-specific antigen-binding domain, (2) a CD3-binding domain, and (3) a domain comprising an Fc region having decreased Fcγ receptor-binding activity. The pharmaceutical composition can be used, for example, for inducing or enhancing immune responses in a tumor tissue-specific manner, or for treating or preventing cancer in a subject. Use of the pharmaceutical composition enables one to induce an immune response in a tumor tissue-specific manner, and to treat or prevent cancer with reduction or elimination of side effects. When a pharmaceutical composition comprising the agonist antibody as an active ingredient is used in combination with the multispecific antibody, it may be administered simultaneously with the multispecific antibody, or it may be administered before or after administration of the multispecific antibody. When the agonist antibody is administered before or after administration of the multispecific antibody, the timing of its administration may be optimized by measuring the residual concentration of the multispecific antibody in a subject. The concentration can be determined using samples collected from the subject by an immunological assay known to those skilled in the art, such as ELISA described below.

Furthermore, the present invention relates to methods for treating or preventing cancer, which comprise administering a pharmaceutical composition of the present invention to a patient in need of treatment. The present invention also relates to a kit for use in a method of the present invention, which comprises the above-mentioned multispecific antibody and the above-mentioned agonist antibody of the present invention. In addition, the present invention relates to use of the above-mentioned multispecific antibody and the above-mentioned agonist antibody of the present invention in the production of a pharmaceutical composition of the present invention (for example, a pharmaceutical composition for treating or preventing cancer). Furthermore, the present invention relates to pharmaceutical compositions of the present invention for use in the methods of the present invention.

As a non-limiting embodiment of the present invention, an agent is provided for reducing or preventing side effects associated with treatment with a tumor necrosis factor (TNF) receptor superfamily agonist antibody, which comprises as an active ingredient a multispecific antibody comprising:

(1) a cancer-specific antigen-binding domain,
(2) a CD3-binding domain, and
(3) a domain comprising an Fc region having decreased Fcγ receptor-binding activity.

Whether side effects are caused in administering a multispecific antibody of the present invention or a tumor necrosis factor (TNF) receptor superfamily agonist antibody of the present invention, or in using them in combination can be confirmed by a well-known method. Herein, "side effects" refer to clinical, medical, physical, physiological, and/or biochemical effects that are observed and/or measured in a patient undergoing disease treatment and are not part of the intended outcome of the treatment. Generally, the aforementioned effects are not desirable in terms of the physical condition of and/or comfort for the patient undergoing treatment, health risk for the patient undergoing treatment, and/or treatment tolerability for the patient undergoing treatment. Specific examples of the side effects include neutropenia, leukopenia, bleeding (gastrointestinal hemorrhage, pulmonary hemorrhage, intracerebral hemorrhage, etc.), hypertension, neurotoxicity, fatigue and malaise, decreased appetite, nausea, stomatitis, alopecia, thrombocytopenia, positive proteinuria, shock, anaphylaxis, gastrointestinal perforation, fistula, delayed wound healing, thromboembolism, hypertensive encephalopathy, hypertensive crisis, reversible posterior leukoencephalopathy syndrome, nephrotic syndrome, myelosuppression, infection, congestive heart failure, pneumonitis, thrombotic microangiopathy, interstitial lung disease, hepatic dysfunction, increased blood bilirubin, dysgeusia, rash, increased blood creatinine, and such. One can assess whether side effects are reduced by use in combination by comparing the frequency, grade, and such of side effects observed in use in combination with those observed in single-agent administration.

As a non-limiting embodiment, the present invention provides a pharmaceutical composition for enhancing effects of the treatment with a tumor necrosis factor (TNF) receptor superfamily agonist antibody, which comprises as an active ingredient a multispecific antibody comprising:

(1) a cancer-specific antigen-binding domain,
(2) a CD3-binding domain, and
(3) a domain comprising an Fc region having decreased Fcγ receptor-binding activity. As a non-limiting embodiment, the present invention provides a pharmaceutical composition for enhancing effects of the treatment with a multispecific antibody comprising: (1) a cancer-specific antigen-binding domain, (2) a CD3-binding domain, and (3) a domain comprising an Fc region having decreased Fcγ receptor-binding activity, which comprises as an active ingredient a tumor necrosis factor (TNF) receptor superfamily agonist antibody.

Herein, "enhancing effects of the treatment" means increasing the response rate of the treatment, reducing the amount of an agent administered for the treatment, and/or shortening the period of treatment with an agent.

As a non-limiting embodiment, the present invention provides a method for enhancing an immune response in a subject, which comprises administering to the subject an effective amount of a tumor necrosis factor (TNF) receptor superfamily agonist antibody in combination with an effective amount of a multispecific antibody comprising: (1) a cancer-specific antigen-binding domain, (2) a CD3-binding domain, and (3) a domain comprising an Fc region having decreased Fcγ receptor-binding activity.

As a non-limiting embodiment, the present invention provides a method for treating or preventing cancer in a subject, which comprises administering to the subject an effective amount of a tumor necrosis factor (TNF) receptor superfamily agonist antibody in combination with an effective amount of a multispecific antibody comprising: (1) a cancer-specific antigen-binding domain, (2) a CD3-binding domain, and (3) a domain comprising an Fc region having decreased Fcγ receptor-binding activity.

As a non-limiting embodiment, the present invention provides a method for reducing side effects associated with treatment with a tumor necrosis factor (TNF) receptor superfamily agonist antibody in a subject, which comprises administering to the subject an effective amount of a multispecific antibody comprising: (1) a cancer-specific antigen-binding domain, (2) a CD3-binding domain, and (3) a domain comprising an Fc region having decreased Fcγ receptor-binding activity in combination with the aforementioned agonist antibody.

As a non-limiting embodiment, the present invention provides a method for inducing an effect of a tumor necrosis factor (TNF) receptor superfamily agonist antibody specifically towards a tumor tissue, wherein the agonist antibody is used in combination with a multispecific antibody comprising: (1) a cancer-specific antigen-binding domain and (2) a CD3-binding domain, in which the method comprises accumulating T cells in the tumor tissue expressing the cancer-specific antigen by using the multispecific antibody.

While it is not intended to be restricted to a particular theory or particularly limited, since the multispecific antibody comprising: (1) a cancer-specific antigen-binding domain and (2) a CD3-binding domain has the effect of accumulating T cells at a local tumor tissue site containing cells that express the cancer-specific antigen, infiltration of T cells into normal tissues can be avoided, and effects (for example, T cell activation) of the tumor necrosis factor (TNF) receptor superfamily agonist antibody used in combination with the multispecific antibody may be induced specifically towards the tumor tissue. Inducing T cells in the local tumor tissue site enables one to reduce only side effects of the tumor necrosis factor (TNF) receptor superfamily agonist antibody, without attenuating antitumor effects of the agonist antibody.

Furthermore, as a non-limiting embodiment, the present invention provides a method for inducing an effect of a tumor necrosis factor (TNF) receptor superfamily agonist antibody specifically towards a tumor tissue, which comprises using a multispecific antibody comprising: (1) a cancer-specific antigen-binding domain and (2) a CD3-binding domain, and removing from normal tissues all or some of the T cells that have infiltrated into the normal tissues, wherein the agonist antibody is used in combination with the multispecific antibody.

As a non-limiting embodiment, the present invention provides a method for inducing an effect of a tumor necrosis factor (TNF) receptor superfamily agonist antibody specifically towards a tumor tissue, which comprises using a multispecific antibody comprising: (1) a cancer-specific antigen-binding domain and (2) a CD3-binding domain, and accumulating all or some of the T cells that have infiltrated into normal tissues at the local tumor tissue site expressing the cancer-specific antigen, wherein the agonist antibody is used in combination with the multispecific antibody.

The "tumor necrosis factor (TNF) receptor superfamily agonist antibody used in combination with the multispecific antibody" in the above-mentioned embodiments means that the multispecific antibody and the agonist antibody can be administered simultaneously, separately, or sequentially. Alternatively, the multispecific antibody and the agonist antibody may be included and provided in the form of a combination preparation. Alternatively, an agent comprising the multispecific antibody and an agent comprising the agonist antibody can be provided separately, and these agents can be used simultaneously, separately, or sequentially. Furthermore, it can be provided as a kit composed of an agent comprising the multispecific antibody and an agent comprising the agonist antibody.

In the above-mentioned pharmaceutical compositions, when the multispecific antibody and the agonist antibody are included and provided in separate agents, the dosage forms of these agents may be the same or different. For example, the two may have different dosage forms from each other, and either one of them may be any one of a parenteral preparation, an injection, a drip, and an intravenous drip; or the two may have the same dosage form of any one of a parenteral preparation, an injection, a drip, or an intravenous drip. Furthermore, the above-mentioned pharmaceutical composition can be further combined with one or more different types of preparations.

## Multispecific antibody

The "multispecific antibody" of the present invention may comprise: (1) a cancer-specific antigen-binding domain, (2) a CD3-binding domain, and (3) a domain comprising an Fc region having decreased Fcγ receptor-binding activity of the present invention, and its structure is not limited. The antibody may further comprise, besides these domains, a peptide or protein having a length of about five amino acids or more. The multispecific antibodies of the present invention are not limited to peptides and proteins derived from a living organism, and for example, they may be polypeptides produced from artificially designed sequences, or they may be any of naturally-occurring polypeptides, synthetic polypeptides, recombinant polypeptides, and such.

By including the above-mentioned two binding domains, it becomes possible for the multispecific antibody to crosslink CD3-expressing T cells with cancer-specific antigen-expressing cells, to specifically induce an immune response in these cells or tumor tissues containing these cells, and to induce excellent (specific) cytotoxic effects on the cancer-specific antigen-expressing cells or tumor tissues containing these cells. The cancer-specific antigen-binding domain and the CD3-binding domain of the present invention can be appropriately selected from regions that bind specifically to the whole or a portion of the cancer-specific antigen described below or CD3 antigen, respectively. These binding domains can be linked directly by peptide bonds or bound *via* linkers.

Multispecific antibodies of the present invention can be produced using known methods described below. For example, when (1) F(ab')$_2$ as a cancer-specific antigen-binding domain, (2) F(ab')$_2$ as a CD3-binding domain, and additionally (3) a domain comprising an Fc region having decreased Fcγ receptor-binding activity are used, and when the antigen-binding domains described in (1) and (2) and the Fc region-containing domain described in (3) are directly linked by peptide bonds, the linked polypeptides will form an antibody structure. Such antibodies can be produced by purification from the undermentioned hybridoma culture medium, and also by purifying antibodies from the culture medium of desired host cells that stably carry polynucleotides encoding polypeptides constituting the antibody.

In addition to the linkers exemplified above, linkers with peptide tags such as His tag, HA tag, myc tag, and FLAG tag may also be suitably used as the linkers to be employed when connecting each of the domains *via* linkers. Furthermore, hydrogen bonding, disulfide bonding, covalent bonding, ionic interaction, or the property of mutual binding as a result of combination thereof may be suitably used. For example, the affinity between antibody CH1 and CL may be used, and Fc regions derived from the undermentioned multispecific antibodies may also be used for heterologous Fc region association.

Preferably, the "domain comprising an Fc region having decreased Fcγ receptor-binding activity" in the multispecific antibody of the present invention comprises an FcRn-binding domain contained in an antibody Fc region. As a method for extending the blood half-life of a protein administered to a living body, the method of adding an FcRn-binding domain of an antibody to the protein of interest and utilizing the function of FcRn-mediated recycling is well known.

For example, a molecular form in which the scFv of an antibody against a cancer antigen is linked to the scFv of an antibody against the CD3 epsilon chain *via* a short polypeptide linker (such as Blinatumomab) is a modified low-molecular-weight antibody molecule without an Fc region. Therefore, the problem is that its blood half-life after administration to a patient is significantly shorter than that of an IgG-type antibody conventionally used as a therapeutic antibody. Since the multispecific antibody of the present invention comprises an antibody Fc region, it has a longer blood half-life compared to the aforementioned modified antibody molecule without an Fc region. Furthermore, by having a decreased Fcγ receptor-binding activity, the "Fc region" in the multispecific antibody of the present invention is able to suppress side effects produced by immunostimulation such as cytokine release caused by the crosslinking between Fcγ receptor-expressing cells and T cell receptor complex-expressing cells.

In the present invention, the "FcRn-binding domain" is not particularly limited as long as it has binding activity to FcRn, and examples include antibody variable regions, Fab and antibody Fc regions whose antigens are FcRn, and fragments thereof. A preferred embodiment of the present invention includes antibody Fc regions or fragments containing an FcRn-binding region of an Fc region. Herein, for example, an Fc region derived from a naturally-occurring IgG may be used as the "Fc region". A naturally-occurring IgG means a polypeptide that comprises the same amino acid sequence as an IgG found in nature, and belongs to a class of antibodies substantially encoded by immunoglobulin gamma genes. A naturally-occurring human IgG means, for example, a naturally-occurring human IgG1, a naturally-occurring human IgG2, a naturally-occurring human IgG3, or a naturally-occurring human IgG4. Naturally-occurring IgGs also include mutants and such that naturally generate therefrom. A plurality of allotype sequences that result from genetic polymorphism have been described in Sequences of Proteins of Immunological Interest, NIH Publication No. 91-3242 for the human IgG1, human IgG2, human IgG3, and human IgG4 antibody constant region, and any of the sequences may be used in the present invention. In particular, the amino acid sequence of positions 356 to 358 according to EU numbering may be DEL or EEM for the human IgG1 sequence.

Existing antibody Fc regions are, for example, IgA1, IgA2, IgD, IgE, IgG1, IgG2, IgG3, IgG4, and IgM-type Fc regions. For example, an Fc region derived from a naturally-occurring human IgG antibody can be used as the antibody Fc region of the present invention. Fc regions derived from a constant region of a naturally-occurring IgG, or more specifically, a constant region derived from a naturally-occurring human IgG1 (SEQ ID NO: 1), a constant region derived from a naturally-occurring human IgG2 (SEQ ID NO: 2), a constant region derived from a naturally-occurring human IgG3 (SEQ ID NO: 3), and a constant region derived from a naturally-occurring human IgG4 (SEQ ID NO: 4), can be used as an Fc region of the present invention. Mutants and such that naturally generate therefrom are also included in the naturally-occurring IgG constant regions.

Such antibody Fc regions can be suitably obtained, for example, by partial digestion of antibodies such as monoclonal antibodies using a protease such as pepsin, then adsorption of the resulting fragments onto a protein A column or a protein G column, and subsequent elution using an appropriate elution buffer and such. The protease is not particularly limited as long as it can digest an antibody such as a monoclonal antibody by appropriately establishing the enzyme reaction conditions such as pH, and examples include pepsin and ficin.

The isotype of an antibody is determined by the structure of the constant region. The constant region of isotypes IgG1, IgG2, IgG3, and IgG4 is called Cγ1, Cγ2, Cγ3, and Cγ4, respectively. The amino acid sequences of polypeptides constituting the Fc regions of human Cγ1, Cγ2, Cγ3, and Cγ4 are exemplified in SEQ ID NOs: 5, 6, 7, and 8. The relationship between amino acid residues constituting each of these amino acid sequences and Kabat's EU numbering (herein, also referred to as EU INDEX) is shown in Fig. 7. Unless otherwise stated herein, the residues in the immunoglobulin heavy chain are numbered according to the EU INDEX in the method described in Sequences of Proteins of Immunological Interest (5th ed., Public Health Service, National Institutes of Health, Bethesda, MD (1991)).

An Fc region refers to a region that excludes $F(ab')_2$ which contains two light chains and two heavy chains containing part of the constant region between the CH1 domain and the CH2 domain such that the disulfide bonds between the chains are formed between the two heavy chains. Fc regions disclosed herein can be obtained suitably by partially digesting the IgG1, IgG2, IgG3, or IgG4 monoclonal antibodies or the like using a protease such as pepsin, and then re-eluting fractions adsorbed to the protein A column. The protease is not particularly limited as long as it can digest a full-length antibody in a restrictive manner to produce $F(ab')_2$ by appropriately establishing the enzyme reaction conditions such as pH. Such proteases include, for example, pepsin and ficin.

An Fc region having decreased Fcγ receptor-binding activity is particularly preferred as the Fc region contained in the multispecific antibody of the present invention. Here, an Fcγ receptor (herein, also denoted as Fcγ receptor, FcγR, or FcgR) refers to a receptor that can bind to the Fc region of IgG1, IgG2, IgG3, or IgG4, and includes all members belonging to the family of proteins substantially encoded by Fcγ receptor genes. In humans, this family includes, but is not limited to, FcγRI (CD64) including isoforms FcγRIa, FcγRIb, and FcγRIc; FcγRII (CD32) including isoforms FcγRIIa (including allotypes H131 (type H) and R131 (type R), FcγRIIb (including FcγRIIb-1 and FcγRIIb-2), and FcγRIIc; and FcγRIII (CD16) including isoforms FcγRIIIa (including allotypes V158 and F158) and FcγRIIIb (including allotypes FcγRIIIb-NA1 and FcγRIIIb-NA2); as well as any undiscovered human FcγRs, and FcγR isoforms or allotypes. FcγRs include, but are not limited to, those derived from humans, mice, rats, rabbits, and monkeys, and may be derived from any organism. Mouse FcγRs include, but are not limited to, FcγRI (CD64), FcγRII (CD32), FcγRIII (CD16), and FcγRIII-2 (CD16-2), as well as any undiscovered mouse FcγRs, and FcγR isoforms or allotypes. Suitable examples of such Fcγ receptors include human FcγRI (CD64), FcγRIIa (CD32), FcγRIIb (CD32), FcγRIIIa (CD16) and/or FcγRIIIb (CD16).

Activating receptors which carry an immunoreceptor tyrosine-based activation motif (ITAM) and inhibitory receptors which carry an immunoreceptor tyrosine-based inhibitory motif (ITIM) are present among FcγRs. FcγRs are categorized into activating FcγRs: FcγRI, FcγRIIa R, FcγRIIa H, FcγRIIIa, and FcγRIIIb, and inhibitory FcγR: FcγRIIb.

The polynucleotide sequence and amino acid sequence of FcγRI are shown in NM_000566.3 and NP_000557.1, respectively; the polynucleotide sequence and amino acid sequence of FcγRIIa are shown in BC020823.1 and AAH20823.1, respectively; the polynucleotide sequence and amino acid sequence of FcγRIIb are shown in BC146678.1 and AAI46679.1, respectively; the polynucleotide sequence and amino acid sequence of FcγRIIIa are shown in BC033678.1 and AAH33678.1, respectively; and the polynucleotide sequence and amino acid sequence of FcγRIIIb are shown in BC128562.1 andAAI28563.1, respectively (RefSeq accession number). There are two types of gene polymorphisms for FcγRIIa, where the amino acid at position 131 of FcγRIIa is substituted into histidine (type H) or arginine (type R) (J. Exp. Med, 172, 19-25, 1990). Furthermore, there are two types of gene polymorphisms for FcγRIIb, where the amino acid at position 232 of FcγRIIb is substituted with isoleucine (type I) or threonine (type T) (Arthritis. Rheum. 46: 1242-1254 (2002)). In addition, there are two types of gene polymorphisms for FcγRIIIa, where the amino acid at position 158 of FcγRIIIa is substituted with valine (type V) or phenylalanine (type F) (J. Clin. Invest. 100(5): 1059-1070 (1997)). There are also two types of gene polymorphisms for FcγRIIIb, which are type NA1 and type NA2 (J. Clin. Invest. 85: 1287-1295 (1990)).

Whether the binding activity to an Fcγ receptor is decreased can be confirmed by well-known methods such as FACS, ELISA format, screening by Amplified Luminescent Proximity Homogeneous Assay (ALPHA), surface plasmon resonance (SPR)-based BIACORE method, and others (Proc. Natl. Acad. Sci. USA (2006) 103(11), 4005-4010).

ALPHA screening is performed with ALPHA technology which uses two beads, a donor and an acceptor bead, based on the following principle. Luminescent signals are detected only when molecules bound to donor beads interact biologically with molecules bound to the acceptor beads, and the two beads are in close proximity to each other. The laser-excited photosensitizer within the donor beads converts ambient oxygen to excited-state singlet oxygen. Singlet oxygen is dispersed around the donor beads; and when it reaches the adjacent acceptor beads, a chemiluminescent reaction is induced within the beads, and light is ultimately emitted. When molecules bound to the donor beads do not interact with molecules bound to the acceptor beads, the chemiluminescent reaction does not take place because singlet oxygen produced by the donor beads does not reach the acceptor beads.

For example, when the antibody of the present invention contains an Fc region, an antibody having a wild-type Fc region and an antibody having a mutant Fc region produced by adding amino acid mutations to change the binding to an Fcγ receptor are prepared, a biotinylated antibody is bound to the donor beads, and an Fcγ receptor tagged with glutathione S transferase (GST) is bond to the acceptor beads. In the presence of the antibody having a mutant Fc region, the antibody having a wild-type Fc region interacts with the Fcγ receptor and produces 520-620 nm signals. When the antibody having a mutant Fc region is untagged, it competes with the antibody having a wild-type Fc region for interaction with the Fcγ receptor. The relative binding affinity can be determined by quantifying the decrease in fluorescence observed as a result of the competition. Biotinylation of antibodies using Sulfo-NHS-biotin and such is well known. As a method for tagging an Fcγ receptor with GST, the method of expressing the Fcγ receptor and GST in a cell carrying a vector that can express a fusion gene produced by fusing a polynucleotide encoding the Fcγ receptor in frame with a GST-encoding polynucleotide, and purifying it using a glutathione column can be appropriately adopted. The obtained signals are suitably analyzed, for example, by fitting them into a one-site competition model that utilizes a non-linear regression analysis with software such as GRAPHPAD PRISM (GraphPad, San Diego).

One of the substances (ligand) observed for interaction is immobilized onto a gold thin film on a sensor chip, and by shining light from the reverse side of the sensor chip so that total reflection takes place at the interface between the gold thin film and glass, a portion with reduced reflection intensity is formed in part of the reflected light (SPR signal). The other substance (analyte) observed for interaction is made to flow over the sensor chip surface; and when the ligand binds to the analyte, the mass of the immobilized ligand molecule increases and the refractive index of the solvent on the sensor chip surface changes. The position of the SPR signal shifts as a result of this change in the refractive index (reversely, the signal position returns if this binding dissociates). The Biacore system shows the amount of shift mentioned above, or more specifically the time variable of mass, by plotting the change in mass on the sensor chip surface on the vertical axis as the measurement data (sensorgram). Kinetic parameters such as association rate constant (ka) and dissociation rate constant (kd) are determined from the curve in the sensorgram, and the affinity (KD) is determined from the ratio of these constants. In the BIACORE method, a method for measuring inhibition is also suitably used. An example of the method for measuring inhibition is described in Proc. Natl. Acad. Sci USA (2006) 103 (11): 4005-4010.

Herein, "decreased Fcγ receptor-binding activity" means that, for example, based on the above-described analytical method, the binding activity of the test antibody is 50% or less, preferably 45% or less, 40% or less, 35% or less, 30% or less, 20% or less, 15% or less, or particularly preferably 10% or less, 9% or less, 8% or less, 7% or less, 6% or less, 5% or less, 4% or less, 3% or less, 2% or less, or 1% or less as compared to the binding activity of the control antibody containing an Fc region.

For the control antibody, antibodies having, for example, a domain comprising an Fc region of a monoclonal IgG1, IgG2, IgG3, or IgG4 antibody may be suitably used. The structures of the Fc regions are shown in SEQ ID NO: 1 (A is added to the N terminus of RefSeq Accession No. AAC82527.1), SEQ ID NO: 2 (A is added to the N terminus of RefSeq Accession No. AAB59393.1), SEQ ID NO: 3 (A is added to the N terminus of RefSeq Accession No. CAA27268.1), and SEQ ID NO: 4 (A is added to the N terminus of RefSeq Accession No. AAB59394.1). Further, when an antibody containing a mutant of an Fc region of a particular antibody isotype is used as the test substance, the effect of a mutation possessed by the mutant on the Fcγ receptor-binding activity is tested by using as a control an antibody having an Fc region of an antibody of that particular isotype. In this way, antibodies containing an Fc region mutant whose binding activity toward the Fcγ receptor verified to be decreased are suitably produced.

Examples of such mutants include mutants with a 231A-2385 deletion (WO 2009/011941), or C226S, C229S, P238S, (C220S) (J. Rheumatol (2007) 34, 11), C226S, C229S (Hum. Antibod. Hybridomas (1990) 1(1), 47-54), C226S, C229S, E233P, L234V, or L235A (Blood (2007) 109, 1185-1192) mutants, where the amino acids are specified by EU numbering.

That is, suitable examples include antibodies having an Fc region in which any of the amino acids at positions 220, 226, 229, 231, 232, 233, 234, 235, 236, 237, 238, 239, 240, 264, 265, 266, 267, 269, 270, 295, 296, 297, 298, 299, 300, 325, 327, 328, 329, 330, 331, and 332 specified according to EU numbering has been substituted in the amino acids constituting the Fc region of an antibody of a specific isotype. The isotype of the antibody from which the Fc region originates is not particularly limited, and the Fc region derived from an IgG1, IgG2, IgG3, or IgG4 monoclonal antibody can be used appropriately, and the Fc region derived from a naturally-occurring human IgG1 antibody is suitably used.

For example, antibodies having an Fc region that comprises any substitution specified below based on EU numbering from among amino acids constituting the IgG1 antibody Fc region (wherein the number indicates the position of the amino acid residue specified according to EU numbering, the one-letter amino acid code positioned before the number indicates the amino acid residue before the substitution, and the one-letter amino acid code positioned after the number indicates the amino acid residue before the substitution):

(a) L234F, L235E, P331S
(b) C226S, C229S, P238S
(c) C226S, C229S
(d) C226S, C229S, E233P, L234V, L235A;

or an Fc region lacking the amino acid sequence of positions 231 to 238 from among amino acids constituting the IgG1 antibody Fc region may be appropriately used.

Furthermore, antibodies having an Fc region that comprises any substitution specified below based on EU numbering from among amino acids constituting the IgG2 antibody Fc region (wherein the number indicates the position of the amino acid residue specified according to EU numbering, the one-letter amino acid code positioned before the number indicates the amino acid residue before the substitution, and the one-letter amino acid code positioned after the number indicates the amino acid residue before the substitution):

(e) H268Q, V309L, A330S, P331 S

(f) V234A
(g) G237A
(h) V234A, G237A
(i) A235E, G237A
(j) V234A, A235E, G237A

may be appropriately used.

Furthermore, antibodies having an Fc region that comprises any substitution specified below based on EU numbering from among amino acids constituting the IgG3 antibody Fc region (wherein the number indicates the position of the amino acid residue specified according to EU numbering, the one-letter amino acid code positioned before the number indicates the amino acid residue before the substitution, and the one-letter amino acid code positioned after the number indicates the amino acid residue before the substitution):

(k) F241A
(1) D265A
(m) V264A

may be appropriately used.

Furthermore, antibodies having an Fc region that comprises any substitution specified below based on EU numbering from among amino acids constituting the IgG4 antibody Fc region (wherein the number indicates the position of the amino acid residue specified according to EU numbering, the one-letter amino acid code positioned before the number indicates the amino acid residue before the substitution, and the one-letter amino acid code positioned after the number indicates the amino acid residue before the substitution):

(n) L235A, G237A, E318A
(o) L235E
(p) F234A, L235A

may be appropriately used.

Other preferred examples include antibodies having an Fc region in which any of the amino acids at positions 233, 234, 235, 236, 237, 327, 330, and 331 specified according to EU numbering in the amino acids constituting the Fc region of a naturally-occurring human IgG1 antibody is substituted with amino acids of corresponding EU numbering in the corresponding IgG2 or IgG4.

Other preferred examples suitably include antibodies having an Fc region in which any one or more of the amino acids at positions 234, 235, and 297 specified according to EU numbering in the amino acids constituting the Fc region of a naturally-occurring human IgG1 antibody are substituted with other amino acids. The type of amino acid present after substitution is not particularly limited, but an antibody having an Fc region in which any one or more of the amino acids at positions 234, 235, and 297 are substituted with alanine is particularly preferred.

Other preferred examples suitably include antibodies having an Fc region in which the amino acid at position 265 specified according to EU numbering in the amino acids constituting an IgG1 antibody Fc region is substituted with another amino acid. The type of amino acid present after substitution is not particularly limited, but an antibody having an Fc region in which the amino acid at position 265 is substituted with alanine is particularly preferred.

The "cancer-specific antigen-binding domain" and "CD3-binding domain" (hereinafter, these two binding domains are collectively referred to as antigen-binding domains) comprised in the multispecific antibodies of the present invention refer to regions that bind specifically to the whole or a portion of their respective antigens which are a cancer-specific antigen or CD3, and an example of the binding domain is a region that contains the antigen-binding region of an antibody. When the molecular weight of the antigen is large, the antigen-binding region of the antibody can bind only to a particular portion of the antigen. This particular portion is called an epitope. The antigen-binding domain may be provided by one or more variable domains of an antibody. Preferably, an antigen-binding domain comprises an antibody light chain variable region (VL) and an antibody heavy chain variable region (VH). Suitable examples of such antigen-binding domains include "single chain Fv (scFv)", "single chain antibody", "Fv", "single chain Fv2 (scFv2)", "Fab", "$F(ab')_2$", and such.

Herein, a "cancer-specific antigen" refers to an antigen expressed by cancer cells, which enables one to distinguish between cancer cells and healthy cells; and for example, it includes antigens that are expressed as cells become malignant, or abnormal sugar chains that appear on protein molecules or cell surface when cells become cancerous. Specific examples include ALK receptor (pleiotrophin receptor); pleiotrophin; KS 1/4 pancreas carcinoma antigen; ovarian carcinoma antigen (CA125); prostatic acid phosphate; prostate-specific antigen (PSA); melanoma-associated antigen p97; melanoma antigen gp75; high molecular weight melanoma antigen (HMW-MAA); prostate-specific membrane antigen; carcinoembryonic antigen (CEA); polymorphic epithelial mucin antigen; human milk fat globule antigen; colorectal tumor-associated antigens such as CEA, TAG-72, CO17-1A, GICA 19-9, CTA-1, and LEA; Burkitt's lymphoma antigen-38.13; CD19; human B-lymphoma antigen-CD20; CD33; melanoma-specific antigens such as ganglioside GD2, ganglioside GD3, ganglioside GM2, and ganglioside GM3; tumor-specific transplantation type cell-surface antigen (TSTA); virus-induced tumor antigens including T antigen and envelope antigens of DNA tumor viruses and RNA tumor viruses; CEA of colon; oncofetal antigens such as5T4 oncofetal trophoblast glycoprotein and bladder tumor oncofetal antigen; α-fetoprotein; differentiation antigens such as human lung carcinoma antigens L6 and L20; antigens of fibrosarcoma; human leukemia T cell antigen-Gp37; neoglycoprotein; sphingolipids; breast cancer antigens such as EGFR (epidermal growth factor receptor); NY-BR-16; NY-BR-16 and HER2 antigen (p185HER2); polymorphic epithelial mucin (PEM); malignant human lymphocyte antigen-APO-1; differentiation antigens such as I antigen found in fetal erythrocytes; primary endoderm I antigen found in adult erythrocytes; preimplantation embryos; I(Ma) found in gastric cancer; M18 and M39 found in mammary epithelium; SSEA-1, VEP8, VEP9, Myl, and VIM-D5 found in myeloid cells; D156-22 found in colorectal cancer; TRA-1-85 (blood group H); SCP-1 found in testis and ovarian cancer; C14 found in colon cancer; F3 found in lung cancer; AH6 found in gastric cancer; Y hapten; Ley found in embryonal carcinoma cells; TL5 (blood group A); EGF receptor found in A431 cells; E1 series (blood group B) found in pancreatic cancer; FC10.2 found in embryonal carcinoma cells; gastric cancer antigen; CO-514 (blood group Lea) found in adenocarcinomas; NS-10 found in adenocarcinomas; CO-43 (blood group Leb); G49 found in EGF receptor of A431 cells; MH2 (blood group ALeb/Ley) found in colon cancer; 19.9 found in colon cancer; gastric cancer mucins; T5A7 found in myeloid cells; R24 found in melanoma; 4.2, GD3, D1.1, OFA-1, GM2, OFA-2, GD2, and M1:22:25:8 found in embryonal carcinoma cells as well as SSEA-3 and SSEA-4 found in 4 to 8-cell stage embryos; subcutaneous T cell lymphoma antigen; MART-1 antigen; sialyl Tn (STn) antigen; colon cancer antigen NY-CO-45; lung cancer antigen NY-LU-12 variant A; adenocarcinoma antigen ART1; paraneoplastic associated brain-testis-cancer antigen (onconeuronal antigen MA2; paraneoplastic neuronal antigen); Neuro-oncological ventral antigen 2 (NOVA2); hemocyte carcinoma antigen gene 520; tumor-associated antigen CO-029; tumor-associated antigens MAGE-C1 (cancer/testis antigen CT7), MAGE-B1 (MAGE-XP antigen), MAGE-B2 (DAM6), MAGE-2, MAGE-4a, MAGE-4b and MAGE-X2; Cancer-Testis Antigen (NY-EOS-1); YKL-40, fragments of any of the aforementioned polypeptides, or structures produced by modification thereof (for example, the above-mentioned modified phosphate group or sugar chain); EpCAM; EREG; CA19-9; CA15-3; sialyl SSEA-1(SLX); HER2; PSMA; CEA; and CLEC12A. Cancer-specific antigens which become targets of the cancer-specific antigen-binding domains of the present invention are, in particular, preferably those expressed on cell surface, and examples of such cancer-specific antigens include CD 19, CD20, EGFR, HER2, EpCAM, and EREG.

The multispecific antibody comprising: (1) a cancer-specific antigen-binding domain, (2) a CD3-binding domain, and (3) a domain comprising an Fc region having decreased Fcγ receptor-binding activity of the present invention may use a domain that binds to a "T cell receptor complex" or the "TNF receptor superfamily" in place of the "CD3-binding domain".

As factors belonging to the "TNF receptor superfamily", ligands having a trimeric structure and receptors having a trimeric structure to which the ligands bind, which contribute to activation of various immune cells, are known (Nat. Rev. Immunol., 2012, 12, 339-51). Examples of the factors belonging to the TNF receptor superfamily include CD137, CD40, OX40, CD27, HVEM, RANK, CD30, and GITR.

The "T cell-receptor complex" may be a T cell receptor itself, or an adaptor molecule constituting a T cell-receptor complex together with a T cell receptor. CD3 is suitable as an adaptor molecule.

For the T cell receptor, an epitope to which the T cell receptor binding domain binds may be a variable region or a constant region, but an epitope present in the constant region is preferred. Examples of the constant region sequence include the T cell receptor α chain of RefSeq Accession No. CAA26636.1 (SEQ ID NO: 9), the T cell receptor β chain of RefSeq Accession No. C25777 (SEQ ID NO: 10), the T cell receptor γ1 chain of RefSeq Accession No. A26659 (SEQ ID NO: 11), the T cell receptor γ2 chain of RefSeq Accession No. AAB63312.1 (SEQ ID NO: 12), and the T cell receptor δ chain of RefSeq Accession No. AAA61033.1 (SEQ ID NO: 13).

The "CD3-binding domain" of the present invention may be provided by one or more antibody variable domains. Preferably, the CD3-binding domain includes a light chain variable region (VL) and a heavy chain variable region (VH) of the CD3 antibody. Suitable examples of such CD3-binding domains include "single chain Fv (scFv)", "single chain antibody", "Fv", "single chain Fv 2 (scFv2)", "Fab", "$F(ab')_2$", and such.

The CD3-binding domain of the present invention may be those that bind to any epitope as long as the epitope exists in the γ-chain, δ-chain, or ε-chain sequence constituting human CD3. In the present invention, preferably, a CD3-binding domain that comprises a light chain variable region (VL) of a CD3 antibody and a heavy chain variable region (VH) of a CD3 antibody, and which binds to an epitope present in the extracellular region of the ε chain of the human CD3 complex, is suitably used. For such CD3-binding domain, a CD3-binding domain comprising the light chain variable region (VL) and heavy chain variable region (VH) of the OKT3 antibody (Proc. Natl. Acad. Sci. USA (1980) 77, 4914-4917) or various known CD3 antibodies is suitably used. A CD3-binding domain derived from a CD3 antibody that has the desired properties and is obtained by immunizing a desired animal with the γ-chain, δ-chain, or ε-chain constituting the human CD3 by the above-mentioned method may be appropriately used. Human antibodies and appropriately humanized antibodies as described below may be suitably used as the CD3 antibody that serves as the origin for the CD3-binding domain. For the structure of the CD3-constituting γ-chain, δ-chain, or ε-chain, their polynucleotide sequences are shown in SEQ ID NOs: 14 (NM_000073.2), 16 (NM_000732.4), and 18 (NM_000733.3), and their polypeptide sequences are shown in SEQ ID NOs: 15 (NP_000064.1), 17 (NP_000723.1), and 19 (NP_000724.1) (the RefSeq accession number is shown in parentheses).

TNF receptor superfamily agonist antibody

The "TNF receptor superfamily agonist antibody" of the present invention refers to an antibody that activates cells expressing the TNF receptor superfamily by at least about 5%, specifically at least about 10%, or more specifically at least about 15% when added to the cells, tissues, or living bodies that express the TNF receptor superfamily, where 100% activation is the level of activation achieved by an equimolar amount of a binding partner under physiological conditions. In various specific examples, the TNF receptor superfamily agonist antibody for use as a pharmaceutical composition of the present invention can activate the activity of the cells by at least about 20%, 30%, 40%, 50%, 60%, 70%, 80%, 90%, 100%, 125%, 150%, 175%, 200%, 250%, 300%, 350%, 400%, 450%, 500%, 750%, or 1000%.

Target molecules of the "TNF receptor superfamily agonist antibody" are not particularly limited as long as they are factors that activate cells expressing the TNF receptor superfamily (such as T cells and NK cells), but are preferably factors belonging to the "TNF superfamily" or the "TNF receptor superfamily". As factors belonging to the "TNF superfamily" or the "TNF receptor superfamily", ligands having a trimeric structure and receptors having a trimeric structure to which the ligands bind, which contribute to activation of various immune cells, are known (Nat. Rev. Immunol., 2012, 12, 339-51). Examples of factors belonging to the TNF superfamily or the TNF receptor superfamily include CD137, CD137L, CD40, CD40L, OX40, OX40L, CD27, CD70, HVEM, LIGHT, RANK, RANKL, CD30, CD153, GITR, and GITRL. Preferred factors include, for example, CD137 and CD40. Amore preferred factor may be, for example, CD 137.

Examples of a CD137 agonist antibody include Urelumab (CAS Registry No. 934823-49-1) and various known CD137 agonist antibodies.

As a non-limiting embodiment, the present invention provides a "TNF receptor superfamily agonist antibody" comprising an Fc region, wherein the Fc region has an enhanced binding activity towards an inhibitory Fcγ receptor.

In one embodiment, an FcγR-binding domain having a higher binding activity to inhibitory FcγR than to activating FcγR may be used as the FcγR-binding domain contained in the agonist antibody of the present invention. For example, as the FcγR-binding domain, an FcγR-binding domain having a higher binding activity to FcγRIIb (including FcγRIIb-1 and FcγRIIb-2) than to activating FcγR selected from any one of FcγRI (CD64) including FcγRIa, FcγRIb, and FcγRIc; FcγRIII (CD16) including isoforms FcγRIIIa (including allotypes V158 and F158) and FcγRIIIb (including allotypes FcγRIIIb-NA1 and FcγRIIIb-NA2); and FcγRII (CD32) including isoforms FcγRIIa (including allotypes H131 and R131) and FcγRIIc may be used. Furthermore, for example, as the FcγR-binding domain, an FcγR-binding domain having a higher binding activity to FcγRIIb-1 and/or FcγRIIb-2 than to FcγRIa, FcγRIb, FcγRIc, FcγRIIIa including allotype V158, FcγRIIIa including allotype F158, FcγRIIIb including allotype FcγRIIIb-NA1, FcγRIIIb including allotype FcγRIIIb-NA2, FcγRIIa including allotype H131, FcγRIIa including allotype R131, and/or FcγRIIc may be used, but is not limited thereto.

Furthermore, whether an FcγR-binding domain has a selective binding activity can be assessed by comparing the binding activities to each FcγR, which are determined by the above-described method. For example, it can be assessed by comparing the value (ratio) obtained by dividing the KD value for activating FcγR by the KD value for inhibitory FcγR, that is, the FcγR selectivity index shown in Equation 1 below.

[Equation 1] FcγR selectivity index = KD value for activating FcγR / KD value for inhibitory FcγR

In Equation 1, the KD value for activating FcγR refers to the KD value for any one or more of FcγRIa, FcγRIb, FcγRIc, FcγRIIIa including allotype V158, FcγRIIIa including allotype F158, FcγRIIIb including allotype FcγRIIIb-NA1, FcγRIIIb including allotype FcγRIIIb-NA2, FcγRIIa including allotype H131, FcγRIIa including allotype R131, and/or FcγRIIc; and the KD value for inhibitory FcγR refers to the KD value for FcγRIIb-1 and/or FcγRIIb-2. The activating FcγR and inhibitory FcγR for use in measuring the KD values may be selected from any combination. For example, a value (ratio) obtained by dividing the KD value for FcγRIIa including allotype H131 by the KD value for FcγRIIb-1 and/or FcγRIIb-2 may be used, but is not limited thereto.

The FcγR selectivity index may be for example, 1.2 or more, 1.3 or more, 1.4 or more, 1.5 or more, 1.6 or more, 1.7 or more, 1.8 or more, 1.9 or more, 2 or more, 3 or more, 5 or more, 6 or more, 7 or more, 8 or more, 9 or more, 10 or more, 15 or more, 20 or more, 25 or more, 30 or more, 35 or more, 40 or more, 45 or more, 50 or more, 55 or more, 60 or more, 65 or more, 70 or more, 75 or more, 80 or more, 85 or more, 90 or more, 95 or more, 100 or more, 110 or more, 120 or more, 130 or more, 140 or more, 150 or more, 160 or more, 170 or more, 180 or more, 190 or more, 200 or more, 210 or more, 220 or more, 230 or more, 240 or more, 250 or more, 260 or more, 270 or more, 280 or more, 290 or more, 300 or more, 310 or more, 320 or more, 330 or more, 340 or more, 350 or more, 360 or more, 370 or more, 380 or more, 390 or more, 400 or more, 410 or more, 420 or more, 430 or more, 440 or more, 450 or more, 460 or more, 470 or more, 480 or more, 490 or more, 500 or more, 520 or more, 540 or more, 560 or more, 580 or more, 600 or more, 620 or more, 640 or more, 660 or more, 680 or more, 700 or more, 720 or more, 740 or more, 760 or more, 780 or more, 800 or more, 820 or more, 840 or more, 860 or more, 880 or more, 900 or more, 920 or more, 940 or more, 960 or more, 980 or more, 1000 or more, 1500 or more, 2000 or more, 2500 or more, 3000 or more, 3500 or more, 4000 or more, 4500 or more, 5000 or more, 5500 or more, 6000 or more, 6500 or more, 7000 or more, 7500 or more, 8000 or more, 8500 or more, 9000 or more, 9500 or more, 10000 or more, or 100000 or more, but is not limited thereto.

In one embodiment, an Fc region in which the amino acid at position 238 (EU numbering) is Asp or an Fc region in which the amino acid at position 328 (EU numbering) is Glu in a human IgG (IgG1, IgG2, IgG3, or IgG4) may be preferably used in an agonist antibody of the present invention containing an Fc region, since it has a higher binding activity to FcγRIIb-1 and/or FcγRIIb-2 than to FcγRIa, FcγRIb, FcγRIc, FcγRIIIa including allotype V158, FcγRIIIa including allotype F158, FcγRIIIb including allotype FcγRIIIb-NA1, FcγRIIIb including allotype FcγRIIIb-NA2, FcγRIIa including allotype H131, FcγRIIa including allotype R131, and/or FcγRIIc, as described specifically in WO2013/125667, WO2012/115241, and WO2013/047752. The agonist antibody of the present invention in this embodiment has binding activities to all activating FcγRs and FcγRIIb, wherein the binding activity to the aforementioned FcγRIIb is maintained or enhanced, and/or the binding activities to all activating FcγRs are reduced as compared to a reference antibody containing a naturally-occurring IgG constant region or a naturally-occurring IgG Fc region.

The degree at which "the binding activities to all activating FcγRs are reduced" mentioned above is not limited, and may be for example, 99% or less, 98% or less, 97% or less, 96% or less, 95% or less, 94% or less, 93% or less, 92% or less, 91% or less, 90% or less, 88% or less, 86% or less, 84% or less, 82% or less, 80% or less, 78% or less, 76% or less, 74% or less, 72% or less, 70% or less, 68% or less, 66% or less, 64% or less, 62% or less, 60% or less, 58% or less, 56% or less, 54% or less, 52% or less, 50% or less, 45% or less, 40% or less, 35% or less, 30% or less, 25% or less, 20% or less, 15% or less, 10% or less, 5% or less, 4% or less, 3% or less, 2% or less, 1% or less, 0.5% or less, 0.4% or less, 0.3% or less, 0.2% or less, 0.1% or less, 0.05% or less, 0.01% or less, or 0.005% or less.

The degree at which "the binding activity to FcγRIIb is maintained or enhanced" mentioned above is not limited, and may be for example, 55% or greater, 60% or greater, 65% or greater, 70% or greater, 75% or greater, 80% or greater, 85% or greater, 87% or greater, 88% or greater, 89% or greater, 90% or greater, 91% or greater, 92% or greater, 93% or greater, 94% or greater, 95% or greater, 96% or greater, 97% or greater, 98% or greater, 99% or greater, 99.5% or greater, 100% or greater, 101% or greater, 102% or greater, 103% or greater, 104% or greater, 105% or greater, 106% or greater, 107% or greater, 108% or greater, 109% or greater, 110% or greater, 112% or greater, 114% or greater, 116% or greater, 118% or greater, 120% or greater, 122% or greater, 124% or greater, 126% or greater, 128% or greater, 130% or greater, 132% or greater, 134% or greater, 136% or greater, 138% or greater, 140% or greater, 142% or greater, 144% or greater, 146% or greater, 148% or greater, 150% or greater, 155% or greater, 160% or greater, 165% or greater, 170% or greater, 175% or greater, 180% or greater, 185% or greater, 190% or greater, 195% or greater, 2-fold or greater, 3-fold or greater, 4-fold or greater, 5-fold or greater, 6-fold or greater, 7-fold or greater, 8-fold or greater, 9-fold or greater, 10-fold or greater, 20-fold or greater, 30-fold or greater, 40-fold or greater, 50-fold or greater, 60-fold or greater, 70-fold or greater, 80-fold or greater, 90-fold or greater, 100-fold or greater, 200-fold or greater, 300-fold or greater, 400-fold or greater, 500-fold or greater, 600-fold or greater, 700-fold or greater, 800-fold or greater, 900-fold or greater, 1000-fold or greater, 10000-fold or greater, or 100000-fold or greater.

In one embodiment, in comparison to a reference antibody containing a naturally-occurring IgG constant region or a naturally-occurring IgG Fc region, the FcγRIIb-binding activity of the Fc region-containing agonist antibodies of the present invention may be maintained or enhanced and their binding activities to FcγRIIa (H type) and FcγRIIa (R type) may be reduced. Such antibodies show an improved binding selectivity for FcγRIIb over FcγRIIa. Among them, alterations that improve the binding selectivity for FcγRIIb over FcγRIIa (R type) are preferred, and alterations that improve the binding selectivity for FcγRIIb over FcγRIIa (H type) are more preferred.

The degree at which "the FcγRIIb-binding activity is maintained or enhanced" mentioned above is not limited, and may be for example, 55% or greater, 60% or greater, 65% or greater, 70% or greater, 75% or greater, 80% or greater, 85% or greater, 87% or greater, 88% or greater, 89% or greater, 90% or greater, 91% or greater, 92% or greater, 93% or greater, 94% or greater, 95% or greater, 96% or greater, 97% or greater, 98% or greater, 99% or greater, 99.5% or greater, 100% or greater, 101% or greater, 102% or greater, 103% or greater, 104% or greater, 105% or greater, 106% or greater, 107% or greater, 108% or greater, 109% or greater, 110% or greater, 112% or greater, 114% or greater, 116% or greater, 118% or greater, 120% or greater, 122% or greater, 124% or greater, 126% or greater, 128% or greater, 130% or greater, 132% or greater, 134% or greater, 136% or greater, 138% or greater, 140% or greater, 142% or greater, 144% or greater, 146% or greater, 148% or greater, 150% or greater, 155% or greater, 160% or greater, 165% or greater, 170% or greater, 175% or greater, 180% or greater, 185% or greater, 190% or greater, 195% or greater, 2-fold or greater, 3-fold or greater, 4-fold or greater, 5-fold or greater, 6-fold or greater, 7-fold or greater, 8-fold or greater, 9-fold or greater, 10-fold or greater, 20-fold or greater, 30-fold or greater, 40-fold or greater, 50-fold or greater, 60-fold or greater, 70-fold or greater, 80-fold or greater, 90-fold or greater, 100-fold or greater, 200-fold or greater, 300-fold or greater, 400-fold or greater, 500-fold or greater, 600-fold or greater, 700-fold or greater, 800-fold or greater, 900-fold or greater, 1000-fold or greater, 10000-fold or greater, or 100000-fold or greater.

The degree at which "binding activities to FcγRIIa (H type) and FcγRIIa (R type) are reduced" mentioned above is not limited, and may be for example, 99% or less, 98% or less, 97% or less, 96% or less, 95% or less, 94% or less, 93% or less, 92% or less, 91% or less, 90% or less, 88% or less, 86% or less, 84% or less, 82% or less, 80% or less, 78% or less, 76% or less, 74% or less, 72% or less, 70% or less, 68% or less, 66% or less, 64% or less, 62% or less, 60% or less, 58% or less, 56% or less, 54% or less, 52% or less, 50% or less, 45% or less, 40% or less, 35% or less, 30% or less, 25% or less, 20% or less, 15% or less, 10% or less, 5% or less, 4% or less, 3% or less, 2% or less, 1% or less, 0.5% or less, 0.4% or less, 0.3% or less, 0.2% or less, 0.1% or less, 0.05% or less, 0.01% or less, or 0.005% or less.

As reported in WO2013/047752, examples of preferred amino acid substitutions for such alterations include, but are not limited to an alteration of substituting Gly at position 237 (EU numbering) with Trp, an alteration of substituting Gly at position 237 (EU numbering) with Phe, an alteration of substituting Pro at position 238 (EU numbering) with Phe, an alteration of substituting Asn at position 325 (EU numbering) with Met, an alteration of substituting Ser at position 267 (EU numbering) with Ile, an alteration of substituting Leu at position 328 (EU numbering) with Asp, an alteration of substituting Ser at position 267 (EU numbering) with Val, an alteration of substituting Leu at position 328 (EU numbering) with Trp, an alteration of substituting Ser at position 267 (EU numbering) with Gln, an alteration of substituting Ser at position 267 (EU numbering) with Met, an alteration of substituting Gly at position 236 (EU numbering) with Asp, an alteration of substituting Ala at position 327 (EU numbering) with Asn, an alteration of substituting Asn at position 325 (EU numbering) with Ser, an alteration of substituting Leu at position 235 (EU numbering) with Tyr, an alteration of substituting Val at position 266 (EU numbering) with Met, an alteration of substituting Leu at position 328 (EU numbering) with Tyr, an alteration of substituting Leu at position 235 (EU numbering) with Trp, an alteration of substituting Leu at position 235 (EU numbering) with Phe, an alteration of substituting Ser at position 239 (EU numbering) with Gly, an alteration of substituting Ala at position 327 (EU numbering) with Glu, an alteration of substituting Ala at position 327 (EU numbering) with Gly, an alteration of substituting Pro at position 238 (EU numbering) with Leu, an alteration of substituting Ser at position 239 (EU numbering) with Leu, an alteration of substituting Leu at position 328 (EU numbering) with Thr, an alteration of substituting Leu at position 328 (EU numbering) with Ser, an alteration of substituting Leu at position 328 (EU numbering) with Met, an alteration of substituting Pro at position 331 (EU numbering) with Trp, an alteration of substituting Pro at position 331 (EU numbering) with Tyr, an alteration of substituting Pro at position 331 (EU numbering) with Phe, an alteration of substituting Ala at position 327 (EU numbering) with Asp, an alteration of substituting Leu at position 328 (EU numbering) with Phe, an alteration of substituting Pro at position 271 (EU numbering) with Leu, an alteration of substituting Ser at position 267 (EU numbering) with Glu, an alteration of substituting Leu at position 328 (EU numbering) with Ala, an alteration of substituting Leu at position 328 (EU numbering) with Ile, an alteration of substituting Leu at position 328 (EU numbering) with Gln, an alteration of substituting Leu at position 328 (EU numbering) with Val, an alteration of substituting Lys at position 326 (EU numbering) with Trp, an alteration of substituting Lys at position 334 (EU numbering) with Arg, an alteration of substituting His at position 268 (EU numbering) with Gly, an alteration of substituting His at position 268 (EU numbering) with Asn, an alteration of substituting Ser at position 324 (EU numbering) with Val, an alteration of substituting Val at position 266 (EU numbering) with Leu, an alteration of substituting Pro at position 271 (EU numbering) with Gly, an alteration of substituting Ile at position 332 (EU numbering) with Phe, an alteration of substituting Ser at position 324 (EU numbering) with Ile, an alteration of substituting Glu at position 333 (EU numbering) with Pro, an alteration of substituting Tyr at position 300 (EU numbering) with Asp, an alteration of substituting Ser at position 337 (EU numbering) with Asp, an alteration of substituting Tyr at position 300 (EU numbering) with Gln, an alteration of substituting Thr at position 335 (EU numbering) with Asp, an alteration of substituting Ser at position 239 (EU numbering) with Asn, an alteration of substituting Lys at position 326 (EU numbering) with Leu, an alteration of substituting Lys at position 326 (EU numbering) with He, an alteration of substituting Ser at position 239 (EU numbering) with Glu, an alteration of substituting Lys at position 326 (EU numbering) with Phe, an alteration of substituting Lys at position 326 (EU numbering) with Val, an alteration of substituting Lys at position 326 (EU numbering) with Tyr, an alteration of substituting Ser at position 267 (EU numbering) with Asp, an alteration of substituting Lys at position 326 (EU numbering) with Pro, an alteration of substituting Lys at position 326 (EU numbering) with His, an alteration of substituting Lys at position 334 (EU numbering) with Ala, an alteration of substituting Lys at position 334 (EU numbering) with Trp, an alteration of substituting His at position 268 (EU numbering) with Gln, an alteration of substituting Lys at position 326 (EU numbering) with Gln, an alteration of substituting Lys at position 326 (EU numbering) with Glu, an alteration of substituting Lys at position 326 (EU numbering) with Met, an alteration of substituting Val at position 266 (EU numbering) with He, an alteration of substituting Lys at position 334 (EU numbering) with Glu, an alteration of substituting Tyr at position 300 (EU numbering) with Glu, an alteration of substituting Lys at position 334 (EU numbering) with Met, an alteration of substituting Lys at position 334 (EU numbering) with Val, an alteration of substituting Lys at position 334 (EU numbering) with Thr, an alteration of substituting Lys at position 334 (EU numbering) with Ser, an alteration of substituting Lys at position 334 (EU numbering) with His, an alteration of substituting Lys at position 334 (EU numbering) with Phe, an alteration of substituting Lys at position 334 (EU numbering) with Gln, an alteration of substituting Lys at position 334 (EU numbering) with Pro, an alteration of substituting Lys at position 334 (EU numbering) with Tyr, an alteration of substituting Lys at position 334 (EU numbering) with He, an alteration of substituting Gln at position 295 (EU numbering) with Leu, an alteration of substituting Lys at position 334 (EU numbering) with Leu, an alteration of substituting Lys at position 334 (EU numbering) with Asn, an alteration of substituting His at position 268 (EU numbering) with Ala, an alteration of substituting Ser at position 239 (EU numbering) with Asp, an alteration of substituting Ser at position 267 (EU numbering) with Ala, an alteration of substituting Leu at position 234 (EU numbering) with Trp, an alteration of substituting Leu at position 234 (EU numbering) with Tyr, an alteration of substituting Gly at position 237 (EU numbering) with Ala, an alteration of substituting Gly at position 237 (EU numbering) with Asp, an alteration of substituting Gly at position 237 (EU numbering) with Glu, an alteration of substituting Gly at position 237 (EU numbering) with Leu, an alteration of substituting Gly at position 237 (EU numbering) with Met, an alteration of substituting Gly at position 237 (EU numbering) with Tyr, an alteration of substituting Ala at position 330 (EU numbering) with Lys, an alteration of substituting Ala at position 330 (EU numbering) with Arg, an alteration of substituting Glu at position 233 (EU numbering) with Asp, an alteration of substituting His at position 268 (EU numbering) with Asp, an alteration of substituting His at position 268 (EU numbering) with Glu, an alteration of substituting Lys at position 326 (EU numbering) with Asp, an alteration of substituting Lys at position 326 (EU numbering) with Ser, an alteration of substituting Lys at position 326 (EU numbering) with Thr, an alteration of substituting Val at position 323 (EU numbering) with Ile, an alteration of substituting Val at position 323 (EU numbering) with Leu, an alteration of substituting Val at position 323 (EU numbering) with Met, an alteration of substituting Tyr at position 296 (EU numbering) with Asp, an alteration of substituting Lys at position 326 (EU numbering) with Ala, an alteration of substituting Lys at position 326 (EU numbering) with Asn, and an alteration of substituting Ala at position 330 (EU numbering) with Met.

The above-mentioned alteration may be at a single position, or alterations at two or more positions may be combined. Favorable examples of such alterations are those listed in Tables 14 to 15, Tables 17 to 24, and Tables 26 to 28 of WO2013/047752. An example includes a variant of a human constant region or a human Fc region, in which the amino acid at position 238 (EU numbering) is Asp and the amino acid at position 271 (EU numbering) is Gly in a human IgG (IgG1, IgG2, IgG3, or IgG4), and any one or more amino acids at positions 233, 234, 237, 264, 265, 266, 267, 268, 269, 272, 296, 326, 327, 330, 331, 332, 333, and 396 (EU numbering) may be further substituted. In such cases, a variant of a human constant region or a human Fc region is exemplified by any one or more of:

the amino acid at position 233 being Asp,
the amino acid at position 234 being Tyr,
the amino acid at position 237 being Asp,
the amino acid at position 264 being Ile,
the amino acid at position 265 being Glu,
the amino acid at position 266 being any one of Phe, Met, and Leu,
the amino acid at position 267 being any one of Ala, Glu, Gly, and Gln,
the amino acid at position 268 being Asp or Glu,
the amino acid at position 269 being Asp,
the amino acid at position 272 being any one of Asp, Phe, Ile, Met, Asn, and Gln,
the amino acid at position 296 being Asp,
the amino acid at position 326 being Ala or Asp,
the amino acid at position 327 being Gly,
the amino acid at position 330 being Lys or Arg,
the amino acid at position 331 being Ser,
the amino acid at position 332 being Thr,
the amino acid at position 333 being any one of Thr, Lys, and Arg,
the amino acid at position 396 being any one of Asp, Glu, Phe, Ile, Lys, Leu, Met, Gln, Arg, and Tyr, as indicated by EU numbering, without being limited thereto.

In another embodiment, in comparison to a reference antibody containing a naturally-occurring IgG constant region or a naturally-occurring IgG Fc region, the FcγRIIb-binding activity of the Fc region-containing agonist antibodies of the present invention may be maintained or enhanced, and their binding activities to FcγRIIa (H type) and FcγRIIa (R type) may be reduced.

The degree at which "FcγRIIb-binding activity is maintained or enhanced" mentioned above is not limited, but may be for example, 55% or greater, 60% or greater, 65% or greater, 70% or greater, 75% or greater, 80% or greater, 85% or greater, 87% or greater, 88% or greater, 89% or greater, 90% or greater, 91% or greater, 92% or greater, 93% or greater, 94% or greater, 95% or greater, 96% or greater, 97% or greater, 98% or greater, 99% or greater, 99.5% or greater, 100% or greater, 101% or greater, 102% or greater, 103% or greater, 104% or greater, 105% or greater, 106% or greater, 107% or greater, 108% or greater, 109% or greater, 110% or greater, 112% or greater, 114% or greater, 116% or greater, 118% or greater, 120% or greater, 122% or greater, 124% or greater, 126% or greater, 128% or greater, 130% or greater, 132% or greater, 134% or greater, 136% or greater, 138% or greater, 140% or greater, 142% or greater, 144% or greater, 146% or greater, 148% or greater, 150% or greater, 155% or greater, 160% or greater, 165% or greater, 170% or greater, 175% or greater, 180% or greater, 185% or greater, 190% or greater, 195% or greater, 2-fold or greater, 3-fold or greater, 4-fold or greater, 5-fold or greater, 6-fold or greater, 7-fold or greater, 8-fold or greater, 9-fold or greater, 10-fold or greater, 20-fold or greater, 30-fold or greater, 40-fold or greater, 50-fold or greater, 60-fold or greater, 70-fold or greater, 80-fold or greater, 90-fold or greater, 100-fold or greater, 200-fold or greater, 300-fold or greater, 400-fold or greater, 500-fold or greater, 600-fold or greater, 700-fold or greater, 800-fold or greater, 900-fold or greater, 1000-fold or greater, 10000-fold or greater, or 100000-fold or greater.

The degree at which "binding activities to FcγRIIa (H type) and FcγRIIa (R type) are reduced" mentioned above is not limited, but may be for example, 99% or less, 98% or less, 97% or less, 96% or less, 95% or less, 94% or less, 93% or less, 92% or less, 91% or less, 90% or less, 88% or less, 86% or less, 84% or less, 82% or less, 80% or less, 78% or less, 76% or less, 74% or less, 72% or less, 70% or less, 68% or less, 66% or less, 64% or less, 62% or less, 60% or less, 58% or less, 56% or less, 54% or less, 52% or less, 50% or less, 45% or less, 40% or less, 35% or less, 30% or less, 25% or less, 20% or less, 15% or less, 10% or less, 5% or less, 4% or less, 3% or less, 2% or less, 1% or less, 0.5% or less, 0.4% or less, 0.3% or less, 0.2% or less, 0.1% or less, 0.05% or less, 0.01% or less, or 0.005% or less.

As reported in WO2014/030728, examples of preferred amino acid substitution sites for such alterations include the amino acid at position 238 (EU numbering), and at least one amino acid selected from amino acids at positions 233, 234, 235, 237, 264, 265, 266, 267, 268, 269, 271, 272, 274, 296, 326, 327, 330, 331, 332, 333, 334, 355, 356, 358, 396, 409, and 419 (EU numbering).

More preferably, an example includes but is not limited to Asp at amino acid position 238 (EU numbering), and at least one amino acid selected from the amino acid group of Asp at amino acid position 233; Tyr at amino acid position 234; Phe at amino acid position 235; Asp at amino acid position 237; Ile at amino acid position 264; Glu at amino acid position 265; Phe, Leu, or Met at amino acid position 266; Ala, Glu, Gly, or Gln at amino acid position 267; Asp, Gln, or Glu at position 268; Asp at amino acid position 269; Gly at amino acid position 271; Asp, Phe, Ile, Met, Asn, Pro, or Gln at amino acid position 272; Gln at amino acid position 274; Asp or Phe at amino acid position 296; Ala or Asp at amino acid position 326; Gly at amino acid position 327; Lys, Arg, or Ser at amino acid position 330; Ser at amino acid position 331; Lys, Arg, Ser, or Thr at amino acid position 332; Lys, Arg, Ser, or Thr at amino acid position 333; Arg, Ser, or Thr at amino acid position 334; Ala or Gln at amino acid position 355; Glu at amino acid position 356; Met at amino acid position 358; Ala, Asp, Glu, Phe, Gly, His, He, Lys, Leu, Met, Asn, Gln, Arg, Ser, Thr, Val, Trp, or Tyr at amino acid position 396; Arg at amino acid position 409; and Glu at amino acid position 419 (EU numbering).

In another embodiment, in comparison to a reference antibody containing a naturally-occurring IgG constant region or a naturally-occurring IgG Fc region, the FcγRIIb-binding activity of the Fc region-containing agonist antibodies of the present invention may be maintained and their binding activities to all activating FcγRs, in particular to FcγRIIa (R type), may be reduced.

The degree at which "the FcγRIIb-binding activity is maintained" mentioned above is not limited, but may be for example, 55% or greater, 60% or greater, 65% or greater, 70% or greater, 75% or greater, 80% or greater, 81% or greater, 82% or greater, 83% or greater, 84% or greater, 85% or greater, 86% or greater, 87% or greater, 88% or greater, 89% or greater, 90% or greater, 91% or greater, 92% or greater, 93% or greater, 94% or greater, 95% or greater, 96% or greater, 97% or greater, 98% or greater, 99% or greater, 99.5% or greater, 100% or greater, 101% or greater, 102% or greater, 103% or greater, 104% or greater, 105% or greater, 106% or greater, 107% or greater, 108% or greater, 109% or greater, 110% or greater, 120% or greater, 130% or greater, 140% or greater, 150% or greater, 175% or greater, or 2-fold or greater.

The degree at which "binding activities to all activating FcγR, in particular to FcγRIIa (R type), are reduced" mentioned above is not limited, but may be for example, 74% or less, 72% or less, 70% or less, 68% or less, 66% or less, 64% or less, 62% or less, 60% or less, 58% or less, 56% or less, 54% or less, 52% or less, 50% or less, 45% or less, 40% or less, 35% or less, 30% or less, 25% or less, 20% or less, 15% or less, 10% or less, 5% or less, 4% or less, 3% or less, 2% or less, 1% or less, 0.5% or less, 0.4% or less, 0.3% or less, 0.2% or less, 0.1% or less, 0.05% or less, 0.01% or less, or 0.005% or less.

As reported in WO2014/163101, examples of preferred amino acid substitution sites for such variants may be the amino acid at position 238 (EU numbering), and additionally at least one amino acid selected from amino acids at positions 235, 237, 241, 268, 295, 296, 298, 323, 324, and 330. More preferably, an example includes but is not limited to Asp at amino acid position 238 (EU numbering), and at least one amino acid selected from the amino acid group of Phe at amino acid position 235; Gln or Asp at amino acid position 237; Met or Leu at amino acid position 241; Pro at amino acid position 268; Met or Val at amino acid position 295; Glu, His, Asn, or Asp at amino acid position 296; Ala or Met at amino acid position 298; Ile at amino acid position 323; Asn or His at amino acid position 324; and His or Tyr at amino acid position 330 (EU numbering).

Binding activity of antibodies

The antigen-binding activity of an antibody can be measured using known means (Antibodies A Laboratory Manual. Ed Harlow, David Lane, Cold Spring Harbor Laboratory, 1988). For example, an enzyme linked immunosorbent assay (ELISA), an enzyme immunoassay (EIA), a radioimmunoassay (RIA), FACS, ALPHA screen (Amplified Luminescent Proximity Homogeneous Assay), surface plasmon resonance (SPR)-based BIACORE method, or a fluoroimmunoassay can be used. Methods for assaying the binding activity of an antibody towards an antigen expressed by a cell include, for example, the methods described on pages 359 to 420 in "Antibodies: A Laboratory Manual".

In particular, methods that use a flow cytometer can be suitably used as a method for measuring the binding between an antigen expressed on the surface of cells suspended in buffer or the like and an antibody against the antigen. Flow cytometers that are used include, for example, FACSCanto™ II, FACSAria™, FACSArray™, FACSVantage™ SE, and FACSCalibur™ (the above are from BD Biosciences); and EPICS ALTRA HyPerSort Cytomics FC 500, EPICS XL-MCL ADC EPICS XL ADC, and Cell Lab Quanta/Cell Lab Quanta SC (the above are all from Beckman Coulter).

An example of a suitable method for measuring the binding activity of a test CD 137 antibody toward an antigen includes the method of reacting CD137-expressing cells with a test antibody, and then staining this with an FITC-labeled secondary antibody that recognizes the test antibody, and subsequently taking measurements using FACSCalibur (BD), and analyzing the obtained fluorescence intensity using the CELL QUEST Software (BD).

Antibody

Herein, an "antibody" refers to a naturally occurring immunoglobulin or an immunoglobulin produced by partial or complete synthesis. Antibodies can be isolated from natural sources such as naturally-occurring plasma and serum, or culture supernatants of antibody-producing hybridoma cells. Alternatively, antibodies can be partially or completely synthesized using techniques such as genetic recombination. Suitable examples of the antibodies include antibodies of an immunoglobulin isotype or subclass of such isotype. Known human immunoglobulins include those of the following nine classes (isotypes): IgG1, IgG2, IgG3, IgG4, IgA1, IgA2, IgD, IgE, and IgM. Of these isotypes, antibodies of the present invention include IgG1, IgG2, IgG3, and IgG4.

Methods for producing antibodies having the desired binding activity are known to those skilled in the art, and the antibodies may be obtained as polyclonal or monoclonal antibodies. Monoclonal antibodies derived from mammals may be suitably produced as the antibodies of the present invention. Such mammalian-derived monoclonal antibodies include antibodies produced by hybridomas and antibodies produced by host cells transformed with an expression vector carrying an antibody gene by genetic engineering techniques.

There is no particular limitation on the mammal to be immunized for obtaining antibodies. It is preferable to select the mammal by considering its compatibility with the parent cells to be used in cell fusion for hybridoma production. In general, rabbits, monkeys, and rodents such as mice, rats, and hamsters are suitably used.

The above animals are immunized with a sensitizing antigen by known methods. Generally performed immunization methods include, for example, intraperitoneal or subcutaneous injection of a sensitizing antigen into mammals. Specifically, a sensitizing antigen is appropriately diluted with Phosphate-Buffered Saline (PBS), physiological saline, or the like. If desired, a conventional adjuvant such as Freund's complete adjuvant is mixed with the antigen, and the mixture is emulsified. Then, the sensitizing antigen is administered to a mammal several times at 4- to 21-day intervals. Appropriate carriers may be used in immunization with the sensitizing antigen. In particular, when a low-molecular-weight partial peptide is used as the sensitizing antigen, it is sometimes desirable to couple the sensitizing antigen peptide to a carrier protein such as albumin or keyhole limpet hemocyanin for immunization.

Alternatively, hybridomas producing a desired antibody can be prepared using DNA immunization as mentioned below. DNA immunization is an immunization method that confers immunostimulation by expressing a sensitizing antigen in an animal immunized as a result of administering a vector DNA constructed to allow expression of an antigen protein-encoding gene in the animal. As compared to conventional immunization methods in which a protein antigen is administered to animals to be immunized, DNA immunization is expected to be superior in that:

- immunostimulation can be provided while retaining the structure of a membrane protein; and
- there is no need to purify the antigen for immunization.

In order to prepare a monoclonal antibody of the present invention using DNA immunization, first, a DNA expressing an antigen protein is administered to an animal to be immunized. The antigen protein-encoding DNA can be synthesized by known methods such as PCR. The obtained DNA is inserted into an appropriate expression vector, and then this is administered to an animal to be immunized. Preferably used expression vectors include, for example, commercially-available expression vectors such as cDNA3.1. Vectors can be administered to an organism using conventional methods. For example, DNA immunization is performed by using a gene gun to introduce expression vector-coated gold particles into cells in the body of an animal to be immunized.

After immunizing a mammal as described above, an increase in the titer of an antigen-binding antibody is confirmed in the serum. Then, immune cells are collected from the mammal, and then subjected to cell fusion. In particular, splenocytes are preferably used as immune cells.

A mammalian myeloma cell is used as a cell to be fused with the above-mentioned immune cells. The myeloma cells preferably comprise a suitable selection marker for screening. A selection marker confers characteristics to cells for their survival (or death) under a specific culture condition. Hypoxanthine-guanine phosphoribosyltransferase deficiency (hereinafter abbreviated as HGPRT deficiency) and thymidine kinase deficiency (hereinafter abbreviated as TK deficiency) are known as selection markers. Cells with HGPRT or TK deficiency have hypoxanthine-aminopterin-thymidine sensitivity (hereinafter abbreviated as HAT sensitivity). HAT-sensitive cells cannot synthesize DNA in a HAT selection medium, and are thus killed. However, when the cells are fused with normal cells, they can continue DNA synthesis using the salvage pathway of the normal cells, and therefore they can grow even in the HAT selection medium.

HGPRT-deficient and TK-deficient cells can be selected in a medium containing 6-thioguanine, 8-azaguanine (hereinafter abbreviated as 8AG), or 5'-bromodeoxyuridine. Normal cells are killed because they incorporate these pyrimidine analogs into their DNA. Meanwhile, cells that are deficient in these enzymes can survive in the selection medium, since they cannot incorporate these pyrimidine analogs. In addition, a selection marker referred to as G418 resistance provided by the neomycin-resistant gene confers resistance to 2-deoxystreptamine antibiotics (gentamycin analogs). Various types of myeloma cells that are suitable for cell fusion are known.

For example, myeloma cells including the following cells can be preferably used:

P3(P3x63Ag8.653) (J. Immunol. (1979) 123 (4), 1548-1550);
P3x63Ag8U.1 (Current Topics in Microbiology and Immunology (1978)81, 1-7);
NS-1 (C. Eur. J. Immunol. (1976)6 (7), 511-519);
MPC-11 (Cell (1976) 8 (3), 405-415);
SP2/0 (Nature (1978) 276 (5685), 269-270);
FO (J. Immunol. Methods (1980) 35 (1-2), 1-21);
S194/5.XX0.BU.1 (J. Exp. Med. (1978) 148 (1), 313-323);
R210 (Nature (1979) 277 (5692), 131-133), etc.

Cell fusions between the immunocytes and myeloma cells are essentially carried out using known methods, for example, a method by Kohler and Milstein et al. (Methods Enzymol. (1981) 73: 3-46).

More specifically, cell fusion can be carried out, for example, in a conventional culture medium in the presence of a cell fusion-promoting agent. The fusion-promoting agents include, for example, polyethylene glycol (PEG) and Sendai virus (HVJ). If required, an auxiliary substance such as dimethyl sulfoxide is also added to improve fusion efficiency.

The ratio of immunocytes to myeloma cells may be arbitrarily set, preferably, for example, one myeloma cell for every one to ten immunocytes. Culture media to be used for cell fusions include, for example, media that are suitable for the growth of myeloma cell lines, such as RPMI1640 medium and MEM medium, and other conventional culture medium used for this type of cell culture. In addition, serum supplements such as fetal calf serum (FCS) may be preferably added to the culture medium.

For cell fusion, predetermined amounts of the above immune cells and myeloma cells are mixed well in the above culture medium. Then, a PEG solution (for example, the average molecular weight is about 1,000 to 6,000) prewarmed to about 37°C is added thereto at a concentration of generally 30% to 60% (w/v). The mixed solution is gently mixed to produce desired fusion cells (hybridomas). Then, an appropriate culture medium mentioned above is gradually added to the cells, and this is repeatedly centrifuged to remove the supernatant. Thus, cell fusion agents and such which are unfavorable to hybridoma growth can be removed.

The hybridomas thus obtained can be selected by culture using a conventional selective medium, for example, HAT medium (a culture medium containing hypoxanthine, aminopterin, and thymidine). Culture is continued in the above medium using the HAT medium for a period of time sufficient to kill cells other than the desired hybridomas (non-fused cells). Typically, the period is several days to several weeks. Then, hybridomas producing the desired antibody are screened and singly cloned by conventional limiting dilution methods.

The hybridomas thus obtained can be selected using a selection medium based on the selection marker possessed by the myeloma used for cell fusion. For example, HGPRT- or TK-deficient cells can be selected by culture using the HAT medium (a culture medium containing hypoxanthine, aminopterin, and thymidine). Specifically, when HAT-sensitive myeloma cells are used for cell fusion, cells successfully fused with normal cells can selectively proliferate in the HAT medium. Culture is continued in the above medium using the HAT medium for a period of time sufficient to kill cells other than the desired hybridomas (non-fused cells). Specifically, desired hybridomas can be selected by culture for generally several days to several weeks. Then, hybridomas producing the desired antibody are screened and singly cloned by conventional limiting dilution methods.

Screening and single cloning of desired antibodies can be suitably performed by screening methods based on known antigen-antibody reaction. For example, a desired antibody can be selected by screening using fluorescence activated cell sorting (FACS). FACS is a system that enables measurement of the binding of an antibody to cell surface by analyzing cells contacted with a fluorescent antibody using laser beam, and measuring the fluorescence emitted from individual cells.

To screen for hybridomas that produce a monoclonal antibody of the present invention by FACS, cells that express the antigen bound by the produced antibody are first prepared. Preferred cells used for screening are mammalian cells that are forced to express the antigen. By using mammalian cells that are used as the host cell but have not been transformed as a control, the activity of an antibody to bind to the cell-surface antigen can be selectively detected. Specifically, hybridomas producing a desired monoclonal antibody can be obtained by selecting hybridomas that produce an antibody which binds to cells forced to express the antigen but not to the host cell.

Alternatively, cells expressing the antigen of interest are immobilized and the activity of an antibody to bind to the antigen-expressing cells can be assessed based on the principle of ELISA. For example, antigen-expressing cells are immobilized to the wells of an ELISA plate. Culture supernatants of hybridomas are contacted with the immobilized cells in the wells, and antibodies that bind to the immobilized cells are detected. When the monoclonal antibodies are derived from mouse, antibodies bound to the cells can be detected using an anti-mouse immunoglobulin antibody. Hybridomas producing a desired antibody having the antigen-binding ability are selected by the above screening, and they can be cloned by a limiting dilution method or the like.

Monoclonal antibody-producing hybridomas thus prepared can be passaged in a conventional culture medium. The hybridomas can be stored in liquid nitrogen for a long period.

The above hybridomas are cultured by a conventional method, and desired monoclonal antibodies can be obtained from the culture supernatants. Alternatively, the hybridomas are administered to and grown in compatible mammals, and monoclonal antibodies can be obtained from the ascites. The former method is suitable for obtaining antibodies with high purity.

Antibodies that are encoded by antibody genes cloned from antibody-producing cells such as the above hybridomas can also be preferably used. A cloned antibody gene is inserted into an appropriate vector, and this is introduced into a host to express the antibody encoded by the gene. Methods for isolating antibody genes, inserting the genes into vectors, and transforming host cells have already been established, for example, by Vandamme et al. (Eur. J. Biochem. (1990) 192(3), 767-775). Methods for producing recombinant antibodies are also known as described below.

Generally, to obtain a cDNA encoding the antibody variable region (V region), total RNA is first extracted from hybridomas. For example, the following methods can be used as methods for extracting mRNAs from cells:

- the guanidine ultracentrifugation method (Biochemistry (1979) 18(24), 5294-5299), and
- the AGPC method (Anal. Biochem. (1987) 162(1), 156-159).

Extracted mRNAs can be purified using the mRNA Purification Kit (GE Healthcare Bioscience) or such. Alternatively, kits for extracting total mRNA directly from cells, such as the QuickPrep mRNA Purification Kit (GE Healthcare Bioscience), are also commercially available. mRNAs can be prepared from hybridomas using such kits. cDNAs encoding the antibody V region can be synthesized from the prepared mRNAs using a reverse transcriptase. cDNAs can be synthesized using the AMV Reverse Transcriptase First-strand cDNA Synthesis Kit (Seikagaku Corporation) or such. Furthermore, the SMART RACE cDNA amplification kit (Clontech) and the PCR-based 5'-RACE method (Proc. Natl. Acad. Sci. USA (1988) 85(23), 8998-9002; Nucleic Acids Res. (1989) 17(8), 2919-2932) can be appropriately used to synthesize and amplify cDNAs. In such a cDNA synthesis process, appropriate restriction enzyme sites described below may be introduced into both ends of a cDNA.

The cDNA fragment of interest is purified from the resulting PCR product, and then this is ligated to a vector DNA. A recombinant vector is thus constructed, and introduced into *E. coli* or such. After colony selection, the desired recombinant vector can be prepared from the colony-forming *E. coli.* Then, whether the recombinant vector has the cDNA nucleotide sequence of interest is tested by a known method such as the dideoxy nucleotide chain termination method.

The 5'-RACE method which uses primers to amplify the variable region gene is conveniently used for isolating the gene encoding the variable region. First, a 5'-RACE cDNA library is constructed by cDNA synthesis using RNAs extracted from hybridoma cells as a template. A commercially available kit such as the SMART RACE cDNA amplification kit is appropriately used to synthesize the 5'-RACE cDNA library.

The antibody gene is amplified by PCR using the prepared 5'-RACE cDNA library as a template. Primers for amplifying the mouse antibody gene can be designed based on known antibody gene sequences. The nucleotide sequences of the primers vary depending on the immunoglobulin subclass. Therefore, it is preferable that the subclass is determined in advance using a commercially available kit such as the Iso Strip mouse monoclonal antibody isotyping kit (Roche Diagnostics).

Specifically, for example, primers that allow amplification of genes encoding γ1, γ2a, γ2b, and γ3 heavy chains and κ and λ light chains are used to isolate mouse IgG-encoding genes. In general, a primer that anneals to a constant region site close to the variable region is used as a 3'-side primer to amplify an IgG variable region gene. Meanwhile, a primer attached to a 5' RACE cDNA library construction kit is used as a 5'-side primer.

Immunoglobulins composed of a combination of heavy and light chains may be reshaped using the thus amplified PCR products. A desired antibody can be selected by screening using the antigen-binding activity of a reshaped immunoglobulin as an indicator. The screening can be carried out, for example, by the following steps:

(1) contacting a desired antigen-expressing cell with an antibody comprising the V region encoded by a cDNA obtained from a hybridoma;
(2) detecting the binding of the antibody to the antigen-expressing cell; and
(3) selecting an antibody that binds to the antigen-expressing cell.

Methods for detecting the binding of an antibody to the antigen-expressing cells are known. Specifically, the binding of an antibody to the antigen-expressing cells can be detected by the above-described techniques such as FACS. Fixed samples of the antigen-expressing cells may be appropriately used to assess the binding activity of an antibody.

For antibody screening methods that use the binding activity as an indicator, panning methods that use phage vectors can also be used suitably. Screening methods using phage vectors are advantageous when the antibody genes are obtained from a polyclonal antibody-expressing cell population as heavy-chain and light-chain subclass libraries. Genes encoding the heavy-chain and light-chain variable regions can be linked by an appropriate linker sequence to form a single-chain Fv (scFv). Phages expressing scFv on their surface can be produced by inserting an scFv-encoding gene into a phage vector. The phages are contacted with an antigen of interest. Then, a DNA encoding scFv having the binding activity of interest can be isolated by collecting phages bound to the antigen. This process can be repeated as necessary to enrich scFv having the binding activity of interest.

After isolation of the cDNA encoding the V region of the antibody of interest, the cDNA is digested with restriction enzymes that recognize the restriction sites introduced into both ends of the cDNA. Preferred restriction enzymes recognize and cleave a nucleotide sequence that occurs in the nucleotide sequence of the antibody gene at a low frequency. Furthermore, a restriction site for an enzyme that produces a sticky end is preferably introduced into a vector to insert a single-copy digested fragment in the correct orientation. The cDNA encoding the V region of the antibody is digested as described above, and this is inserted into an appropriate expression vector to construct an antibody expression vector. In this case, if a gene encoding the antibody constant region (C region) and a gene encoding the above V region are fused in-frame, a chimeric antibody is obtained. Herein, a "chimeric antibody" means that the origin of the constant region is different from that of the variable region. Thus, in addition to mouse/human heterochimeric antibodies, human/human allochimeric antibodies are included in the chimeric antibodies of the present invention. A chimeric antibody expression vector can be constructed by inserting the above V region gene into an expression vector that already has the constant region. Specifically, for example, a recognition sequence for a restriction enzyme that excises the above V region gene can be appropriately placed on the 5' side of an expression vector carrying a DNA that encodes a desired antibody constant region (C region). A chimeric antibody expression vector is constructed by fusing in-frame two genes digested with the same combination of restriction enzymes.

To produce a monoclonal antibody, antibody genes are inserted into an expression vector so that the genes are expressed under the control of an expression regulatory region. The expression regulatory region for antibody expression includes, for example, enhancers and promoters. Furthermore, an appropriate signal sequence may be attached to the amino terminus so that the expressed antibody is secreted to the outside of cells. The signal sequence is cleaved from the carboxyl terminus of the expressed polypeptide, and the resulting antibody can be secreted to the outside of cells. Then, appropriate host cells are transformed with the expression vector, and recombinant cells expressing the antibody-encoding DNA can be obtained.

DNAs encoding the antibody heavy chain (H chain) and light chain (L chain) are separately inserted into different expression vectors to express the antibody gene. An antibody molecule having the H and L chains can be expressed by co-transfecting the same host cell with vectors inserted with the H chain and L chain. Alternatively, host cells can be transformed with a single expression vector into which DNAs encoding the H and L chains are inserted (see WO 94/11523).

There are many known combinations of host cells and expression vectors for antibody preparation by introducing isolated antibody genes into appropriate hosts. All these expression systems are applicable to isolation of the cancer-specific antigen-binding domains of the present invention, tumor necrosis factor receptor superfamily (TNFRSF) and T cell receptor complex-binding domain.

Appropriate eukaryotic cells used as host cells include animal cells, plant cells, and fungal cells. Specifically, the animal cells include, for example, the following cells.

(1) mammalian cells: CHO, COS, myeloma, baby hamster kidney (BHK), HeLa, Vero, or such;
(2) amphibian cells: Xenopus oocytes, or such; and
(3) insect cells: sf9, sf21, Tn5, or such.

In addition, as a plant cell, an antibody gene expression system using cells derived from the *Nicotiana* genus such as *Nicotiana tabacum* is known. Callus cultured cells can be appropriately used to transform plant cells.

Furthermore, the following cells can be used as fungal cells:

yeasts: the *Saccharomyces* genus such as *Saccharomyces cerevisiae,* and the *Pichia* genus such as *Pichia pastoris*; and
filamentous fungi: the *Aspergillus* genus such as *Aspergillus niger.*

Furthermore, antibody gene expression systems that utilize prokaryotic cells are also known. For example, when using bacterial cells, *E. coli* cells, *Bacillus subtilis* cells, and such can suitably be utilized in the present invention. Expression vectors carrying the antibody genes of interest are introduced into these cells by transfection. The transfected cells are cultured *in vitro,* and the desired antibody can be prepared from the culture of transformed cells.

In addition to the above-described host cells, transgenic animals can also be used to produce a recombinant antibody. That is, the antibody can be obtained from an animal into which the gene encoding the antibody of interest is introduced. For example, the antibody gene can be constructed as a fusion gene by inserting in frame into a gene that encodes a protein produced specifically in milk. Goat β-casein or such can be used, for example, as the protein secreted in milk. DNA fragments containing the fused gene inserted with the antibody gene is injected into a goat embryo, and then this embryo is introduced into a female goat. Desired antibodies can be obtained as a protein fused with the milk protein from milk produced by the transgenic goat born from the embryo-recipient goat (or progeny thereof). In addition, to increase the volume of milk containing the desired antibody produced by the transgenic goat, hormones can be administered to the transgenic goat as necessary (Bio/Technology (1994) 12 (7), 699-702).

When an antibody described herein is administered to human, an antigen-binding domain derived from a genetically recombinant antibody that has been artificially modified to reduce the heterologous antigenicity against human and such, can be appropriately used as the various binding domains in the molecule when domains comprising an antibody variable region are used. Such genetically recombinant antibodies include, for example, humanized antibodies. These modified antibodies are appropriately produced by known methods.

An antibody variable region used to produce the various binding domains of antibodies described herein is generally formed by three complementarity-determining regions (CDRs) that are separated by four framework regions (FRs). CDR is a region that substantially determines the binding specificity of an antibody. The amino acid sequences of CDRs are highly diverse. On the other hand, the FR-forming amino acid sequences often have high identity even among antibodies with different binding specificities. Therefore, generally, the binding specificity of a certain antibody can be introduced into another antibody by CDR grafting.

A humanized antibody is also called a reshaped human antibody. Specifically, humanized antibodies prepared by grafting the CDR of a non-human animal antibody such as a mouse antibody to a human antibody and such are known. Common genetic engineering techniques for obtaining humanized antibodies are also known. Specifically, for example, overlap extension PCR is known as a method for grafting a mouse antibody CDR to a human FR. In overlap extension PCR, a nucleotide sequence encoding a mouse antibody CDR to be grafted is added to primers for synthesizing a human antibody FR. Primers are prepared for each of the four FRs. It is generally considered that when grafting a mouse CDR to a human FR, selecting a human FR that has high identity to a mouse FR is advantageous for maintaining the CDR function. That is, it is generally preferable to use a human FR comprising an amino acid sequence which has high identity to the amino acid sequence of the FR adjacent to the mouse CDR to be grafted.

Nucleotide sequences to be ligated are designed so that they will be connected to each other in frame. Human FRs are individually synthesized using the respective primers. As a result, products in which the mouse CDR-encoding DNA is attached to the individual FR-encoding DNAs are obtained. Nucleotide sequences encoding the mouse CDR of each product are designed so that they overlap with each other. Then, complementary strand synthesis reaction is conducted to anneal the overlapping CDR regions of the products synthesized using a human antibody gene as template. Human FRs are ligated *via* the mouse CDR sequences by this reaction.

The full length V region gene, in which three CDRs and four FRs are ultimately ligated, is amplified using primers that anneal to its 5'- or 3'-end, which are added with suitable restriction enzyme recognition sequences. An expression vector for humanized antibody can be produced by inserting the DNA obtained as described above and a DNA that encodes a human antibody C region into an expression vector so that they will ligate in frame. After the recombinant vector is transfected into a host to establish recombinant cells, the recombinant cells are cultured, and the DNA encoding the humanized antibody is expressed to produce the humanized antibody in the cell culture (see, European Patent Publication No. EP 239400 and International Patent Publication No. WO 1996/002576).

By qualitatively or quantitatively measuring and evaluating the antigen-binding activity of the humanized antibody produced as described above, one can suitably select human antibody FRs that allow CDRs to form a favorable antigen-binding site when ligated through the CDRs. Amino acid residues in FRs may be substituted as necessary, so that the CDRs of a reshaped human antibody form an appropriate antigen-binding site. For example, amino acid sequence mutations can be introduced into FRs by applying the PCR method used for grafting a mouse CDR into a human FR. More specifically, partial nucleotide sequence mutations can be introduced into primers that anneal to the FR. Nucleotide sequence mutations are introduced into the FRs synthesized by using such primers. Mutant FR sequences having the desired characteristics can be selected by measuring and evaluating the activity of the amino acid-substituted mutant antibody to bind to the antigen by the above-mentioned method (Sato, K. et al., Cancer Res. (1993) 53: 851-856).

Alternatively, desired human antibodies can be obtained by immunizing transgenic animals having the entire repertoire of human antibody genes (see WO 1993/012227; WO 1992/003918; WO 1994/002602; WO 1994/025585; WO 1996/034096; WO 1996/033735) by DNA immunization.

Furthermore, techniques for preparing human antibodies by panning using human antibody libraries are also known. For example, the V region of a human antibody is expressed as a single-chain antibody (scFv) on phage surface by the phage display method. Phages expressing an scFv that binds to the antigen can be selected. The DNA sequence encoding the human antibody V region that binds to the antigen can be determined by analyzing the genes of selected phages. The DNA sequence of the scFv that binds to the antigen is determined. An expression vector is prepared by fusing the V region sequence in frame with the C region sequence of a desired human antibody, and inserting this into an appropriate expression vector. The expression vector is introduced into cells appropriate for expression such as those described above. The human antibody can be produced by expressing the human antibody-encoding gene in the cells. These methods are already known (see WO 1992/001047; WO 1992/020791; WO 1993/006213; WO 1993/011236; WO 1993/019172; WO 1995/001438; WO 1995/015388).

In addition to the phage display method, techniques that use a cell-free translation system, techniques for displaying antigen-binding molecules on the surface of viruses or cells, and techniques that use emulsions are also known as techniques for obtaining human antibodies by panning using human antibody libraries. For example, the ribosome display method where a complex is formed between the translated protein and mRNA *via* the ribosome by removing the stop codon and such, the cDNA display method or the mRNA display method where a genetic sequence and the translated protein are covalently linked using a compound such as puromycin, the CIS display method where a complex is formed between the gene and the translated protein using a nucleic acid-binding protein, or such may be used as techniques of using a cell-free translation system. For the technique of presenting antigen-binding molecules on the surface of cells or viruses, besides the phage display method, the *E. coli* display method, Gram-positive bacteria display method, yeast display method, mammalian cell display method, virus display method, and such may be used. As a technique that uses emulsions, the *in vitro* virus display method which involves incorporating genes and translation-related molecules into an emulsion, and such may be used. These methods are already publicly known (Nat Biotechnol. 2000 Dec;18(12):1287-92; Nucleic Acids Res. 2006;34(19): e127; Proc Natl Acad Sci U S A. 2004 Mar 2;101(9):2806-10; Proc Natl Acad Sci USA. 2004 Jun 22;101(25):9193-8; Protein Eng Des Sel. 2008 Apr;21(4):247-55; Proc NatlAcad Sci U S A. 2000 Sep 26;97(20):10701-5; MAbs. 2010 Sep-Oct;2(5):508-18; and Methods Mol Biol.

As methods for defining CDRs, the method by Kabat el al. (Sequences of Proteins of Immunological Interest, 5th Ed (1991), Bethesda, MD), the method by Chothia et al. (Science (1986) 233, 755-758), and the method based on antigen-antibody contact regions (J Mol Biol (1996) 262, 732-745) are known. Specifically, each of the methods defines the CDRs as follows:

| CDR | Kabat | Chothia | Contact |
|---|---|---|---|
| L1 | L24-L34 | L24-L34 | L30-L36 |
| L2 | L50-L56 | L50-L56 | L46-L55 |
| L3 | L89-L97 | L89-L97 | L89-L96 |
| H1 | H31-H35B | H26-H32/34 | H30-H35B (Kabat numbering) |
| H1 | H31-H35 | H26-H32 | H30-H35 (Chothia numbering) |
| H2 | H50-H65 | H52-H56 | H47-H58 |
| H3 | H95-H102 | H95-H102 | H93-H101 |

2012;911:183-98).

In the present invention, "specific" means a condition where one of the molecules involved in specific binding does not show any significant binding to molecules other than a single or a number of binding partner molecules. Furthermore, "specific" is also used when an antigen-binding domain is specific to a particular epitope among multiple epitopes contained in an antigen. When an epitope bound by an antigen-binding domain is contained in multiple different antigens, antibodies containing the antigen-binding domain can bind to various antigens that have the epitope.

"Epitope" means an antigenic determinant in an antigen, and refers to an antigen site to which various binding domains in antibodies disclosed herein bind. Thus, for example, an epitope can be defined according to its structure. Alternatively, the epitope may be defined according to the antigen-binding activity of an antibody that recognizes the epitope. When the antigen is a peptide or polypeptide, the epitope can be specified by the amino acid residues that form the epitope. Alternatively, when the epitope is a sugar chain, the epitope can be specified by its specific sugar chain structure.

A linear epitope is an epitope that contains an epitope whose primary amino acid sequence is recognized. Such a linear epitope typically contains at least three and most commonly at least five, for example, about 8 to 10 or 6 to 20 amino acids in its specific sequence.

In contrast to the linear epitope, "conformational epitope" is an epitope in which the primary amino acid sequence containing the epitope is not the only determinant of the recognized epitope (for example, the primary amino acid sequence of a conformational epitope is not necessarily recognized by an epitope-defining antibody). Conformational epitopes may contain a greater number of amino acids compared to linear epitopes. A conformational epitope-recognizing antibody recognizes the three-dimensional structure of a peptide or protein. For example, when a protein molecule folds and forms a three-dimensional structure, amino acids and/or polypeptide main chains that form a conformational epitope become aligned, and the epitope is made recognizable by the antibody. Methods for determining epitope conformations include, for example, X ray crystallography, two-dimensional nuclear magnetic resonance spectroscopy, site-specific spin labeling, and electron paramagnetic resonance spectroscopy, but are not limited thereto. See, for example, Epitope Mapping Protocols in Methods in Molecular Biology (1996), Vol. 66, Morris (ed.).

Examples of a method for assessing the binding of an epitope in a cancer-specific antigen by a test antibody are shown below. According to the examples below, methods for assessing the binding of an epitope in a target antigen by another binding domain can also be appropriately conducted.

For example, whether a test antibody that comprises an antigen-binding domain for a cancer-specific antigen recognizes a linear epitope in the antigen molecule can be confirmed for example as mentioned below. For example, a linear peptide comprising an amino acid sequence forming the extracellular domain of a cancer-specific antigen is synthesized for the above purpose. The peptide can be synthesized chemically, or obtained by genetic engineering techniques using a region in a cDNA of a cancer-specific antigen encoding the amino acid sequence that corresponds to the extracellular domain. Then, a test antibody containing an antigen-binding domain for a cancer-specific antigen is assessed for its binding activity towards a linear peptide comprising the extracellular domain-constituting amino acid sequence. For example, an immobilized linear peptide can be used as an antigen to evaluate the binding activity of the antibody towards the peptide by ELISA. Alternatively, the binding activity towards a linear peptide can be assessed based on the level at which the linear peptide inhibits binding of the antibody to cancer-specific antigen-expressing cells. The binding activity of the antibody towards the linear peptide can be demonstrated by these tests.

Whether the above-mentioned test antibody containing an antigen-binding domain towards an antigen recognizes a conformational epitope can be confirmed as below. For example, an antibody that comprises an antigen-binding domain for a cancer-specific antigen strongly binds to cancer-specific antigen-expressing cells upon contact, but does not substantially bind to an immobilized linear peptide comprising an amino acid sequence forming the extracellular domain of the cancer-specific antigen. Herein, "does not substantially bind" means that the binding activity is 80% or less, generally 50% or less, preferably 30% or less, and particularly preferably 15% or less compared to the binding activity to antigen-expressing cells.

Methods for assaying the binding activity of a test antibody comprising an antigen-binding domain to antigen-expressing cells include, for example, the methods described in Antibodies A Laboratory Manual (Ed Harlow, David Lane, Cold Spring Harbor Laboratory (1988) 359-420). Specifically, the assessment can be performed based on the principle of ELISA or fluorescence activated cell sorting (FACS) using antigen-expressing cells as antigen.

In the ELISA format, the binding activity of a test antibody comprising an antigen-binding domain towards antigen-expressing cells can be assessed quantitatively by comparing the levels of signals generated by enzymatic reaction. Specifically, a test antibody is added to an ELISA plate onto which antigen-expressing cells are immobilized. Then, the test antibody bound to the cells is detected using an enzyme-labeled antibody that recognizes the test antibody. Alternatively, when FACS is used, a dilution series of a test antibody is prepared, and the antibody-binding titer for antigen-expressing cells can be determined to compare the binding activity of the test antibody towards antigen-expressing cells.

The binding of a test antibody to an antigen expressed on the surface of cells suspended in buffer or the like can be detected using a flow cytometer. Known flow cytometers include, for example, the following devices:

FACSCanto™ II
FACSAria™
FAC $S_{Arr}$ayTM
FACSVantage™ SE
FACSCalibur™ (all are trade names of BD Biosciences)
EPICS ALTRA HyPerSort
Cytomics FC 500
EPICS XL-MCL ADC EPICS XL ADC
Cell Lab Quanta/Cell Lab Quanta SC (all are trade names of Beckman Coulter).

Suitable methods for assaying the binding activity of the above-mentioned test antibody comprising an antigen-binding domain towards an antigen include, for example, the method below. First, antigen-expressing cells are reacted with a test antibody, and then this is stained with an FITC-labeled secondary antibody that recognizes the antibody. The test antibody is appropriately diluted with a suitable buffer to prepare the antibody at a desired concentration. For example, the antibody can be used at a concentration within the range of 10 μg/ml to 10 ng/ml. Then, the fluorescence intensity and cell count are determined using FACSCalibur (BD). The fluorescence intensity obtained by analysis using the CELL QUEST Software (BD), i.e., the Geometric Mean value, reflects the quantity of antibody bound to the cells. That is, the binding activity of a test antibody, which is represented by the quantity of the test antibody bound, can be measured by determining the Geometric Mean value.

Whether a test antigen-binding molecule comprising an antigen-binding domain of the present invention shares a common epitope with another antibody can be assessed based on competition between the two molecules for the same epitope. The competition between antibodies can be detected by a cross-blocking assay or the like. For example, the competitive ELISA assay is a preferred cross-blocking assay.

Specifically, in a cross-blocking assay, the antigen coating the wells of a microtiter plate is pre-incubated in the presence or absence of a candidate competitor antibody, and then a test antibody is added thereto. The quantity of test antibody bound to the antigen in the wells indirectly correlates with the binding ability of a candidate competitor antibody that competes for the binding to the same epitope. That is, the greater the affinity of the competitor antibody for the same epitope, the lower the binding activity of the test antibody towards the antigen-coated wells.

The quantity of the test antigen-binding molecule bound to the wells *via* the antigen can be readily determined by labeling the antibody in advance. For example, a biotin-labeled antibody can be measured using an avidin/peroxidase conjugate and appropriate substrate. In particular, a cross-blocking assay that uses enzyme labels such as peroxidase is called "competitive ELISA assay". The antibody can also be labeled with other labeling substances that enable detection or measurement. Specifically, radiolabels, fluorescent labels, and such are known.

When the candidate competitor antibody can block the binding of a test antibody comprising an antigen-binding domain by at least 20%, preferably at least 20 to 50%, and more preferably at least 50% compared to the binding activity in a control experiment conducted in the absence of the competitor antibody, the test antibody is determined to substantially bind to the same epitope bound by the competitor antibody, or to compete for binding to the same epitope.

When the structure of an epitope bound by a test antibody comprising an antigen-binding domain of the present invention is already identified, whether the test antibody and control antigen-binding molecule share a common epitope can be assessed by comparing the binding activities of the two antibodies towards a peptide prepared by introducing amino acid mutations into the peptide forming the epitope.

As a method for measuring such binding activities, for example, the binding activities of test and control antibodies towards a linear peptide into which a mutation is introduced are measured by comparison in the above ELISA format. Besides the ELISA methods, the binding activity towards the mutant peptide bound to a column can be determined by passing the test and control antibodies through the column, and then quantifying the antibody eluted in the eluate. Methods for adsorbing a mutant peptide to a column, for example, in the form of a GST fusion peptide, are known.

Alternatively, when the identified epitope is a conformational epitope, whether test and control antibodies share a common epitope can be assessed by the following method. First, cells expressing an antigen targeted by an antigen-binding domain and cells expressing an antigen having an epitope introduced with a mutation are prepared. The test and control antibodies are added to a cell suspension prepared by suspending these cells in an appropriate buffer such as PBS. Then, the cell suspension is appropriately washed with a buffer, and an FITC-labeled antibody that can recognize the test and control antibodies is added thereto. The fluorescence intensity and number of cells stained with the labeled antibody are determined using FACSCalibur (BD). The test and control antibodies are appropriately diluted using a suitable buffer, and used at desired concentrations. For example, they may be used at a concentration within the range of 10 μg/ml to 10 ng/ml. The fluorescence intensity determined by analysis using the CELL QUEST Software (BD), i.e., the Geometric Mean value, reflects the quantity of the labeled antibody bound to the cells. That is, the binding activities of the test and control antibodies, which are represented by the quantity of the labeled antibody bound, can be measured by determining the Geometric Mean value.

Furthermore, herein, the terms "scFv", "single-chain antibody", and "$sc(Fv)_2$" all refer to an antibody fragment of a single polypeptide chain that contains variable regions derived from the heavy and light chains, but not the constant region. In general, a single-chain antibody also contains a polypeptide linker between the VH and VL domains, which enables formation of a desired structure that is thought to allow antigen binding. The single-chain antibody is discussed in detail by Pluckthun in "The Pharmacology of Monoclonal Antibodies, Vol. 113, Rosenburg and Moore, eds., Springer-Verlag, New York, 269-315 (1994)". See also International Patent Publication WO 1988/001649; US Patent Nos. 4,946,778 and 5,260,203. In a particular embodiment, the single-chain antibody can be bispecific and/or humanized.

scFv is an antigen-binding domain in which VH and VL forming Fv are linked together by a peptide linker (Proc. Natl. Acad. Sci. U.S.A. (1988) 85(16), 5879-5883). VH and VL can be retained in close proximity by the peptide linker.

$sc(Fv)_2$ is a single-chain antibody in which four variable regions of two VL and two VH are linked by linkers such as peptide linkers to form a single chain (J Immunol. Methods (1999) 231(1-2), 177-189). The two VH and two VL may be derived from different monoclonal antibodies. Such $sc(Fv)_2$ preferably includes, for example, a bispecific $sc(Fv)_2$ that recognizes two types of epitopes present in a single antigen as disclosed in the Journal of Immunology (1994) 152(11), 5368-5374. $sc(Fv)_2$ can be produced by methods known to those skilled in the art. For example, $sc(Fv)_2$ can be produced by linking scFv by a linker such as a peptide linker.

Herein, the form of an antigen-binding domain forming an $sc(Fv)_2$ include an antibody in which the two VH units and two VL units are arranged in the order of VH, VL, VH, and VL ([VH]-linker-[VL]-linker-[VH]-linker-[VL]) beginning from the N terminus of a single-chain polypeptide. The order of the two VH units and two VL units is not limited to the above form, and they may be arranged in any order. Example order of the form is listed below.

[VL]-linker-[VH]-linker-[VH]-linker-[VL]
[VH]-linker-[VL]-linker-[VL]-linker-[VH]
[VH]-linker-[VH]-linker-[VL]-linker-[VL]
[VL]-linker-[VL]-linker-[VH]-linker-[VH]
[VL]-linker-[VH]-linker-[VL]-linker-[VH]

The molecular form of $sc(Fv)_2$ is also described in detail in WO2006/132352. According to these descriptions, those skilled in the art can appropriately prepare desired $sc(Fv)_2$ to produce the antibodies disclosed herein.

Herein, the term "variable fragment (Fv)" refers to the minimum unit of an antibody-derived antigen-binding domain composed of a pair of the antibody light chain variable region (VL) and antibody heavy chain variable region (VH). In 1988, Skerra and Pluckthun found that homogeneous and active antibodies can be prepared from the *E. coli* periplasm fraction by inserting an antibody gene downstream of a bacterial signal sequence and inducing expression of the gene in *E. coli* (Science (1988) 240(4855), 1038-1041). In the Fv prepared from the periplasm fraction, VH associates with VL in a manner so as to bind to an antigen.

Furthermore, the antibody of the present invention may be conjugated with a carrier polymer such as PEG or an organic compound such as an anticancer agent. Alternatively, a glycosylation sequence can be inserted to suitably add a sugar chain for the purpose of producing a desired effect.

The linkers to be used for linking the variable regions of an antibody comprise arbitrary peptide linkers that can be introduced by genetic engineering, synthetic linkers, and linkers disclosed in, for example, Protein Engineering, 9(3), 299-305, 1996. However, peptide linkers are preferred in the present invention. The length of the peptide linkers is not particularly limited, and can be suitably selected by those skilled in the art according to the purpose. The length is preferably five amino acids or more (without particular limitation, the upper limit is generally 30 amino acids or less, preferably 20 amino acids or less), and particularly preferably 15 amino acids. When $sc(Fv)_2$ contains three peptide linkers, their length may be all the same or different.

For example, such peptide linkers include:

Ser
Gly·Ser
Gly·Gly·Ser
Ser·Gly·Gly
Gly·Gly·Gly·Ser (SEQ ID NO: 20)
Ser·Gly·Gly·Gly (SEQ ID NO: 21)
Gly·Gly·Gly·Gly·Ser (SEQ ID NO: 22)
Ser·Gly·Gly·Gly·Gly (SEQ ID NO: 23)
Gly·Gly·Gly·Gly·Gly·Ser (SEQ ID NO: 24)
Ser·Gly·Gly·Gly·Gly·Gly (SEQ ID NO: 25)
Gly·Gly·Gly·Gly·Gly·Gly·Ser (SEQ ID NO: 26)
Ser·Gly·Gly·Gly·Gly·Gly·Gly (SEQ ID NO: 27)

(Gly·Gly·Gly·Gly·Ser (SEQ ID NO: 22))n
(Ser·Gly·Gly·Gly·Gly (SEQ ID NO: 23))n
where n is an integer of 1 or larger. The length or sequences of peptide linkers can be selected accordingly by those skilled in the art depending on the purpose.

Synthetic linkers (chemical crosslinking agents) is routinely used to crosslink peptides, and for example:

N-hydroxy succinimide (NHS),
disuccinimidyl suberate (DSS),
bis(sulfosuccinimidyl) suberate (BS3),
dithiobis(succinimidyl propionate) (DSP),
dithiobis(sulfosuccinimidyl propionate) (DTSSP),
ethylene glycol bis(succinimidyl succinate) (EGS),
ethylene glycol bis(sulfosuccinimidyl succinate) (sulfo-EGS),
disuccinimidyl tartrate (DST), disulfosuccinimidyl tartrate (sulfo-DST),
bis[2-(succinimidoxycarbonyloxy)ethyl] sulfone (BSOCOES),
and bis[2-(sulfosuccinimidoxycarbonyloxy)ethyl] sulfone (sulfo-BSOCOES). These crosslinking agents are commercially available.

In general, three linkers are required to link four antibody variable regions together. The linkers to be used may be of the same type or different types.

Furthermore, "Fab" is composed of a single light chain, and a CH1 domain and variable region from a single heavy chain. The heavy chain of Fab molecule cannot form disulfide bonds with another heavy chain molecule.

"F(ab')$_2$" or "Fab'" is produced by treating an immunoglobulin (monoclonal antibody) with a protease such as pepsin and papain, and refers to an antibody fragment generated by digesting an immunoglobulin (monoclonal antibody) at near the disulfide bonds present between the hinge regions in each of the two H chains. For example, papain cleaves IgG upstream of the disulfide bonds present between the hinge regions in each of the two H chains to generate two homologous antibody fragments, in which an L chain comprising VL (L-chain variable region) and CL (L-chain constant region) is linked to an H-chain fragment comprising VH (H-chain variable region) and CHγ1 (γ1 region in an H-chain constant region) *via* a disulfide bond at their C-terminal regions. Each of these two homologous antibody fragments is called Fab'.

"F(ab')$_2$" contains two light chains and two heavy chains comprising the constant region of a CH1 domain and a portion of a CH2 domain so that disulfide bonds are formed between the two heavy chains. The F(ab')$_2$ constituting an antibody disclosed herein can be preferably obtained as below. A full-length monoclonal antibody or such comprising a desired antigen-binding domain is partially digested with a protease such as pepsin, and then Fc fragments are removed by adsorption onto a Protein A column. The protease is not particularly limited, as long as it can digest the full-length antibody in a restrictive manner to produce F(ab')$_2$ under an appropriately established enzyme reaction condition such as pH. Such proteases include, for example, pepsin and ficin.

A preferred embodiment of the "multispecific antibody" of the present invention includes a bispecific antibody. When using an Fc region with decreased Fcγ receptor-binding activity as the Fc region of a bispecific antibody, an Fc region derived from a bispecific antibody may also be used appropriately.

For association of multispecific antibodies, one can apply the technique of introducing charge repulsion at the interface of the second constant region of the antibody H chain (CH2) or the third constant region of the H chain (CH3) to suppress undesired associations between H chains (WO2006/106905).

In the technique of suppressing unintended association between H chains by introducing charge repulsion at the interface of CH2 or CH3, examples of the amino acid residues that are contacted at the interface of other constant regions of the H chain include the region facing the residue at position 356 (EU numbering), the residue at position 439 (EU numbering), the residue at position 357 (EU numbering), the residue at position 370 (EU numbering), the residue at position 399 (EU numbering), and the residue at position 409 (EU numbering) in the CH3 region.

More specifically, for example, for an antibody comprising two types of H chain CH3 regions, the antibody can be made so that one to three pairs of amino acid residues selected from the amino acid residue pairs shown below in (1) to (3) in the first H chain CH3 region have the same charge: (1) amino acid residues at positions 356 and 439 (EU numbering) which are amino acid residues contained in the H chain CH3 region; (2) amino acid residues at positions 357 and 370 (EU numbering) which are amino acid residues contained in the H chain CH3 region; and (3) amino acid residues at positions 399 and 409 (EU numbering) which are amino acid residues contained in the H chain CH3 region.

Furthermore, the antibody can be made so that one to three pairs of amino acid residues corresponding to the amino acid residue pairs shown above in (1) to (3) having the same type of charge in the first H chain CH3 region, which are amino acid residue pairs selected from the amino acid residue pairs shown above in (1) to (3) in the second H chain CH3 region which differs from the first H chain CH3 region, have a charge opposite to the corresponding amino acid residues in the aforementioned first H chain CH3 region.

The respective amino acid residues of (1) to (3) mentioned above are positioned close to each other when associated. For the desired H chain CH3 region or H chain constant region, those skilled in the art can find sites corresponding to the above-mentioned amino acid residues of (1) to (3) by homology modeling and such using commercially available software, and amino acid residues of these sites can be subjected to modifications as appropriate.

In the above-mentioned antibodies, "amino acid residues having a charge" are preferably selected, for example, from amino acid residues contained in either one of groups (a) and (b) below:

(a) glutamic acid (E) and aspartic acid (D); and
(b) lysine (K), arginine (R), and histidine (H).

Regarding the above-mentioned antibodies, "having the same type of charge" means, for example, that two or more amino acid residues all have amino acid residues included in either one of the above-mentioned groups (a) and (b). The phrase "having the opposite charge" means that, for example, when at least one of the two or more amino acid residues has an amino acid residue included in either one of the above-mentioned groups (a) and (b), the remaining amino acid residue(s) will have an amino acid residue included in the other group.

In a preferred embodiment of the above-mentioned antibody, the first H chain CH3 region and the second H chain CH3 region may be cross-linked by a disulfide bond.

In the present invention, the amino acid residue to be subjected to alteration is not limited to an amino acid residue of the constant region or variable region of the antibody described above. With regard to polypeptide mutants or heteromultimers, those skilled in the art can find amino acid residues that form the interface through homology modeling and such using commercially available software, and can subject the amino acid residues at those sites to alterations so that association is regulated.

Other known techniques can also be used for the association of multispecific antibodies of the present invention. Polypeptides with different amino acids having an Fc region can be efficiently associated with each other by substituting an amino acid side chain present in one of the H chain variable regions of the antibody with a larger side chain (knob), and substituting an amino acid side chain present in the corresponding variable region of the other H chain with a smaller side chain (hole), to allow placement of the knob within the hole (WO1996/027011; Ridgway JB et al., Protein Engineering (1996) 9, 617-621; Merchant AM et al. Nature Biotechnology (1998) 16, 677-681; and US20130336973).

In addition, other known techniques can also be used to form multispecific antibodies of the present invention. Association of polypeptides having different sequences can be induced efficiently by complementary association of CH3s, using a strand-exchange engineered CH3 domain produced by changing part of CH3 in one of the H chains of an antibody into its corresponding IgA-derived sequence, and introducing into the complementary portion of the CH3 in the other H chain its corresponding IgA-derived sequence (Protein Engineering Design & Selection, 23; 195-202, 2010). This known technique can also be used to efficiently form multispecific antibodies of interest.

In addition, the following techniques and such may be used for the formation of multispecific antibodies: techniques for antibody production using association of antibody CH1 and CL, and association of VH and VL as described in WO 2011/028952, WO2014/018572, and Nat Biotechnol. 2014 Feb;32(2):191-8; techniques for producing bispecific antibodies using separately prepared monoclonal antibodies in combination (Fab Arm Exchange) as described in WO2008/119353 and WO2011/131746; techniques for regulating association between antibody heavy chain CH3s as described in WO2012/058768 and WO2013/063702; techniques for producing bispecific antibodies composed of two types of light chains and one type of heavy chain as described in WO2012/023053; techniques for producing bispecific antibodies using two bacterial cell strains that individually express one of the chains of an antibody comprising a single H chain and a single L chain as described by Christoph et al. (Nature Biotechnology Vol. 31, p 753-758 (2013)).

An embodiment of multispecific antibody formation includes methods for obtaining bispecific antibodies by mixing two types of monoclonal antibodies in the presence of a reducing agent to cleave the disulfide bonds in the core hinge region, followed by re-association for heterodimerization (FAE) as described above. Meanwhile, introduction of electrostatic interactions at the interacting interface of the CH3 region (WO2006/106905) can induce even more efficient heterodimerization during the re-association (WO2015/046467). In FAE using naturally-occurring IgG, re-association takes place randomly; and thus theoretically, bispecific antibodies can only be obtained at 50% efficiency; however, in this method, bispecific antibodies can be produced in high yield.

Alternatively, even when a multispecific antibody of interest cannot be formed efficiently, a multispecific antibody of the present invention can be obtained by separating and purifying the multispecific antibody of interest from the produced antibodies. For example, a method has been reported that enables purification of two types of homologous forms and the heterologous antibody of interest by ion exchange chromatography, by conferring a difference in the isoelectric points by introducing amino acid substitutions into the variable regions of the two types of H chains (WO2007114325). To date, as a method for purifying heterologous forms, a method using Protein A to purify a heterodimerized antibody comprising a mouse IgG2a H chain that binds to Protein A and a rat IgG2b H chain that does not bind to Protein A has been reported (WO98050431 and WO95033844). Furthermore, the heterodimerized antibody per se can be purified efficiently using a Protein A column by changing the interaction between each of the H chains and Protein A, by using H chains in which amino acid residues at the IgG-Protein A binding site, positions 435 and 436 (EU numbering), are substituted with amino acids that yield a different binding strength to Protein A such as Tyr, His, or such.

Alternatively, a common L chain that can confer binding ability to a plurality of different H chains can be obtained and used as the common L chain of a multispecific antibody. Efficient expression of a multispecific IgG can be achieved by introducing the genes of such a common L chain and a plurality of different H chains into cells and expressing the IgG (Nature Biotechnology (1998) 16, 677-681). A method for selecting a common L chain that shows strong binding ability to any different H chains can also be used when selecting a common H chain (WO 2004/065611).

Furthermore, an Fc region whose C-terminal heterogeneity has been improved can be appropriately used as an Fc region of the present invention. More specifically, Fc regions lacking glycine at position 446 and lysine at position 447, as specified by EU numbering, in the amino acid sequences of two polypeptides constituting an Fc region derived from IgG1, IgG2, IgG3, or IgG4, are provided.

A plurality, such as two or more, of these techniques can be used in combination. Furthermore, these techniques can be appropriately and separately applied to the two H chains to be associated. Furthermore, these techniques can be used in combination with the above-mentioned Fc region of which Fcγ receptor-binding activity has been decreased. Furthermore, an antibody of the present invention may be an antibody produced separately based on an antibody subjected to the above-described modifications so as to have the same amino acid sequence.

The present invention also relates to polynucleotides encoding the agonist antibodies against the TNF receptor superfamily or the multispecific antibodies of the present invention. The polynucleotides can be incorporated into arbitrary expression vectors. Suitable hosts can be transformed with the expression vectors to produce antibody-expressing cells. Agonist antibodies against the TNF receptor superfamily or multispecific antibodies encoded by the polynucleotides can be obtained by culturing cells that express the agonist antibodies against the TNF receptor superfamily or multispecific antibodies, and collecting expression products from the culture supernatants. That is, the present invention relates to vectors comprising a polynucleotide that encodes a TNF receptor superfamily agonist antibody or a multispecific antibody of the present invention, cells carrying the vector, and methods for producing the TNF receptor superfamily agonist antibody or multispecific antibody, which comprise culturing the cells and collecting the antibody from the culture supernatant. These can be obtained by techniques similar to those for recombinant antibodies mentioned above.

## Pharmaceutical compositions

From another viewpoint, the present invention relates to cytotoxicity-inducing pharmaceutical compositions (cytotoxicity-inducing therapeutic agents), cell proliferation inhibitors, and anticancer agents, which comprise a multispecific antibody of the present invention as an active ingredient, for use in combination with a TNF receptor superfamily agonist antibody. Furthermore, the present invention relates to cytotoxicity-inducing pharmaceutical compositions (cytotoxicity-inducing therapeutic agents), cell proliferation inhibitors, and anticancer agents, which comprise a TNF receptor superfamily agonist antibody of the present invention as an active ingredient, for use in combination with a multispecific antibody of the present invention. The pharmaceutical compositions of the present invention can be used as agents for treating cancer or agents for preventing cancer. The cytotoxicity-inducing therapeutic agents, cell proliferation inhibitors, and anticancer agents of the present invention are preferably administered to subjects suffering from cancer or subjects who may undergo relapse.

Furthermore, in the present invention, the above-mentioned cytotoxicity-inducing therapeutic agents, cell proliferation inhibitors, and anticancer agents can be presented as methods for inducing cytotoxicity, methods for suppressing cell proliferation, methods for activating immunity against cancer cells or tumor tissues containing cancer cells, or methods for preventing or treating cancer, which comprise the step of administering a multispecific antibody and/or an agonist antibody to the subject. They can be also presented as use of a multispecific antibody and/or an agonist antibody in the production of cytotoxicity-inducing therapeutic agents (pharmaceutical compositions for inducing cytotoxicity), cell proliferation inhibitors, and anticancer agents. They can be also presented as a multispecific antibody and/or an agonist antibody for use in inducing cytotoxicity, suppressing cell proliferation, activating immunity against cancer cells or tumor tissues containing cancer cells, or treating or preventing cancer.

In the present invention, the term "treating" means that administration of a pharmaceutical composition of the present invention to a subject kills cancer cells or reduces the cell number, suppresses the proliferation of cancer cells, and ameliorates various symptoms caused by cancer. Furthermore, the term "preventing" means inhibiting an increase in the reduced number of cancer cells due to repopulation, and inhibiting repopulation of cancer cells whose proliferation has been suppressed.

In the present invention, "comprising a multispecific antibody as an active ingredient" or "comprising an agonist antibody as an active ingredient" means containing a multispecific antibody or an agonist antibody as a major active component, and it does not limit the content of the multispecific antibody or the agonist antibody.

Herein, "use in combination/combined use" includes cases where a pharmaceutical composition or such comprising a multispecific antibody of the present invention as an active ingredient and a pharmaceutical composition or such comprising a TNF receptor superfamily agonist antibody of the present invention as an active ingredient are simultaneously administered to a subject, and cases where they are separately administered to a subject. Their dosage forms may be the same or different. Furthermore, these pharmaceutical compositions or such may be provided as a kit.

Furthermore, the present invention provides a method that utilizes the effects produced by combined use of the above-mentioned multispecific antibody or a pharmaceutical composition or such comprising the multispecific antibody as an active ingredient and the above-mentioned TNF receptor superfamily agonist antibody or a pharmaceutical composition or such comprising the agonist antibody as an active ingredient to enhance the cytotoxic activity or antitumor effect of the above-mentioned TNF receptor superfamily agonist antibody or the pharmaceutical composition or such comprising the agonist antibody as an active ingredient by using the above-mentioned multispecific antibody or the pharmaceutical composition or such comprising the multispecific antibody as an active ingredient. Furthermore, the present invention provides a method for enhancing the cytotoxic activity or antitumor effect of the above-mentioned multispecific antibody or the pharmaceutical composition or such comprising the multispecific antibody as an active ingredient by using the above-mentioned TNF receptor superfamily agonist antibody or the pharmaceutical composition or such comprising the agonist antibody as an active ingredient.

Furthermore, pharmaceutical compositions or such of the present invention can be used by combining multiple types of a multispecific antibody of the present invention and/or a TNF receptor superfamily agonist antibody of the present invention as necessary. For example, by using a cocktail of a plurality of antibodies of the present invention that bind to the same antigen, one can enhance the cytotoxic effect against cells expressing the antigen.

If necessary, the antibodies of the present invention may be encapsulated in microcapsules (microcapsules made from hydroxymethylcellulose, gelatin, poly[methylmethacrylate], and the like), and made into components of colloidal drug delivery systems (liposomes, albumin microspheres, microemulsions, nano-particles, and nano-capsules) (for example, see "Remington's Pharmaceutical Science 16th edition", Oslo Ed. (1980)). Moreover, methods for preparing agents as sustained-release agents are known, and these can be applied to the antibodies of the present invention (J. Biomed. Mater. Res. (1981) 15, 267-277; Chemtech. (1982) 12, 98-105; US Patent No. 3773719; European Patent Application (EP) Nos. EP58481 and EP133988; Biopolymers (1983) 22, 547-556).

The pharmaceutical compositions, cell proliferation-suppressing agents, or anticancer agents of the present invention may be administered either orally or parenterally to patients. Parental administration is preferred. Specifically, such administration methods include injection, nasal administration, transpulmonary administration, and percutaneous administration. Injections include, for example, intravenous injections, intramuscular injections, intraperitoneal injections, and subcutaneous injections. For example, pharmaceutical compositions, therapeutic agents for inducing cellular cytotoxicity, cell proliferation-suppressing agents, or anticancer agents of the present invention can be administered locally or systemically by injection. Furthermore, appropriate administration methods can be selected according to the patient's age and symptoms. The administered dose can be selected, for example, from the range of 0.0001 mg to 1,000 mg per kg of body weight for each administration. Alternatively, the dose can be selected, for example, from the range of 0.001 mg/body to 100,000 mg/body per patient. However, the dose of a pharmaceutical composition of the present invention is not limited to these doses.

The pharmaceutical compositions of the present invention can be formulated according to conventional methods (for example, Remington's Pharmaceutical Science, latest edition, Mark Publishing Company, Easton, U.S.A.), and may also contain pharmaceutically acceptable carriers and additives. Examples include, but are not limited to, surfactants, excipients, coloring agents, flavoring agents, preservatives, stabilizers, buffers, suspension agents, isotonic agents, binders, disintegrants, lubricants, fluidity promoting agents, and corrigents, and other commonly used carriers can be suitably used. Specific examples of the carriers include light anhydrous silicic acid, lactose, crystalline cellulose, mannitol, starch, carmellose calcium, carmellose sodium, hydroxypropyl cellulose, hydroxypropyl methylcellulose, polyvinylacetal diethylaminoacetate, polyvinylpyrrolidone, gelatin, medium-chain triglyceride, polyoxyethylene hardened castor oil 60, saccharose, carboxymethyl cellulose, corn starch, inorganic salt, and such.

Furthermore, the present invention provides methods of inducing damage to cells expressing a cancer-specific antigen or to tumor tissues containing cells expressing a cancer-specific antigen and methods for suppressing proliferation of these cells or these tumor tissues, by contacting cells that express the certain cancer-specific antigen to a multispecific antibody of the present invention and an agonist antibody against a TNF receptor superfamily of the present invention. The cells bound by a multispecific antibody of the present invention that binds to the cancer-specific antigen are not particularly limited as long as they are cells that express the cancer-specific antigens. Suitable examples of the preferred cancer antigen-expressing cells of the present invention are specifically, cells of ovarian cancer, prostate cancer, breast cancer, uterine cancer, hepatic cancer, lung cancer, pancreatic cancer, gastric cancer, bladder cancer, and colorectal cancer.

In the present invention, "contact" is carried out, for example, by adding an antibody of the present invention that binds to the cancer antigen to a solution of cancer antigen-expressing cells cultured *in vitro.* In this case, a form suitable for use of the added antibody may be a solution, or a solid or such obtained by freeze-drying, and the like. When added as an aqueous solution, an aqueous solution containing purely the antibody of the present invention alone may be used, or a solution containing surfactants, excipients, coloring agents, perfumes, preservatives, stabilizers, buffers, suspending agents, isotonization agents, binders, disintegrants, lubricants, fluidity promoting agents, flavoring agents, and such described above may be used. The concentration used for the addition is not particularly limited, but a suitable final concentration in the culture solution is preferably in the range of 1 pg/ml to 1 g/ml, more preferably 1 ng/ml to 1 mg/ml, and even more preferably 1 $\mu$g/ml to 1 mg/ml.

Furthermore, in another embodiment, "contact" of the present invention is carried out by administering a multispecific antibody and/or a TNF receptor superfamily agonist antibody of the present invention that binds to a cancer antigen to a non-human animal with cells expressing the cancer-specific antigen transplanted into their bodies, and to an animal having cancer cells that intrinsically express the cancer-specific antigens. The method of administration may be oral or parenteral, and parenteral administration is particularly preferred. Specific examples of the administration method include administration by injection, transnasal administration, transpulmonary administration, and transdermal administration. Examples of administration by injection include intravenous injection, intramuscular injection, intraperitoneal injection, and subcutaneous injection. A pharmaceutical composition of the present invention or a pharmaceutical composition for inducing cytotoxicity, a cell proliferation inhibitor, and an anticancer agent can be administered systemically or locally, for example, through administration by injection. The method of administration can be selected appropriately according to the age and symptoms of the test animal. When administered as an aqueous solution, an aqueous solution containing purely the antibody of the present invention alone may be used, or a solution containing surfactants, excipients, coloring agents, perfumes, preservatives, stabilizers, buffers, suspending agents, isotonization agents, binders, disintegrants, lubricants, fluidity promoting agents, flavoring agents, and such described above may be used. The dose can be selected, for example, from the range of 0.0001 mg to 1000 mg per kilogram body weight for a single administration. Alternatively, for example, the dose may be selected from the range of 0.001 mg/body to 100000 mg/body per patient. However, the dose of the antibody of the present invention is not limited to these doses.

The following method is suitably used as a method for evaluating or measuring cytotoxicity induced in cells expressing a cancer-specific antigen bound by the cancer specific antigen-binding domain constituting a multispecific antibody of the present invention, as a result of contacting the multispecific antibody of the present invention and/or TNF receptor superfamily agonist antibody of the present invention with the cells. Examples of a method for evaluating or measuring the cytotoxic activity *in vitro* include methods for measuring cytotoxic T cell activity, and such. Whether an antibody of the present invention has T cell cytotoxicity can be measured by known methods (for example, Current protocols in Immunology, Chapter 7. Immunologic studies in humans, Editor, John E. Coligan et al., John Wiley & Sons, Inc., (1993) and the like). For activity measurements, a multispecific antibody with an antigen-binding domain that binds to an antigen which differs from the antigen bound in the present invention and is an antigen not expressed in the cells used for the examination can be used as a control and in the same manner as the multispecific antibody of the present invention, and the activity can be determined to be present when the multispecific antibody of the present invention shows a stronger cytotoxic activity than that of the multispecific antibody used as a control.

To evaluate or measure cytotoxic activity *in vivo,* for example, cells expressing an antigen bound by a cancer-specific antigen-binding domain that constitutes a multispecific antibody of the present invention are intradermally or subcutaneously transplanted into a non-human test animal, and then a test antibody is intravenously or intraperitoneally administered daily or with an interval of few days, starting from the day of transplantation or the following day. Tumor size is measured daily and the difference in the change of tumor size can be defined as the cytotoxic activity. In a similar manner to the *in vitro* evaluation, a control antibody is administered, and an antibody of the present invention can be determined as exhibiting cytotoxic activity based on the finding that the tumor size in the group subjected to administration of an antibody of the present invention is significantly smaller than the tumor size in the group subjected to administration of the control antibody.

As a method for evaluating or measuring the suppressive effect on proliferation of cells expressing an antigen bound by a cancer-specific antigen-binding domain that constitutes an antibody of the present invention by contact with the antibody, a method of measuring the uptake of isotope-labeled thymidine into cells, or the MTT method may be suitably used. As a method for evaluating or measuring the cell proliferation-suppressing activity *in vivo,* the same method as that described above for evaluating or measuring cytotoxic activity *in vivo* may be suitably used.

The present invention also provides kits for use in the methods of the present invention, which comprise an antibody of the present invention or an antibody produced by a production method of the present invention. Additionally, the kit may include in its package, a pharmaceutically acceptable carrier, solvent, and instructions describing the method of use.

The present invention also relates to an antibody of the present invention or an antibody produced by a production method of the present invention for use in a method of the present invention.

Herein, unless a limitation referring to a numerical amount such as "a single" or "multiple" is recited to describe a term, the terms of this description are not interpreted as being particularly limited in numerical quantity, and are understood to be the terms with a meaning of "one or a plurality of".

Those skilled in the art will naturally understand that optional combinations of one or more of the embodiments described herein are included in the present invention, as long as they are not technically inconsistent based on common technical knowledge of those skilled in the art.

All prior art references cited herein are incorporated by reference into this description.

Examples

Herein below, the present invention will be specifically described with reference to the Examples, but the scope of the present invention is not to be construed as being limited thereto.

[Reference Example 1] Construction of antibody expression vectors, and expression and purification of antibodies

Synthesis of full-length genes encoding the nucleotide sequences of the H chain and L chain of the antibody variable regions was carried out by production methods known to those skilled in the art using Assemble PCR and such. Introduction of amino acid substitutions was carried out by methods known to those skilled in the art using PCR or such. The obtained plasmid fragment was inserted into an animal cell expression vector, and the H-chain expression vector and L-chain expression vector were produced. The nucleotide sequence of the obtained expression vectors was determined by methods known to those skilled in the art. The produced plasmids were transiently introduced into the HEK293H cell line derived from human embryonic kidney cancer cells (Invitrogen) or into FreeStyle293 cells (Invitrogen) for antibody expression. The obtained culture supernatant was collected, and then passed through a 0.22 μm MILLEX(R)-GV filter (Millipore), or through a 0.45 μm MILLEX(R)-GV filter (Millipore) to obtain the culture supernatant. The antibodies were purified from the obtained culture supernatant by methods known to those skilled in the art using rProtein A Sepharose Fast Flow (GE Healthcare) or Protein G Sepharose 4 Fast Flow (GE Healthcare). For the concentration of the purified antibodies, their absorbance at 280 nm was measured using a spectrophotometer. From the obtained value, the antibody concentration was calculated using the extinction coefficient determined by methods such as PACE (Protein Science 1995; 4: 2411-2423).

[Reference Example 2] Experimental animals and cell lines

The experimental animals used were female C57BL/6 mice (Charles River Laboratories Japan, Inc.) or female Balb/c mice (Charles River Laboratories Japan, Inc.). They were bred in a breeding room under constant conditions (temperature: 20°C to 26°C; lighting: 12-hour light-dark cycle) with ad libitum access to feed and water. The human GPC3 gene was integrated into the chromosome of the mouse lung cancer cell line LLC (ATCC No. CRL-1642) by a method well known to those skilled in the art to obtain an LLC-GPC3 cell line that expresses human GPC3 in high levels. The expression level of human GPC3 ($2.3 \times 10^5$/cell) was determined using the QIFI kit (Dako) by the manufacturer's recommended method. Similarly, the human GPC3 gene was integrated into the mouse colorectal cancer cell line CT-26 (ATCC No. CRL-2638) to obtain the high expression CT26-GPC3 cell line (expression level: $3.1 \times 10^5$/cell). To maintain the human GPC3 gene, these recombinant cell lines were cultured in ATCC-recommended media by adding Geneticin (GIBCO) at 400 μg/ml for LLC-GPC3 and 200 μg/ml for CT26-GPC3. After culturing, these cells were detached using 2.5 g/L trypsin-1 mM EDTA (nacalai tesque), and then used for each of the experiments.

[Example 1] Preparation of an anti-mouse CD137 antibody

1D8VH-MB492 (SEQ ID NO: 29) was prepared according to the method of Reference Example 1 by using as the antibody H chain variable region, 1D8VH (SEQ ID NO: 28) which is a variable region against mouse CD137 disclosed in WO2005/017148, and using as the antibody H-chain constant region, a naturally-occurring mouse IgG1 H chain constant region into which modifications (T230E, V231P, P232N, S238E, S239D, and N324D) that enhance mFcgRII binding have been introduced. 1D8VL disclosed in WO2005/017148 was used as the antibody L chain variable region, and 1D8VL-mk0 (SEQ ID NO: 30) which has the constant region of the mouse κ chain was used as the L chain constant region. They were expressed and purified according to the method of Reference Example 1 to obtain 1D8VH-MB492/1D8VL-mk0. Herein below, this antibody will be described as the anti-mouse CD137 antibody for simplicity.

[Example 2] Preparation of an anti-human GPC3/anti-mouse CD3 bispecific antibody

An anti-human GPC3/anti-mouse CD3 bispecific antibody (GPC3 ERY22-3-2C11) was prepared. The Cross-Mab technique reported by Schaefer *et al.* (Schaefer, Proc. Natl. Acad. Sci., 2011, 108, 11187-11192) was used to regulate the association between the H and L chains and efficiently obtain the bispecific antibodies. More specifically, these molecules were produced by exchanging the VH and VL domains of Fab against human GPC3 described in WO2012/073985. For promotion of heterologous association, the Knobs-into-Holes technology was used for the antibody H chain constant region. The Knobs-into-Holes technology is a technique that enables efficient preparation of heterodimerized antibodies of interest through promotion of the heterodimerization of H chains by substituting an amino acid side chain present in the CH3 region of one of the H chains with a larger side chain (Knob) and substituting an amino acid side chain in the CH3 region of the other H chain with a smaller side chain (Hole) so that the knob will be placed into the hole (Burmeister, Nature, 1994, 372, 379-383). Hereinafter, the constant region into which the Knob modification has been introduced will be indicated as Kn, and the constant region into which the Hole modification has been introduced will be indicated as H1. Furthermore, the modifications described in WO2011/108714 were used to reduce the FcγR binding. Specifically, the IgG1 type was introduced with modifications of substituting Ala for the amino acids at positions 234, 235, and 297 (EU numbering). Gly at position 446 and Lys at position 447 (EU numbering) were removed from the C termini of the antibody H chains. In order to further facilitate purification after antibody expression, a histidine tag was added to the C terminus of the anti-human GPC3 H chain, and a FLAG tag was added to the C terminus of the anti-mouse CD3 H chain. The anti-human GPC3 H chain prepared by introducing the above-mentioned modifications was GC33(2)H-G1dKnHS (SEQ ID NO: 31). As the H chain variable region of the anti-mouse CD3 antibody, 2C11VH-G1dH1S (SEQ ID NO: 33) was prepared by using the sequence of 2C11VH (SEQ ID NO: 32). As the antibody L chains, GC33(2)L-k0 (SEQ ID NO: 34) was used for the anti-human GPC3 side, and 2C11VL-k0 (SEQ ID NO: 35) was used for the anti-mouse CD3 side to obtain the bispecific antibody of interest. The culture supernatant obtained by expression according to the method of Reference Example 1 was added to a MabSelect SuRe column (GE Healthcare), and the column was washed, followed by elution with 50 mM acetic acid. The antibody-containing fraction was added to a HisTrap HP column (GE Healthcare) or a Ni Sepharose FF column (GE Healthcare), and the column was washed, followed by elution with imidazole. The antibody-containing fraction was concentrated using an ultrafiltration membrane, and then, the concentrated solution was added to a Superdex 200 column (GE Healthcare). Only the monomeric antibodies in the eluate were collected to obtain the purified antibody.

[Example 3] Antitumor effect by combined use of an anti-mouse CD137 antibody and an anti-human GPC3/anti-mouse CD3 bispecific antibody

The recombinant mouse colorectal cancer cell line CT26-GPC3 which expresses human GPC3 (Reference Example 2) was suspended in Hanks' Balanced Salt Solution (HBSS) at 1 x $10^7$ cells/mL, and 100 μL of this (1 x $10^6$ cells) was transplanted subcutaneously into the abdomen of BALB/c mice (female, 5-weeks old, Charles River Laboratories Japan Inc.). The mice were randomly divided into four groups of five individuals each, and then the antibodies were administered by intravenous injection through the tail vein 14 days and 17 days after transplantation. The anti-mouse CD137 antibody (1D8-MB492) or the anti-human GPC3/anti-mouse CD3 bispecific antibody (2C11) was diluted with vehicle (0.05% Tween20-PBS) and then made into a 0.5 mg/mL preparation, and this was administered at 10 mL/kg (each at 5 mg/kg). In the combination group, the anti-mouse CD137 antibody and the anti-human GPC3/anti-mouse CD3 bispecific antibody were each administered at 5 mg/kg. The percentage of tumor growth-inhibition (%) was assessed from the tumor volume calculated using the equation below.

$$\text{Tumor volume (mm}^3\text{)} = \text{major axis (mm) x minor axis (mm) x minor axis (mm) / 2}$$

$$\text{Percentage of tumor growth inhibition (\%)} = [1 - (T - T0) / (C - C0)] \times 100$$

T: Average tumor volume of each group on each assay date
T0: Average tumor volume of each group on the first day of administration
C: Average tumor volume of the control group on each assay date
C0: Average tumor volume of the control group on the first day of administration

As shown in Fig. 1, the tumor volume was measured for each group over time. As a result, the percentage of tumor growth inhibition eleven days after the second administration was 96% in the anti-mouse CD137 antibody-administered group, and 48% for the anti-human GPC3/anti-mouse CD3 bispecific antibody. On the other hand, the percentage of tumor growth inhibition was 103% in the combination group.

[Example 4] Hepatotoxicity-reducing effect by combined use of an anti-mouse CD137 antibody and an anti-human GPC3/anti-mouse CD3 bispecific antibody

At the end of the drug efficacy tests for antibody administration, the mice were euthanized by exsanguination under anesthesia, and then plasma was isolated. The plasma was used to measure aspartate amino transferase (AST; JSCC Transferable method), alanine amino transferase (ALT; JSCC Transferable method), and total bilirubin (TBIL; enzyme method) on the TBA-120FR automatic analyzer (Toshiba Medical Systems Corporation). The liver was collected during autopsy, fixed in a 10% neutrally-buffered formalin solution to prepare a tissue preparation of paraffin-embedded thin-tissue sections (hematoxylin-eosin (HE)) and an anti-mouse CD3 immunohistological preparation by following general methods, and the preparations were histopathologically observed under a light microscope.

As a result, as shown in Figs. 2 to 5, in the anti-mouse CD137 antibody-administered group, blood ALT and TBIL were found to increase in all cases, and blood ALT, AST, and TBIL were found to increase remarkably in one case of emergency necropsy in mid-course due to exacerbation of conditions. Histopathologically, liver injury such as mild to severe degeneration/necrosis of liver cells and inflammation accompanying infiltration of CD3-positive cells was found in all cases. On the other hand, in the group to which the anti-mouse CD137 antibody and anti-human GPC3/anti-mouse CD3 bispecific antibody were administered in combination, increases of blood ALT and TBIL that were observed in the anti-mouse CD137 antibody-administered group were suppressed. Histopathologically, slight to mild degeneration/necrosis of liver cells or inflammation was found in all cases, and liver injury was reduced. More specifically, liver injury induced by administration of the anti-mouse CD137 antibody was suggested to be reduced due to combined administration.

RNAs were isolated from a portion of the liver tissues collected by autopsy at the end of the antibody administration tests, and analyzed for the expression levels of inflammatory markers. After treatment of the liver tissues with RNAlater (QIAGEN), total RNA was purified using the automatic nucleic acid separation device, QIAcube (QIAGEN), according to the method specified by the manufacturer. Furthermore, complementary DNA was synthesized using the Transcriptor First strand cDNA synthesis kit (Roche Life Science) according to a method recommended by the manufacturer. Using this complementary DNA as a template, real-time PCR of each target gene shown in Fig. 6 was performed using Power SYBR Green PCR master Mix (Applied Biosystems) and LightCycler 480 (Roche Life Science) by following methods recommended by the manufacturer. Data was corrected using β-actin as the internal standard, and are presented as comparative values by defining the value for the vehicle as 1. As a result, in the anti-mouse CD137 antibody-administered group, expression levels of the inflammatory factors, CD137, IFNg, and granzyme (GZMB), and the surface markers of aggressive T cells, CD3 and CD8, were remarkably enhanced, whereas in the group administered in combination, the expression of these factors was extremely reduced.

The above-mentioned results suggest that combined use of the anti-mouse CD137 antibody and anti-human GPC3/anti-mouse CD3 bispecific antibody reduces liver injury induced by the anti-mouse CD137 antibody.

[Example 5] Preparation of an anti-human GPC3/anti-mouse CD3 bispecific antibody

An anti-human GPC3/anti-mouse CD3 bispecific antibody was constructed by preparing an anti-human GPC3 antibody and an anti-mouse CD3 antibody independently and combining them. As a heavy chain of the anti-human GPC3 antibody, H0000-F760nN17 (SEQ ID NO: 36) carrying a heavy chain constant region that has been modified to reduce Fcγ receptor-binding and enable heterologous association was produced. Furthermore, GL4-k0 (SEQ ID NO: 37) was used for the light chain. As a heavy chain of the anti-mouse CD3 antibody, 2C11VH-F760nP17 (SEQ ID NO: 38) carrying a heavy chain constant region that has been modified to reduce Fcγ receptor-binding and enable heterologous association was produced. Furthermore, 2C11 VL-k0 (SEQ ID NO: 35) was used for the light chain of the anti-mouse CD3 antibody.

The antibodies were each expressed and purified according to the method of Reference Example 1 to obtain an anti-human GPC3 antibody (H0000-F760nN17/GL4-k0) and an anti-mouse CD3 antibody (2C11VH-F760nP17/2C11VL-k0). The purified antibodies were each mixed by a technique known to those skilled in the art (WO2015/046467) that uses difference in the electric charge of the constant regions to produce the bispecific antibody of interest.

[Example 6] Antitumor effect by combined use of an anti-mouse CD137 antibody and an anti-human GPC3/anti-mouse CD3 bispecific antibody

The recombinant mouse lung cancer cell line LLC-GPC3 which expresses human GPC3 (Reference Example 2) was suspended in Hanks' Balanced Salt Solution (HBSS) at 5 x $10^6$ cells/mL, and 200 μL of this (1 x $10^6$ cells) was transplanted subcutaneously into the abdomen of C57BL/6NJcl mice (female, 6-weeks old, CLEA Japan Inc.). Ten days after transplantation, based on the tumor volume data, the mice were randomly divided into four groups of nine individuals each, and then the antibodies were administered by intravenous injection through the tail vein ten days and 14 days after transplantation. The anti-mouse CD137 antibody (1D8-MB492) or the anti-human GPC3/anti-mouse CD3 bispecific antibody (2C11) was diluted with vehicle (0.05% Tween20-PBS) and then made into a 0.5 mg/mL preparation, and this was administered at 10 mL/kg (each at 5 mg/kg). In the combination group, the anti-mouse CD137 antibody and the anti-human GPC3/anti-mouse CD3 bispecific antibody were each administered at 5 mg/kg. Samples were collected 17 days, 21 days, and 25 days after transplantation from three individuals of each group (nine individuals). The percentage of tumor growth-inhibition (%) was assessed from the tumor volume calculated using the equation below.

$$\text{Tumor volume (mm}^3\text{) = major axis (mm) x minor axis (mm) x minor axis (mm) / 2}$$

$$\text{Percentage of tumor growth inhibition (\%)} = [1 - (T - T0) / (C - C0)] \times 100$$

T: Average tumor volume of each group on each assay date
T0: Average tumor volume of each group on the first day of administration
C: Average tumor volume of the control group on each assay date
C0: Average tumor volume of the control group on the first day of administration

As shown in Fig. 8, the tumor volume was measured for each group over time. As a result, the percentage of tumor growth inhibition eleven days after the second administration was 43% in the anti-mouse CD137 antibody-administered group, and 75% for the anti-human GPC3/anti-mouse CD3 bispecific antibody. On the other hand, the percentage of tumor growth inhibition was 104% in the combination group. Since one individual in the anti-mouse CD137 antibody-administered group died 23 days after tumor transplantation, the data 25 days after transplantation for this group as shown in Fig. 8 is the mean of two cases.

[Example 7] Effect in reducing hepatotoxicity by combined use of an anti-mouse CD137 antibody and an anti-human GPC3/anti-mouse CD3 bispecific antibody

On 17 days, 21 days, and 25 days after tumor transplantation in drug efficacy tests for antibody administration using LLC-GPC3 tumor-bearing mice, individuals in the four experimental groups were euthanized by exsanguination under anesthesia, and plasma was isolated. The plasma was used to measure alanine amino transferase (ALT; JSCC Transferable method) on the automatic analyzer TBA-120FR (Toshiba Medical Systems Corporation). Since one individual in the anti-mouse CD137 antibody-administered group died 23 days after tumor transplantation, the data 25 days after transplantation for this group was obtained by calculating the mean of the data from two cases. As a result, as shown in Fig. 9, increases in blood ALT over time were observed for all cases in the anti-mouse CD137 antibody-administered group, whereas the increases in blood ALT were significantly suppressed and alleviation of liver injury was suggested in the group to which the anti-mouse CD137 antibody and the anti-human GPC3/anti-mouse CD3 bispecific antibody were administered in combination.

## Industrial Applicability

It has been shown that pharmaceutical compositions of the present invention can reduce side effects such as liver injury observed when the tumor necrosis factor (TNF) receptor superfamily agonist antibody is prescribed alone, and can induce an excellent antitumor activity specifically towards cancer cells that express a cancer-specific antigen and tumor tissues containing the cancer cells. Activation of immune cells in a cancer antigen-dependent manner yields cytotoxic effects that target various cells including cancer cells, and enables treatment or prevention of various cancers. The patients can have desirable treatments that are not only highly safe but also physically less burdensome and highly convenient.

## Claims

1. A pharmaceutical composition that comprises as an active ingredient a multispecific antibody comprising:

   (1) a cancer-specific antigen-binding domain,
   (2) a CD3-binding domain, and
   (3) a domain comprising an Fc region having decreased Fcγ receptor-binding activity,

   for use in combination with a tumor necrosis factor (TNF) receptor superfamily agonist antibody.

2. The pharmaceutical composition of claim 1, for reducing or eliminating a side effect associated with treatment with the tumor necrosis factor (TNF) receptor superfamily agonist antibody.

3. The pharmaceutical composition of claim 1 or 2, which is a composition for use in the treatment of cancer.

4. The pharmaceutical composition of any one of claims 1 to 3, wherein the tumor necrosis factor (TNF) receptor superfamily agonist antibody is an agonist antibody against CD 137.

5. The pharmaceutical composition of claim 4, wherein the agonist antibody against CD137 comprises an Fc region, wherein the Fc region is an antibody Fc region having an increased binding activity to an inhibitory Fcγ receptor.

6. The pharmaceutical composition of any one of claims 2 to 5, wherein the side effect is mainly liver injury.

7. The pharmaceutical composition of any one of claims 1 to 6, wherein the multispecific antibody is a bispecific antibody.

8. The pharmaceutical composition of any one of claims 1 to 7, wherein an effect of the tumor necrosis factor (TNF) receptor superfamily agonist antibody is a cytotoxicity-inducing effect.

**9.** The pharmaceutical composition of any one of claims 1 to 8, which is administered simultaneously with the tumor necrosis factor (TNF) receptor superfamily agonist antibody.

**10.** The pharmaceutical composition of any one of claims 1 to 8, which is administered separately from the tumor necrosis factor (TNF) receptor superfamily agonist antibody.

**11.** A pharmaceutical composition that comprises a tumor necrosis factor (TNF) receptor superfamily agonist antibody as an active ingredient, for use in combination with a multispecific antibody comprising:

(1) a cancer-specific antigen-binding domain,
(2) a CD3-binding domain, and
(3) a domain comprising an Fc region having decreased Fcγ receptor-binding activity.

**12.** The pharmaceutical composition of claim 11, for inducing an effect of the tumor necrosis factor (TNF) receptor superfamily agonist antibody specifically towards a tumor tissue.

**13.** The pharmaceutical composition of claim 11 or 12, which is a composition for use in the treatment of cancer.

**14.** The pharmaceutical composition of any one of claims 11 to 13, wherein the tumor necrosis factor (TNF) receptor superfamily agonist antibody is an agonist antibody against CD 137.

**15.** The pharmaceutical composition of claim 14, wherein the agonist antibody against CD137 comprises an Fc region, wherein the Fc region is an antibody Fc region having an increased binding activity to an inhibitory Fcγ receptor.

**16.** The pharmaceutical composition of any one of claims 11 to 15, wherein the multispecific antibody is a bispecific antibody.

**17.** The pharmaceutical composition of any one of claims 11 to 16, wherein an effect of the multispecific antibody is an effect of reducing or eliminating a side effect associated with treatment with the tumor necrosis factor (TNF) receptor superfamily agonist antibody.

**18.** The pharmaceutical composition of claim 17, wherein the side effect is mainly liver injury.

**19.** The pharmaceutical composition of any one of claims 11 to 18, which is administered simultaneously with the multispecific antibody.

**20.** The pharmaceutical composition of any one of claims 11 to 18, which is administered separately from the multispecific antibody.

**21.** A pharmaceutical composition that comprises a combination of a multispecific antibody comprising:

(1) a cancer-specific antigen-binding domain,
(2) a CD3-binding domain, and
(3) a domain comprising an Fc region having decreased Fcγ receptor-binding activity,

and a tumor necrosis factor (TNF) receptor superfamily agonist antibody.

**22.** The pharmaceutical composition of claim 21, wherein the pharmaceutical composition is a combination preparation.

**23.** The pharmaceutical composition of claim 21, wherein the multispecific antibody and the agonist antibody are used in combination.

**24.** The pharmaceutical composition of claim 23, wherein the multispecific antibody and the agonist antibody are administered simultaneously or sequentially.

**25.** The pharmaceutical composition of claim 23, wherein the multispecific antibody and the agonist antibody are administered separately.

**26.** The pharmaceutical composition of any one of claims 21 to 25, for reducing or eliminating a side effect associated with treatment with the tumor necrosis factor (TNF) receptor superfamily agonist antibody.

**27.** The pharmaceutical composition of any one of claims 21 to 26, which is a composition for use in the treatment of cancer.

**28.** The pharmaceutical composition of any one of claims 21 to 27, wherein the tumor necrosis factor (TNF) receptor superfamily agonist antibody is an agonist antibody against CD 137.

**29.** The pharmaceutical composition of claim 28, wherein the agonist antibody against CD137 comprises an Fc region, wherein the Fc region is an antibody Fc region having an increased binding activity to an inhibitory Fcγ receptor.

**30.** The pharmaceutical composition of any one of claims 26 to 29, wherein the side effect is mainly liver injury.

**31.** The pharmaceutical composition of any one of claims 21 to 30, wherein the multispecific antibody is a bispecific antibody.

**32.** A method for inducing an effect of a tumor necrosis factor (TNF) receptor superfamily agonist antibody specifically towards a tumor tissue, which comprises using a multispecific antibody comprising:

(1) a cancer-specific antigen-binding domain, and
(2) a CD3-binding domain; and

accumulating T cells in the tumor tissue expressing the cancer-specific antigen, wherein the tumor necrosis factor (TNF) receptor superfamily agonist antibody is used in combination with the multispecific antibody.

**33.** The method of claim 32, for reducing or eliminating a side effect associated with the agonist antibody treatment.

**34.** The method of claim 32 or 33, wherein the tumor necrosis factor (TNF) receptor superfamily agonist antibody is an agonist antibody against CD 137.

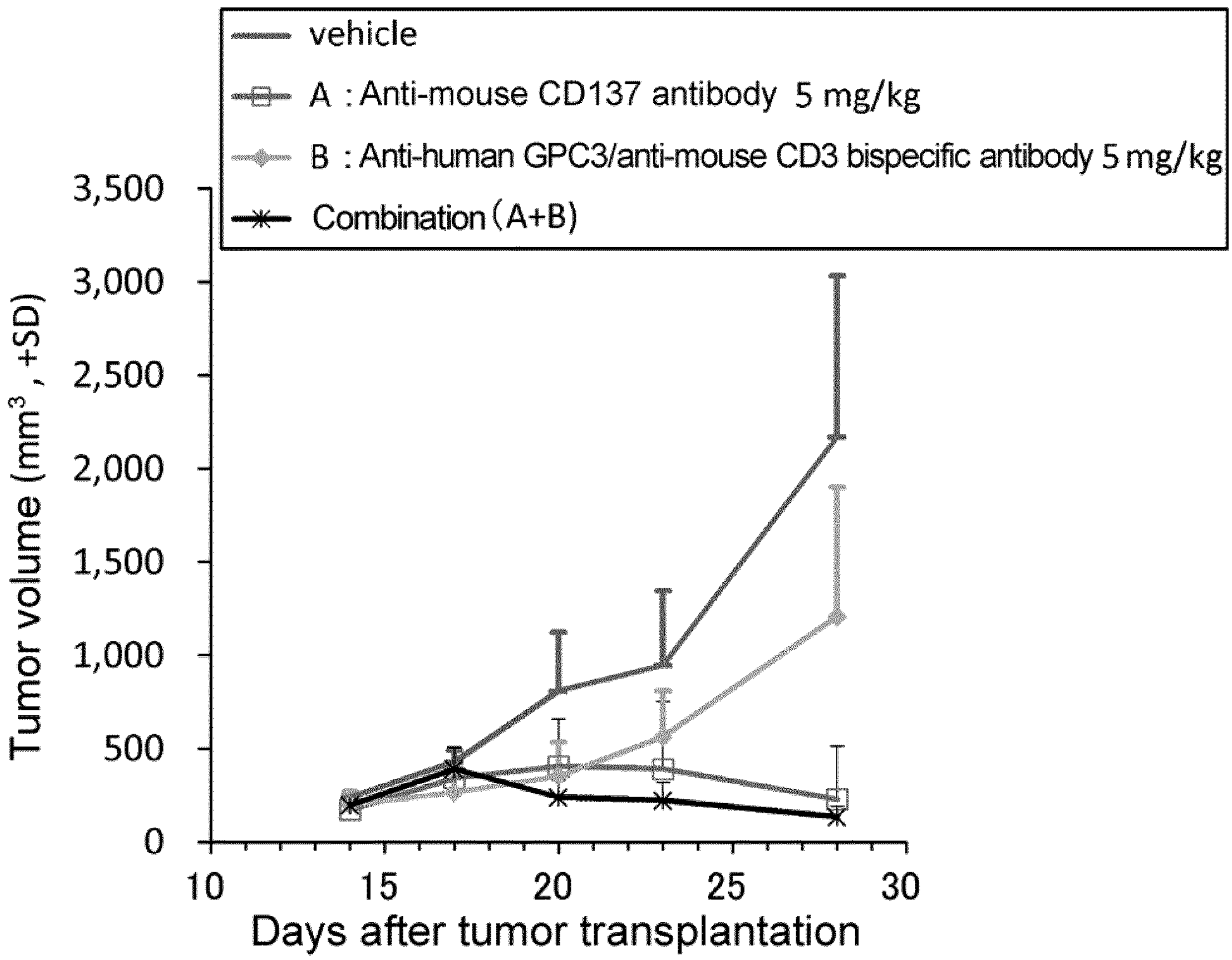
vehicle
A : Anti-mouse CD137 antibody 5 mg/kg
B : Anti-human GPC3/anti-mouse CD3 bispecific antibody 5 mg/kg
Combination (A+B)
Tumor volume (mm3 , +SD)
3,500
3,000
2,500
2,000
1,500
1,000
500
0
10
15
20
25
30
Days after tumor transplantation

FIG. 1

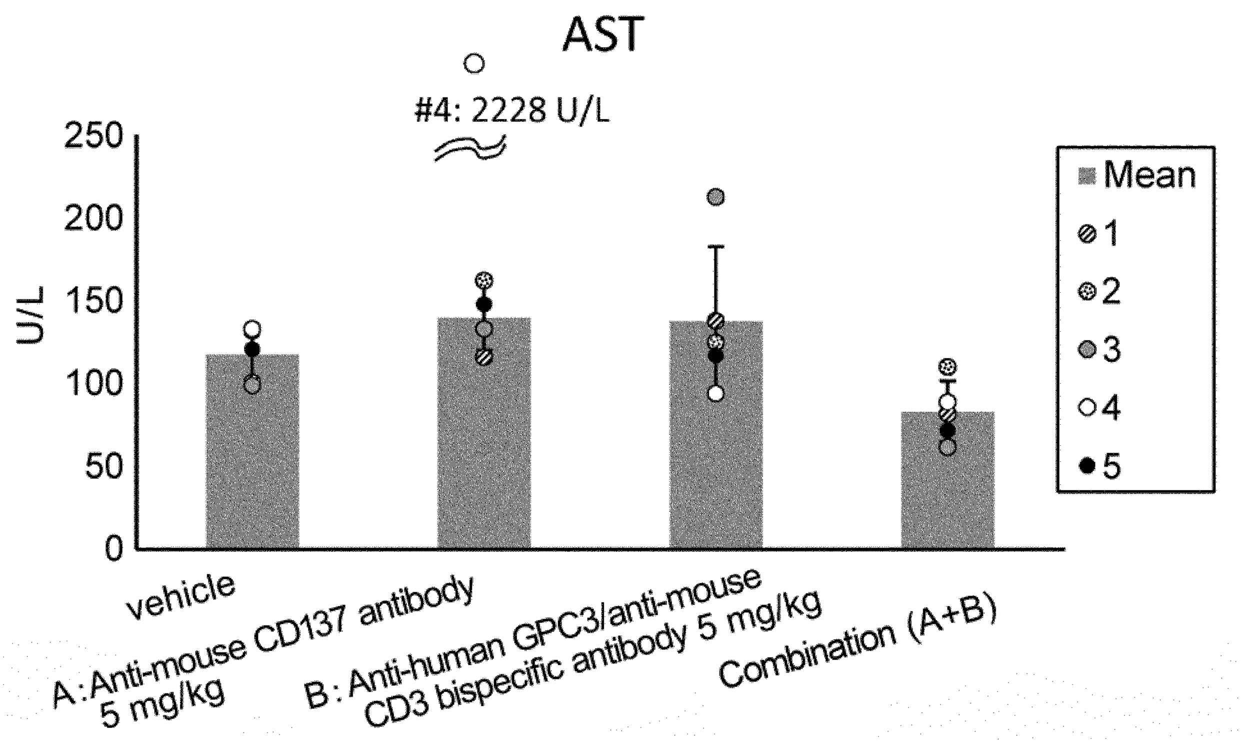
AST
#4: 2228 U/L
250
200
150
100
50
0
U/L
Mean
1
2
3
4
5
vehicle
A: Anti-mouse CD137 antibody 5 mg/kg
B: Anti-human GPC3/anti-mouse CD3 bispecific antibody 5 mg/kg
Combination (A+B)

FIG. 2

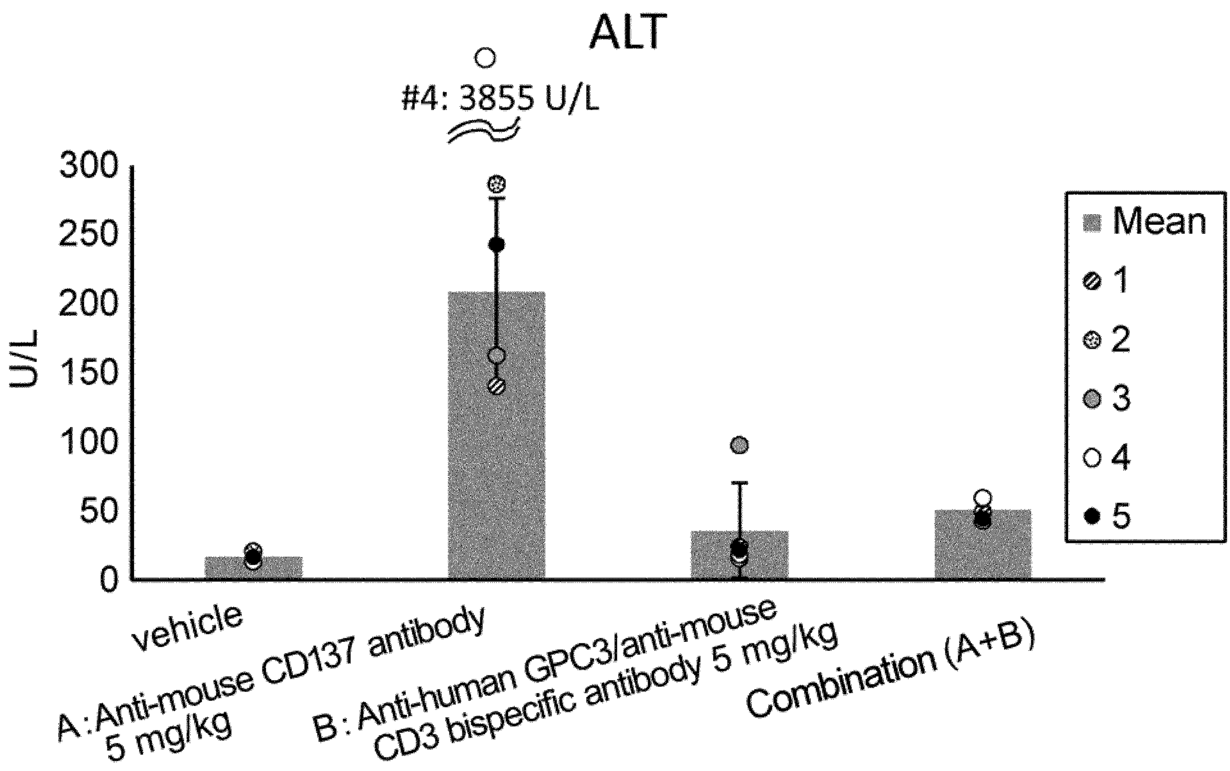
ALT
#4: 3855 U/L
300
250
200
150
100
50
0
U/L
Mean
1
2
3
4
5
vehicle
A:Anti-mouse CD137 antibody 5 mg/kg
B: Anti-human GPC3/anti-mouse CD3 bispecific antibody 5 mg/kg
Combination (A+B)

FIG. 3

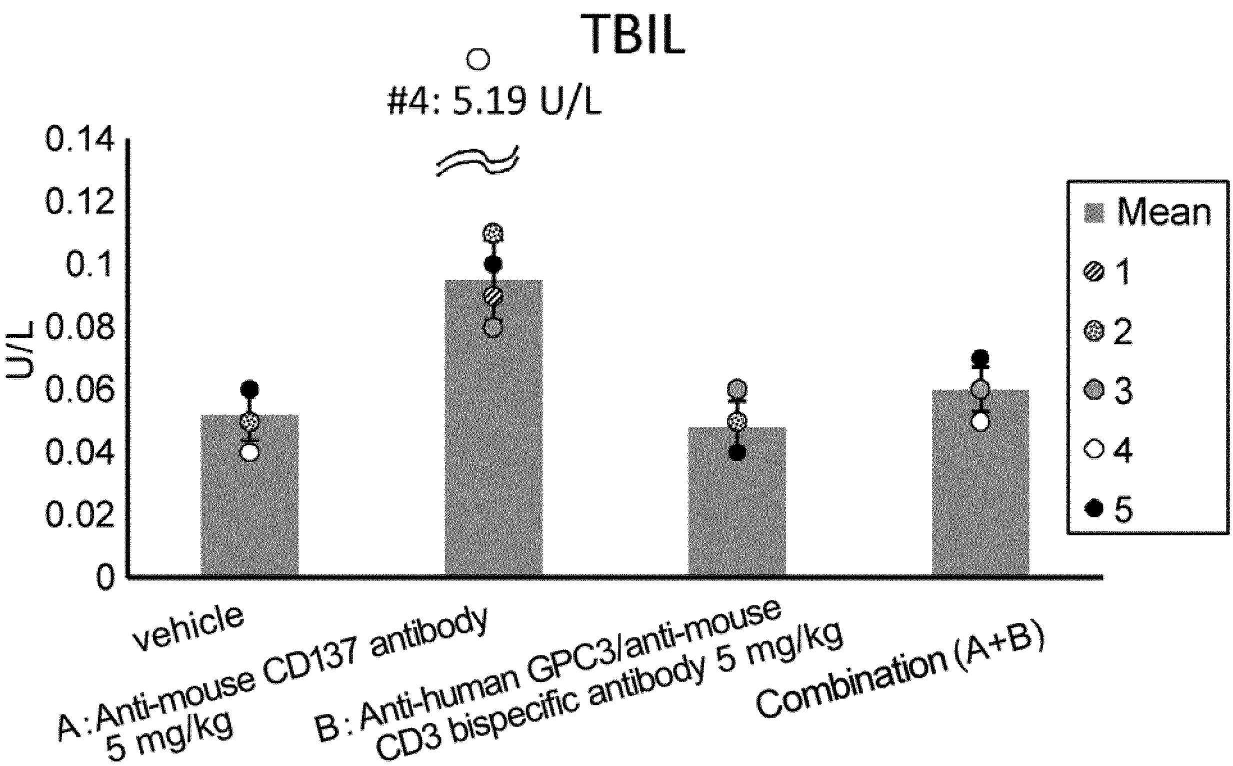
TBIL
#4: 5.19 U/L
0.14
0.12
0.1
0.08
0.06
0.04
0.02
0
U/L
Mean
1
2
3
4
5
vehicle
A: Anti-mouse CD137 antibody 5 mg/kg
B: Anti-human GPC3/anti-mouse CD3 bispecific antibody 5 mg/kg
Combination (A+B)

FIG. 4

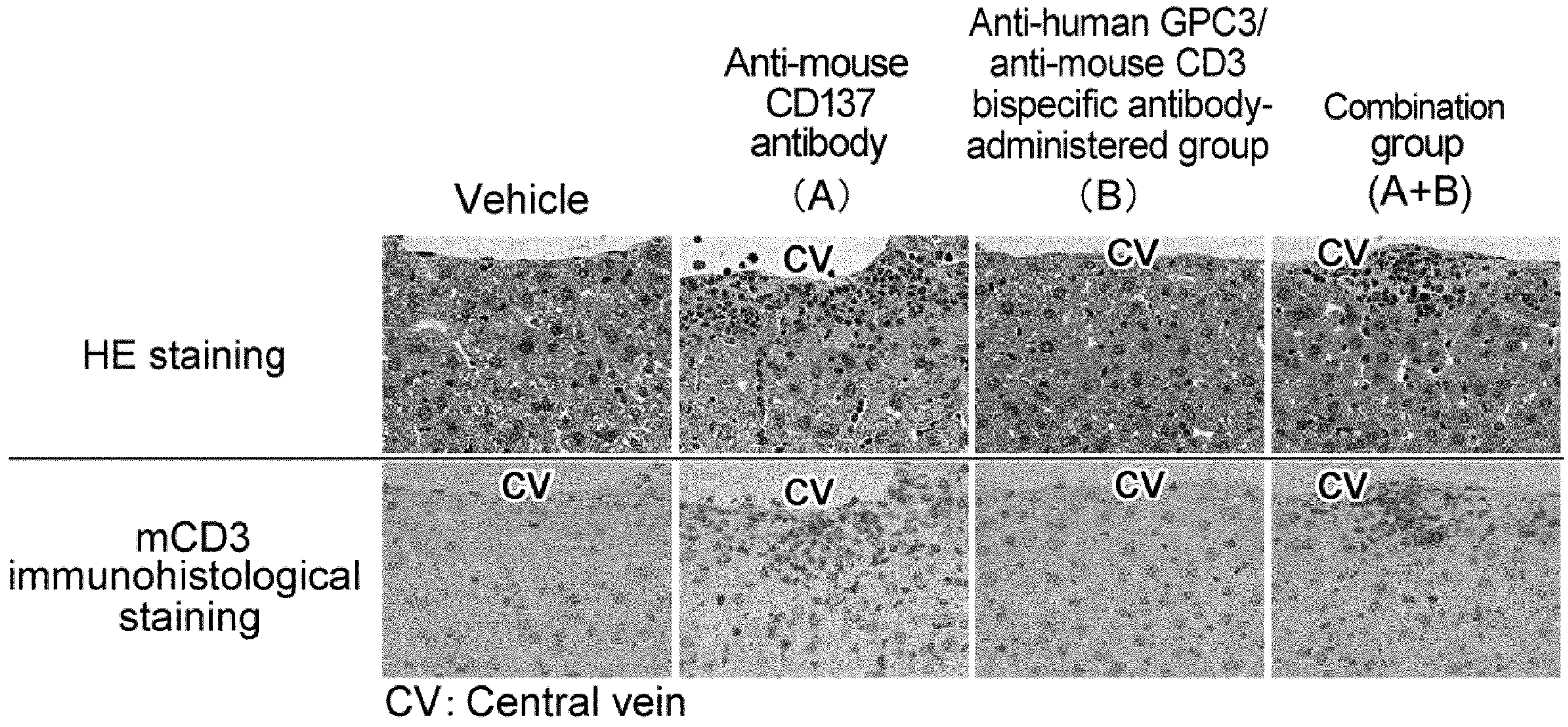
Vehicle
Anti-mouse CD137 antibody (A)
Anti-human GPC3/ anti-mouse CD3 bispecific antibody-administered group (B)
Combination group (A+B)
HE staining
mCD3 immunohistological staining
CV
CV: Central vein

FIG. 5

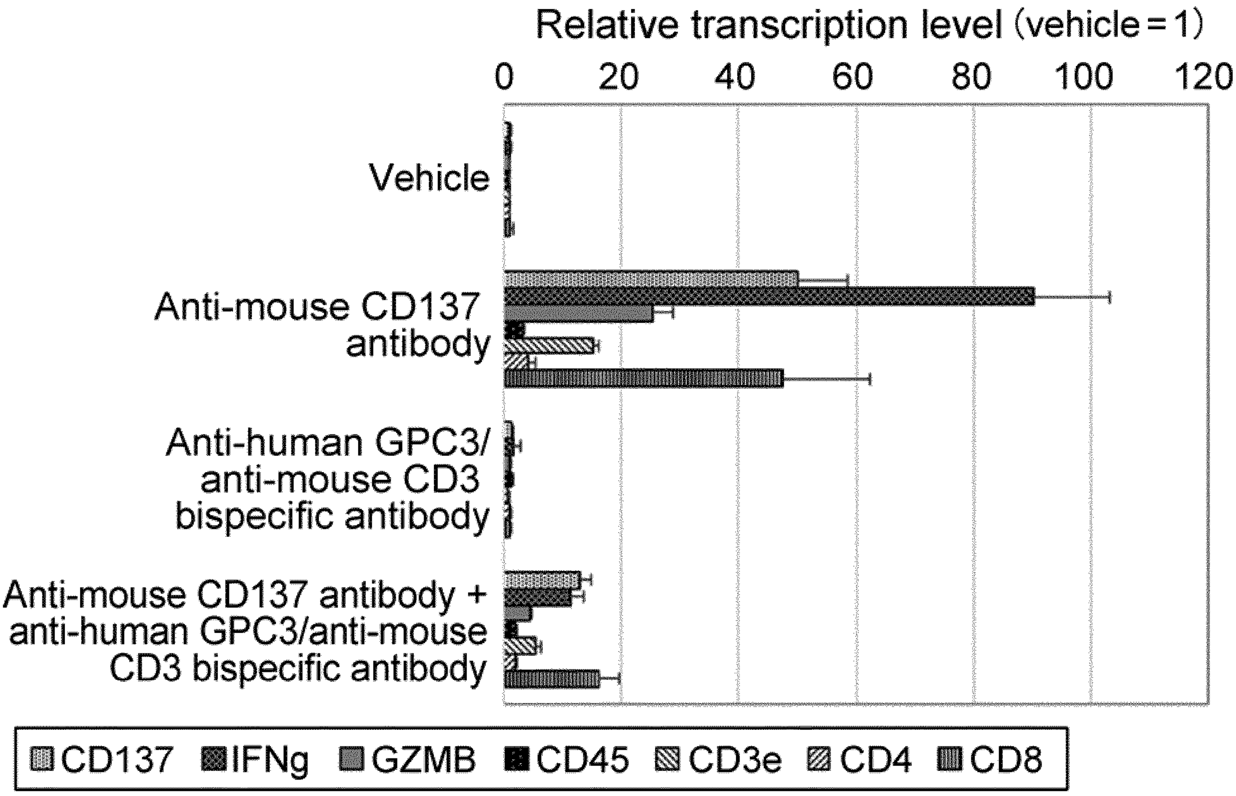
Relative transcription level (vehicle = 1)
0
20
40
60
80
100
120
Vehicle
Anti-mouse CD137 antibody
Anti-human GPC3/ anti-mouse CD3 bispecific antibody
Anti-mouse CD137 antibody + anti-human GPC3/anti-mouse CD3 bispecific antibody
CD137
IFNg
GZMB
CD45
CD3e
CD4
CD8

FIG. 6

```
Kabat     1                                                                            2
EU index  1-2---------3---------4---------5---------6---------7---------8---------9---------0---------1---------2--
          8-0---------0---------0---------0---------0---------0---------0---------0---------0---------0---------0--
IgG1      ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCD-
IgG2      ASTKGPSVFPLAPCSRSTSESTAALGCLVKDYFPEPVTVSWNSGALTSGVHTFPAVLQSSGLYSLSSVVTVPSSNFGTQTYTCNVDHKPSNTKVDKTVERK--CC
IgG3      ASTKGPSVFPLAPCSRSTSGGTAALGCLVKDYFPEPVTVSWNSGALTSGVHTFPAVLQSSGLYSLSSVVTVPSSSLGTQTYTCNVNHKPSNTKVDKRVELKTPLG
IgG4      ASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYG--

Kabat      2
EU index   2------------------------------------------------------3---------4---------5---------6---------7---------8
           2-----8------------------------------------------------0---------0---------0---------0---------0---------0
IgG1      -KTHTCPP------------------------------------------------CPAPELLGGPSVFLFPPKPKDTLMISRTPEVTCVVVDVSHEDPEVKFNWYVD
IgG2      -V—E-CPP------------------------------------------------CPAPPVA-GPSVFLFPPKPKDTLMISRTPEVTCVVVDVSHEDPEVQFNWYVD
IgG3      DTTHTCPRCPEPKSCDTPPPCPRCPEPKSCDTPPPCPRCPEPKSCDTPPPCPRCPAPELLGGPSVFLFPPKPKDTLMISRTPEVTCVVVDVSHEDPEVQFKWYVD
IgG4      ---PPCPS------------------------------------------------CPAPEFLGGPSVFLFPPKPKDTLMISRTPEVTCVVVDVSQEDPEVQFNWYVD

Kabat     2                  3
EU index  8--------9---------0---------1---------2---------3---------4---------5---------6---------7---------8-----
          1--------0---------0---------0---------0---------0---------0---------0---------0---------0---------0-----
IgG1      GVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNG
IgG2      GVEVHNAKTKPREEQFNSTFRVVSVLTVVHQDWLNGKEYKCKVSNKGLPAPIEKTISKTKGQPREPQVYTLPPSREEMTKNQVSLTCLVKGFYPSDIAVEWESNG
IgG3      GVEVHNAKTKPREEQYNSTFRVVSVLTVLHQDWLNGKEYKCKVSNKALPAPIEKTISKTKGQPREPQVYTLPPSREEMTKNQVSLTCLVKGFYPSDIAVEWESSG
IgG4      GVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNG

Kabat     3             4
EU index  8---9---------0---------1---------2---------3---------4
          6---0---------0---------0---------0---------0---------0------7
IgG1      QPENNYKTTPPVLDSDGSFFLYSKLTVDKSRWQQGNVFSCSVMHEALHNHYTQKSLSLSPGK  (SEQ ID NO:5)
IgG2      QPENNYKTTPPMLDSDGSFFLYSKLTVDKSRWQQGNVFSCSVMHEALHNHYTQKSLSLSPGK  (SEQ ID NO:6)
IgG3      QPENNYNTTPPMLDSDGSFFLYSKLTVDKSRWQQGNIFSCSVMHEALHNRFTQKSLSLSPGK  (SEQ ID NO:7)
IgG4      QPENNYKTTPPVLDSDGSFFLYSRLTVDKSRWQEGNVFSCSVMHEALHNHYTQKSLSLSLGK  (SEQ ID NO:8)
```

FIG. 7

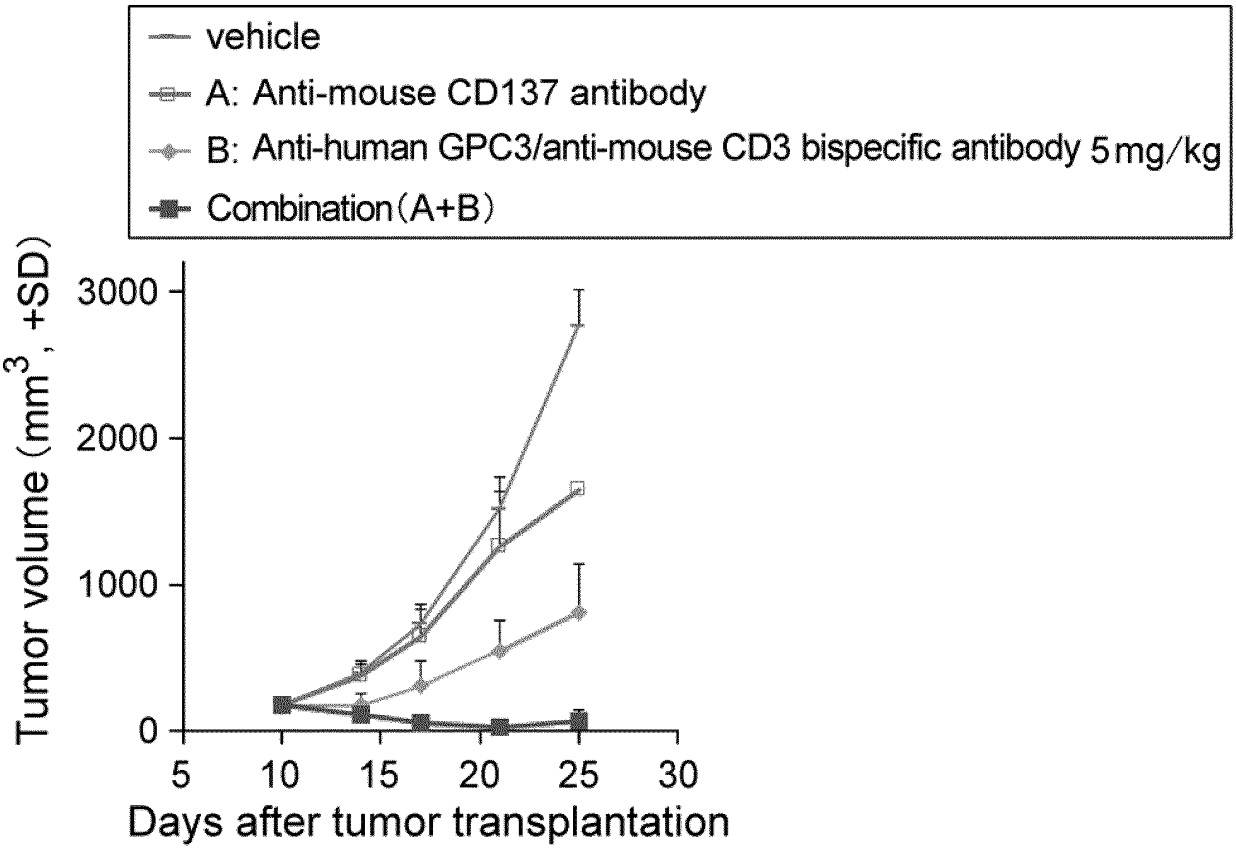
vehicle
A: Anti-mouse CD137 antibody
B: Anti-human GPC3/anti-mouse CD3 bispecific antibody 5mg/kg
Combination (A+B)
Tumor volume (mm3, +SD)
3000
2000
1000
0
5
10
15
20
25
30
Days after tumor transplantation

FIG. 8

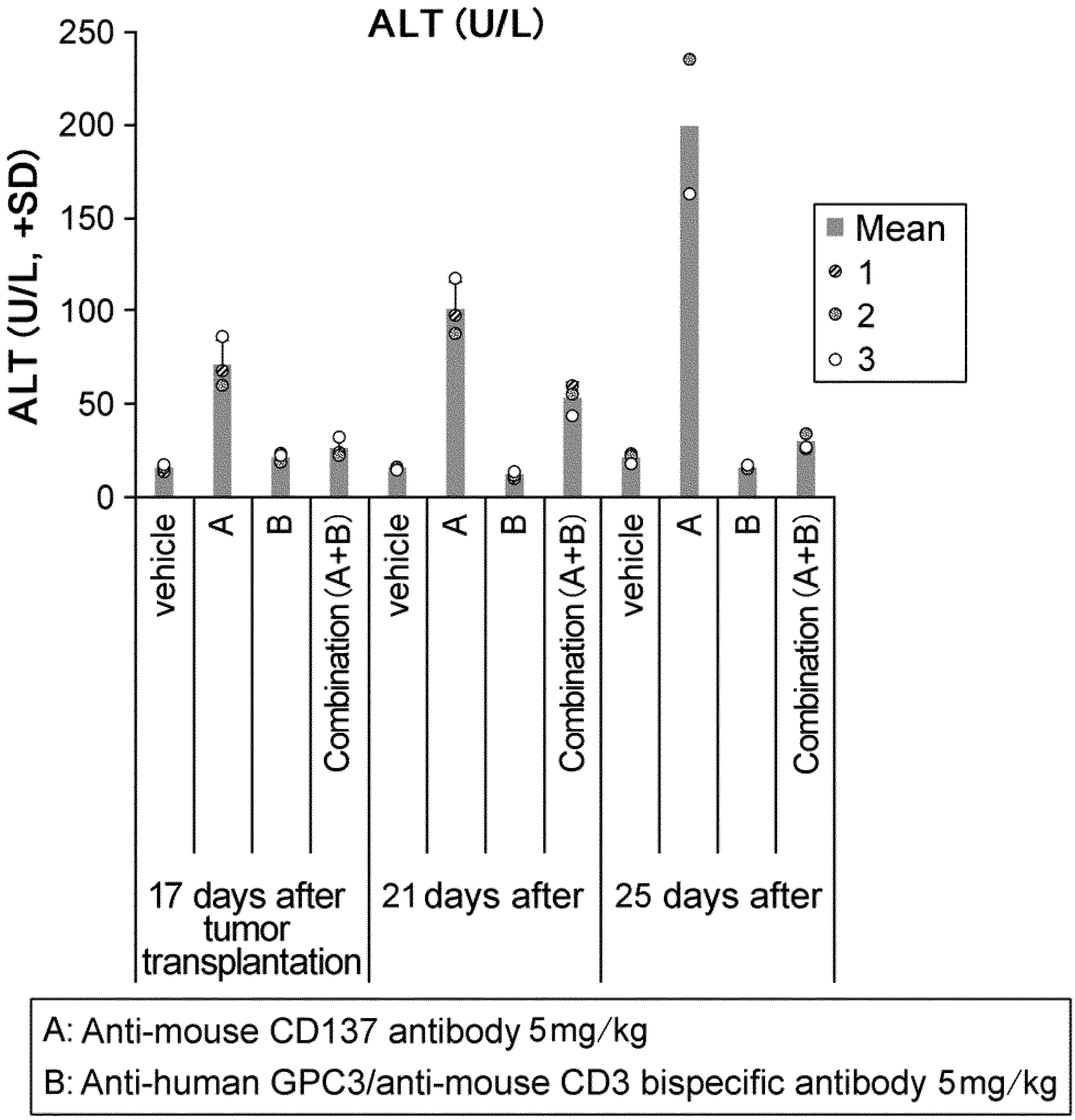
ALT (U/L)
ALT (U/L, +SD)
250
200
150
100
50
0
Mean
1
2
3
vehicle
A
B
Combination (A+B)
vehicle
A
B
Combination (A+B)
vehicle
A
B
Combination (A+B)
17 days after tumor transplantation
21 days after
25 days after
A: Anti-mouse CD137 antibody 5mg/kg
B: Anti-human GPC3/anti-mouse CD3 bispecific antibody 5mg/kg

FIG. 9

## INTERNATIONAL SEARCH REPORT

International application No. PCT/JP2016/066331

A. CLASSIFICATION OF SUBJECT MATTER
*A61K39/395*(2006.01)i, *A61P35/00*(2006.01)i

According to International Patent Classification (IPC) or to both national classification and IPC

B. FIELDS SEARCHED

Minimum documentation searched (classification system followed by classification symbols)
A61K39/395, A61P35/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
JSTPlus/JMEDPlus/JST7580(JDreamIII), CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| Y | JP 2007-532095 A (Bristol-Myers Squibb Co.), 15 November 2007 (15.11.2007), claims 1, 6; paragraphs [0001], [0005], [0017] & US 2005/0095244 A1 & US 2009/0068192 A1 & US 2010/0183621 A1 & US 2012/0141494 A1 & US 2014/0193422 A1 & WO 2005/035584 A1 claims 1 and 6; page 1, lines 2 to 3; page 3, 2nd paragraph; page 8, the last paragraph & EP 1670828 A1 & CA 2542044 A & IS 8401 A & NO 20061394 A & KR 10-2006-0126455 A & CN 1867585 A & ZA 200602834 A & NZ 546017 A & RU 2006115835 A & AU 2004279877 A & IL 174460 A & AR 46094 A & PE 9622005 A & RS 20060234 A & MX PA06003788 A & KR 10-1119266 B1 & ES 2432357 T & DK 1670828 T | 1-34 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 July 2016 (01.07.16) | 19 July 2016 (19.07.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No. PCT/JP2016/066331

C (Continuation). DOCUMENTS CONSIDERED TO BE RELEVANT

| Category* | Citation of document, with indication, where appropriate, of the relevant passages | Relevant to claim No. |
|---|---|---|
| | & PT 1670828 E & SI 1670828 T<br>& HK 1085226 A | |
| Y | WO 2014/108483 A1 (GENMAB B.V.),<br>17 July 2014 (17.07.2014),<br>claims 1, 24, 25, 31; page 13, 3rd paragraph<br>& JP 2016-508153 A & US 2015/0337049 A1<br>& US 2015/0175707 A1 & US 2014/0242075 A1<br>& WO 2014/006217 A1 & WO 2013/004842 A2<br>& WO 2015/001085 A1 & WO 2015/104346 A1<br>& EP 2943507 A1 & EP 2869845 A1<br>& EP 2729170 A2 & CA 2915575 A<br>& AU 2014286116 A & KR 10-2016-0027177 A<br>& AU 2013285355 A & CN 104736174 A<br>& AU 2012280222 A & CA 2840147 A<br>& EA 201400108 A & CN 104220093 A<br>& MX 2013014961 A & KR 10-2014-0146040 A | 1-34 |
| Y | WO 2012/115241 A1 (Chugai Pharmaceutical Co., Ltd.),<br>30 August 2012 (30.08.2012),<br>paragraph [0123]<br>& JP 2014-528906 A & US 2014/0093496 A1<br>paragraph [0226]<br>& US 2014/0105889 A1 & US 2014/0335089 A1<br>& US 2016/0046693 A & US 2014/0363428 A1<br>& WO 2012/133782 A & WO 2013/047752 A<br>& WO 2013/125667 A & WO 2013/046704 A<br>& WO 2012/115241 A1 & EP 2679681 A1<br>& EP 2698431 A1 & EP 2762166 A1<br>& EP 2818183 A1 & EP 2760890 A2<br>& TW 201321412 A & CA 2827923 A<br>& SG 192945 A & AU 2012222252 A<br>& AU 2012233313 A & CA 2831770 A<br>& CN 103492565 A & SG 194076 A<br>& TW 201343673 A & MX 2013009774 A<br>& KR 10-2014-0014167 A & KR 10-2014-0015501 A<br>& CN 103703129 A & AU 2012317418 A<br>& CA 2850194 A & KR 10-2014-0076593 A<br>& CN 103974721 A & MX 2013011366 A<br>& AU 2013223087 A & CA 2865158 A<br>& MX 2014003830 A & CN 104244980 A<br>& MX 2014010140 A & KR 10-2014-0130707 A<br>& HK 1198578 A & RU 2013143303 A<br>& RU 2013148116 A & HK 1201476 A<br>& RU 2014117505 A & RU 2014138499 A<br>& TW 201326209 A & AU 2012313670 A<br>& CA 2850035 A & KR 10-2014-0069332 A<br>& CN 103958547 A & MX 2014003891 A<br>& RU 2014117500 A | 5,15,29 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009011941 A
- WO 2013125667 A
- WO 2012115241 A
- WO 2013047752 A
- WO 2014030728 A
- WO 2014163101 A
- WO 9411523 A
- EP 239400 A
- WO 1996002576 A
- WO 1993012227 A
- WO 1992003918 A
- WO 1994002602 A
- WO 1994025585 A
- WO 1996034096 A
- WO 1996033735 A
- WO 1992001047 A
- WO 1992020791 A
- WO 1993006213 A
- WO 1993011236 A
- WO 1993019172 A
- WO 1995001438 A
- WO 1995015388 A
- WO 1988001649 A
- US 4946778 A
- US 5260203 A
- WO 2006132352 A
- WO 2006106905 A
- WO 1996027011 A
- US 20130336973 A
- WO 2011028952 A
- WO 2014018572 A
- WO 2008119353 A
- WO 2011131746 A
- WO 2012058768 A
- WO 2013063702 A
- WO 2012023053 A
- WO 2015046467 A
- WO 2007114325 A
- WO 98050431 A
- WO 95033844 A
- WO 2004065611 A
- US 3773719 A
- EP 58481 A
- EP 133988 A
- WO 2005017148 A
- WO 2012073985 A
- WO 2011108714 A


### Non-patent literature cited in the description


- **HANAHAN.** *Cell,* 2011, vol. 144, 646-74
- **PRIETO.** *Clin Cancer Res.,* 2012, vol. 18, 2039-47
- **HAMID.** *Expert Opin. Biol. Ther.,* 2013, vol. 6, 847-61
- **SUMMERS.** *Nat Rev Immunol,* 2012, vol. 12, 339-51
- **VINAY.** *Cellular & Molecular Immunology,* 2011, vol. 8, 281-284
- **HOUOT.** *Blood,* 2009, vol. 114, 3431-8
- **ASCIERTO.** *Semin Oncol,* 2010, vol. 37, 508-16
- **DUBROT.** *Cancer Immunol Immunother,* 2010, vol. 59, 1223-33
- **SCHABOWSKY.** *Vaccine,* 2009, vol. 28, 512-22
- **YONEZAWA.** *Clin. Cancer Res.,* 23 April 2015
- **SON.** *J Immunol Methods,* 2004, vol. 286, 187-201
- **PORTER.** *N ENGL J MED,* 2011, vol. 365, 725-733
- **BRANDL.** *Cancer Immunol Immunother,* 2007, vol. 56, 1551-63
- **FILMUS.** *J. Clin. Invest.,* 2001, vol. 108, 497-501
- **ZHU-ZU-W.** *Gut,* 2001, vol. 48, 558-564
- **YAMAUCHI.** *Mod. Pathol.,* 2005, vol. 18, 1591-1598
- **YORITA.** *Liver Int.,* 2010, vol. 1, 120-131
- Sequences of Proteins of Immunological Interest. Public Health Service, National Institutes of Health, 1991
- *J. Exp. Med,* 1990, vol. 172, 19-25
- *Arthritis. Rheum.,* 2002, vol. 46, 1242-1254
- *J. Clin. Invest.,* 1997, vol. 100 (5), 1059-1070
- *J. Clin. Invest.,* 1990, vol. 85, 1287-1295
- *Proc. Natl. Acad. Sci. USA,* 2006, vol. 103 (11), 4005-4010
- *Proc. Natl. Acad. Sci USA,* 2006, vol. 103 (11), 4005-4010

- *J. Rheumatol,* 2007, vol. 34, 11
- *Hum. Antibod. Hybridomas,* 1990, vol. 1 (1), 47-54
- *Blood,* 2007, vol. 109, 1185-1192
- *Nat. Rev. Immunol.,* 2012, vol. 12 (3), 39-51
- *Proc. Natl. Acad. Sci. USA,* 1980, vol. 77, 4914-4917
- *CHEMICAL ABSTRACTS,* 934823-49-1
- Antibodies A Laboratory Manual. Cold Spring Harbor Laboratory, 1988
- *J. Immunol.,* 1979, vol. 123 (4), 1548-1550
- *Current Topics in Microbiology and Immunology,* 1978, vol. 81, 1-7
- *C. Eur. J. Immunol.,* 1976, vol. 6 (7), 511-519
- *Cell,* 1976, vol. 8 (3), 405-415
- *Nature,* 1978, vol. 276 (5685), 269-270
- *J. Immunol. Methods,* 1980, vol. 35 (1-2), 1-21
- *J. Exp. Med.,* 1978, vol. 148 (1), 313-323
- *Nature,* 1979, vol. 277 (5692), 131-133
- **KOHLER ; MILSTEIN et al.** *Methods Enzymol.,* 1981, vol. 73, 3-46
- **VANDAMME et al.** *Eur. J. Biochem.,* 1990, vol. 192 (3), 767-775
- *Biochemistry,* 1979, vol. 18 (24), 5294-5299
- *Anal. Biochem.,* 1987, vol. 162 (1), 156-159
- *Proc. Natl. Acad. Sci. USA,* 1988, vol. 85 (23), 8998-9002
- *Nucleic Acids Res.,* 1989, vol. 17 (8), 2919-2932
- *Bio/Technology,* 1994, vol. 12 (7), 699-702
- **SATO, K. et al.** *Cancer Res.,* 1993, vol. 53, 851-856
- *Nat Biotechnol.,* December 2000, vol. 18 (12), 1287-92
- *Nucleic Acids Res.,* 2006, vol. 34 (19), e127
- *Proc Natl Acad Sci U S A.,* 02 March 2004, vol. 101 (9), 2806-10
- *Proc Natl Acad Sci USA.,* 22 June 2004, vol. 101 (25), 9193-8
- *Protein Eng Des Sel.,* April 2008, vol. 21 (4), 247-55
- *Proc NatlAcad Sci U S A.,* 26 September 2000, vol. 97 (20), 10701-5
- *MAbs.,* September 2010, vol. 2 (5), 508-18
- **KABAT.** Sequences of Proteins of Immunological Interest. 1991
- **CHOTHIA et al.** *Science,* 1986, vol. 233, 755-758
- *J Mol Biol,* 1996, vol. 262, 732-745
- Epitope Mapping Protocols in Methods in Molecular Biology. 1996, vol. 66
- Antibodies A Laboratory Manual. Cold Spring Harbor Laboratory, 1988, 359-420
- The Pharmacology of Monoclonal Antibodies. Springer-Verlag, 1994, vol. 113, 269-315
- *Proc. Natl. Acad. Sci. U.S.A.,* 1988, vol. 85 (16), 5879-5883
- *J Immunol. Methods,* 1999, vol. 231 (1-2), 177-189
- *the Journal of Immunology,* 1994, vol. 152 (11), 5368-5374
- *Science,* 1988, vol. 240 (4855), 1038-1041
- *Protein Engineering,* 1996, vol. 9 (3), 299-305
- **RIDGWAY JB et al.** *Protein Engineering,* 1996, vol. 9, 617-621
- **MERCHANT AM et al.** *Nature Biotechnology,* 1998, vol. 16, 677-681
- *Protein Engineering Design & Selection,* 2010, vol. 23, 195-202
- *Nat Biotechnol.,* February 2014, vol. 32 (2), 191-8
- **CHRISTOPH et al.** *Nature Biotechnology,* 2013, vol. 31, 753-758
- *Nature Biotechnology,* 1998, vol. 16, 677-681
- Remington's Pharmaceutical Science 16th edition. 1980
- *J. Biomed. Mater. Res.,* 1981, vol. 15, 267-277
- *Chemtech.,* 1982, vol. 12, 98-105
- *Biopolymers,* 1983, vol. 22, 547-556
- Remington's Pharmaceutical Science. Mark Publishing Company
- Immunologic studies in humans. Current protocols in Immunology. John Wiley & Sons, Inc, 1993
- *Protein Science,* 1995, vol. 4, 2411-2423
- **SCHAEFER.** *Proc. Natl. Acad. Sci.,* 2011, vol. 108, 11187-11192
- **BURMEISTER.** *Nature,* 1994, vol. 372, 379-383